# EUROPÄISCHE PATENTANMELDUNG

(11) **EP 2 316 830 A2**
(43) Veröffentlichungstag der Anmeldung: **04.05.2011**
(21) Anmeldenummer: 10013067.3
(22) Anmeldetag: 17.05.2004
(51) Int. Cl.: C07D 295/18, A61K 31/445, A61K 31/495, C07D 211/26, C07D 211/62, C07D 205/04, C07D 307/79, C07K 5/062

(54) **N-sulfonylierte Aminosäurederivate und ihre Verwendung als Matriptaseinhibitoren**

(30) Priorität: 16.05.2003 DE 10322191
(62) Teilanmeldung aus: 04733311.7
(71) Anmelder: The Medicines Company (Leipzig) GmbH, 04103 Leipzig (DE)
(72) Erfinder: Stürzebecher, Ute, 99094 Erfurt (DE); Steinmetzer, Prof. Dr. Torsten, 07743 Jena (DE); Schweinitz, Dr. Andrea, D- 07749 Jena (DE); Stürzebecher, Anne, 99427 Weimar (DE); Uhland, Kerstin, 82166 Gräfelfing (DE)
(74) Vertreter: Bösl, Raphael Konrad

(57) **Zusammenfassung**

Die Erfindung betrifft N-sulfonylierte Aminosäurederivate der Formel I'' 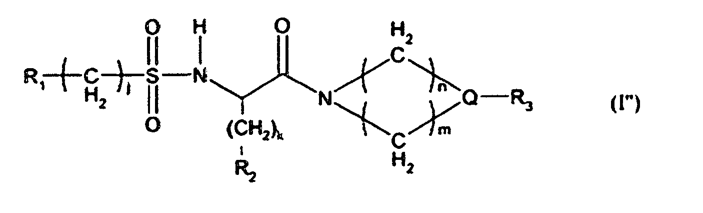 Ebenso betrifft die Erfindung Verfahren zur Herstellung dieser Verbindungen und ihre Verwendung, insbesondere als Hemmstoffe der Matriptase.

## Beschreibung

Die vorliegende Erfindung betrifft N-sulfonylierte Aminosäurederivate, wobei über die Sulfonylgruppe N-terminal ein Arylrest an die Aminosäure, gebunden ist sowie C-terminal über die Carbonylgruppe ein Rest gebunden ist, der mindestens eine Iminogruppe und mindestens eine weitere basische Gruppe, die eine gegebenenfalls modifizierte Amino-, Amidino- oder Guanidinogruppe darstellt, enthält. Ebenso betrifft die Erfindung Verfahren zur Herstellung dieser Verbindungen und ihre Verwendung, bevorzugt als Arzneimittel und diesbezüglich insbesondere als Hemmstoffe der Matriptase.

Proteasen regulieren zahlreiche physiologische Prozesse, die das Wachstum und die Metastasierung von Tumorzellen ermöglichen bzw. stimulieren. Das betrifft insbesondere den proteolytischen Abbau der die Tumorzellen umgebenden extrazellulären Matrixproteine, durch den die Invasion der von Tumoren abgesiedelten Tumorzellen in angrenzende Gewebe und in das Lymph- bzw. Blutsystem ermöglicht wird. Proteasen sind auch an der Aktivierung von Wachstumsfaktoren beteiligt, die z.B. die Proliferation von Tumorzellen oder die Angiogenese stimulieren und damit das Tumorwachstum ermöglichen. Zu diesen proteolytischen Enzymen gehören verschiedene Matrix-Metalloproteasen, membrangebundene Metalloproteasen, lysosomale Cysteinproteasen und eine Vielzahl von Serinproteasen, wie z.B. Urokinase, Plasmin, Elastase, Thrombin oder Cathepsin G, und auch die Type-II-Transmembran-Serinprotease Matriptase oder MT-SP1 (Hopper et al., J. Biol. Chem. 276, 857-860, 2001).

Es gibt zahlreiche Versuche, durch den Einsatz von Proteaseinhibitoren das Wachstum und die Metastasierung von Tumoren zu hemmen, doch zeigten Versuche mit Hemmstoffen der Matrix-Metalloproteasen bisher kaum Wirkung in klinischen Studien (Coussens et al., Science 295, 2387-2392, 2002). Inzwischen wurden auch erste klinische Untersuchungen mit Hemmstoffen der Urokinase initiiert, wobei aber noch keine Ergebnisse über deren Wirksamkeit bekannt sind.

Matriptase, ist eine trypsinartige Serinprotease, die ursprünglich aus Brustkrebszellen isoliert wurde und bevorzugt C-terminal Peptidbindungen der basischen Aminosäure Arginin spaltet. (Shi et al., Cancer Res. 53, 1409-1415, 1993; Lin et al., J. Biol. Chem. 272, 9147-9152, 1997).

1998 wurde das Gen der Matriptase als putativer Tumorsuppressor durch ein subtraktives Hybridisierungsverfahren, bei dem gesundes und kanzerogenes Darmgewebe verwendet wurden, kloniert (Zhang et al. Cytogenet. Cell Genet. 83, 56-57, 1998).

Matriptase und MT-SP1 (Abkürzung für "membrane-type serine protease 1) (Takeuchi et al., Proc. Natl. Acad. Sci. USA 96, 11054-11061, 1999; Takeuchi et al.; J. Biol. Chem. 275, 26333-26342, 2000) besitzen die gleiche cDNA. Allerdings ist aufgrund alternativen Splicings die Proteinsequenz der Matriptase im Vergleich zur MT-SP1 um 172 Aminosäuren am N-Terminus verkürzt. Das Gen für MT-SP1 wurde aus einer epithelialen Zelllinie eines Prostatatumors isoliert.

Im Rahmen der vorliegenden Erfindung bezeichnet der Begriff "Matriptase" jedes trypsinartige Protein mit einem Molekulargewicht von 72 bis 92 kDa, das von den Gensequenzen mit den Eintragsnummer AF118224, F133086, BANKIt257050 und NM021978 (GenBank/EBI Data Bank) abgeleitet ist und bereits früher beschrieben wurde (Takeuchi et al., Proc. Natl. Acad. Sci. USA 96, 11054-11061, 1999; Lin et al., J. Biol. Chem. 274, 18231-18236, 1999). Insbesondere bezeichnet der Begriff "Matriptase" sowohl die Einzel- und Doppelkettenform des Proteins. Die zymogene, inaktive Form der Matriptase ist ein Einzelkettenprotein. Die Doppelkettenform der Matriptase ist ihre aktive Form, die katalytische Aktivität aufweist. Inbesondere bezeichnet daher im Rahmen der vorliegenden Erfindung der Begriff "Matriptase" die weiter oben beschriebene ursprüngliche Matriptase als auch MT-SP1.

Das Enzym ist mittels einer Transmembran-Domäne in der Membran von Epithel- oder Krebszellen verankert, wobei die Serinproteasedomäne der Matriptase auf der Zelloberfläche und damit im extrazellulären Raum lokalisiert ist (Hopper et al., J. Biol. Chem. 276, 857-860, 2001). Deshalb wurde vermutet, dass Matriptase an der Proliferation und Metastasierung von Brustkrebszellen durch Ab- bzw. Umbau extrazellulärer Matrixproteine, der Aktivierung von latenten Wachstumsfaktoren und anderen proteolytischen Kaskaden beteiligt sein könnte (Shi et al., Cancer Res. 53, 1409-1415, 1993; Lin et al., J. Biol. Chem. 272, 9147-9152, 1997).

Matriptase konnte auch aus menschlicher Milch isoliert werden, doch lag sie in diesem Fall fast vollständig als proteolytisch inaktiver Komplex mit dem endogenen Inhibitor HAI-1 vor (Lin et al., J. Biol. Chem. 274,18237-18242, 1999). Im Gegensatz dazu liegt die Matriptase aus Brustkrebszellen weitestgehend in unkomplexierter und dadurch katalytisch wirksamer Form vor, und nur ein geringer Teil ist an HAI-1 gebunden

Inzwischen wurden erste potentielle Substrate der Matriptase beschrieben. Matriptase ist in der Lage, den Hepatocyten-Wachstumsfaktor (HGF) zu aktivieren, der auch als Scattering-Faktor bezeichnet wird (Lee et al., J. Biol. Chem. 275, 36720-36725, 2000). Pro-HGF wird von Krebs- oder Stromazellen in inaktiver Form als einkettiges Protein sekretiert und wird im extrazellulären Raum durch Spaltung C-terminal des Arg495 in die aktive zweikettige Form (HGF) umgewandelt. Durch die Bindung von HGF wird der Zelloberflächenrezeptor c-Met aktiviert und an bestimmten Tyrosinresten phosphoryliert. Kürzlich wurde eine enge Korrelation zwischen einer hohen Expression von c-Met, Matriptase und HAI-1 und einer schlechten Prognose bei Brustkrebspatienten nachgewiesen (Kang et al., Cancer Res. 63, 1101-1105, 2003). Auch bei der Untersuchung von Ovarialtumoren konnte gezeigt werden, dass Matriptase in verstärktem Maße exprimiert wird. Dabei wurde gefunden, dass im Gegensatz zu Tumoren des Typs I/II vor allem in fortgeschrittenen Tumoren des Typs III/IV Matriptase fast ausschließlich ohne HAI-1 exprimiert wird. Dies deutet daraufhin, dass im fortgeschrittenen Stadium ein Ungleichgewicht zwischen Matriptase und dem Inhibitor HAI-1 besteht, wodurch die proteolytische Aktivität der Matriptase und dadurch wahrscheinlich auch das invasive Potential der Tumorzellen verstärkt wird (Oberst et al., Clin. Cancer Res. 8, 1101-1107, 2002).

Neben der Aktivierung von Pro-HGF ist Matriptase möglicherweise auch an der Aktivierung der Plasminogenaktivator-Kaskade beteiligt. So ist Matriptase in der Lage, Pro-Urokinase zu Urokinase (uPA) zu aktivieren (Lee et al., J. Biol. Chem. 275, 36720-36725, 2000; Takeuchi et al., J. Biol. Chem. 275, 26333-26342, 2000), die Plasminogen in Plasmin umwandelt. Plasmin ist der prinzipielle Aktivator der Matrix-Metalloproteasen, die am Abbau extrazellulärer. Matrixproteine beteiligt sind, was als Voraussetzung der Metastasierung angesehen wird.

Von Ihara et al. (J. Biol. Chem. 277, 16960-16967, 2002) konnte gezeigt werden, dass Magenkrebszellen verstärkt β1-6-N-Acetylglucosaminyltransferase (GnT-V) exprimieren, die in der Lage ist, Matriptase zu glycosilieren. Durch diese Modifizierung wird die Matriptase, abbaustabiler und liegt in erhöhter Konzentration in proteolytisch aktiver Form vor.

Aus diesen Befunden kann abgeleitet werden, dass mit der Entwicklung eines wirksamen und selektiven Hemmstoffs der Matriptase eine Möglichkeit besteht, die Proliferation von Tumoren und ihre Metastasierung zu hemmen. Obwohl inzwischen auch die Röntgenstruktur der katalytischen Domäne der Matriptase im Komplex mit Benzamidin und dem Rinderpankreas-Trypsininhibitor aufgeklärt werden konnte, sind bisher allerdings erst wenige Hemmstoffe der Matriptase bekannt (Friedrich et al., J. Biol. Chem. 277, 2160-2168, 2002).

Von Enyedy et al. (J. Med. Chem. 44, 1349-1355, 2001) wurden Bis-Benzamidine beschrieben, wobei der wirksamste Inhibitor einen Kᵢ-Wert von 0,19 µM aufweist.

Die WO 01/97794 beschreibt ein Verfahren zur Hemmung einer Carcinomprogression bei der Matriptase eine Rolle spielt. Dabei werden Verbindungen eingesetzt, die zwei Gruppen enthalten, die in der Lage sind, bei einem physiologischen pH-Wert positiv geladen zu sein. Dabei sind diese Gruppen durch eine chemische Struktureinheit miteinander verbunden, die eine Länge von 5 bis 30, bevorzugt 15 bis 24 Angstrom aufweist. Als positiv geladene Gruppen werden die Amino-, Amidino-, Guanidinogruppe sowie eine aus der Amidino- bzw. Guanidinogruppe abgeleitete cyclische Gruppe offenbart. Aminosäurederivate sind in der WO 01/97794 nicht erwähnt, insbesondere demgemäß keine sulfonylierten Aminosäurederivate. Vielmehr unterscheiden sich die in der WO . 01/97794 explizit offenbarten Verbindungen fundamental von den im Rahmen der vorliegenden Erfindung beanspruchten Verbindungen.

In WO 02/20475 sind Tripeptidaldehyde mit C-terminalem Arginal veröffentlicht. Nach Vorinkubation von Matriptase mit diesen Inhibitoren über einen Zeitraum von 30 Minuten wurden IC₅₀-Werte kleiner 100 nM für die wirksamsten Verbindungen bestimmt, exakte Hemmkonstanten wurden allerdings nicht angegeben. Diese Inhibitoren binden vermutlich kovalent unter Ausbildung eines Halbacetals an Matriptase. Im Falle der Entwicklung von Hemmstoffen für andere trypsinartige Serinproteasen, wie z.B. Thrombin oder Faktor Xa, wurde jedoch gezeigt, dass solche übergangszustandsanalo-, gen Peptidaldehyde für die Entwicklung eines medizinisch einsetzbaren Wirkstoffs nicht geeignet sind.

Von Long et al. (Bioorganic. Med. Chem. Lett. 11, 2515-2519, 2001) wurde die Synthese eines bizyklischen Peptides aus 14 Aminosäuren beschrieben, das ursprünglich aus dem Samen von Sonnenblumen isoliert wurde. Das Peptid inhibiert Matriptase mit einer Hemmkonstante von 0,92 nM, doch ist davon auszugehen, dass diese Strukturen nicht für die Entwicklung eines Wirkstoffs geeignet sind.

Eine der der vorliegenden Erfindung zugrunde liegenden Aufgaben war es daher, einen auch für therapeutische Anwendungen geeigneten Wirkstoff bereitzustellen, der Matriptase mit hoher Aktivität und Spezifität hemmt.

Demgemäß betrifft die vorliegende Erfindung eine Verbindung gemäß Formel (I) 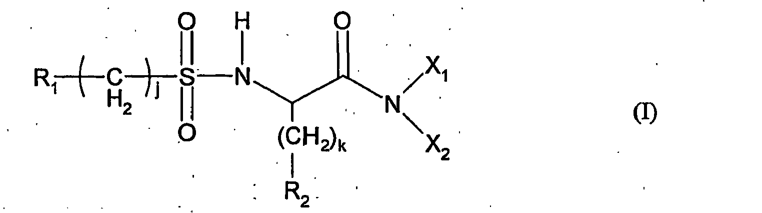 oder ein Salz oder ein Prodrug dieser Verbindung, wobei
(a) X₁ und X₂ unabhängig voneinander Wasserstoff oder ein Alkylrest mit 1, 2 oder 3 C-Atomen sind und mindestens einer der Reste X₁ und X₂ ein Rest der Struktur (I') 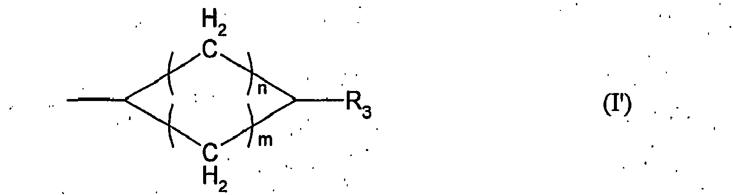 ist, wobei
   g egebenenfalls mindestens eine der mit m oder n indizierten Methylengruppen gemäß (I') mindestens einfach mit einer Hydroxyl-, einer Halogen-, einer Pseudohalogen- oder einer COOR₂'-Gruppe substituiert ist und R₂' eine lineare, verzweigte oder cyclische Alkylgruppe mit 1 bis 10 C-Atomen ist, und/oder
   g egebenenfalls mindestens eines der C-Atome der mit m oder n indizierten Methylengruppen gemäß (I') durch S, N oder 0 ersetzt ist und/oder
   g egebenenfalls mindestens eine der den Cyclus 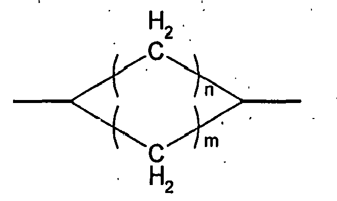 gemäß (I') bildenden Bindungen eine Doppelbindung ist, oder wobei
(b) X₁ und X₂ derart zu einem Cyclus verbrückt sind, dass die Verbindung gemäß (I) die Struktur (I") 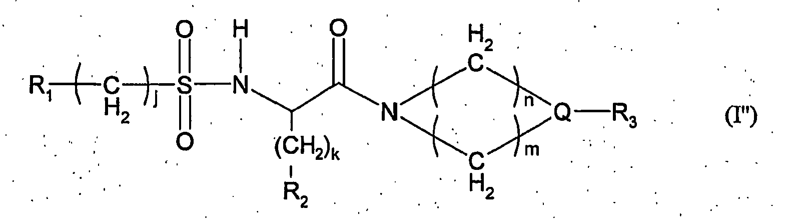 aufweist, wobei
   - gegebenenfalls mindestens eine der mit m oder n indizierten Methylengruppen gemäß (I") mindestens einfach mit einer Hydroxyl-, einer Halogen-, einer Pseudohalogen- oder einer COOR2'-Gruppe substituiert ist und R₂' eine lineare, verzweigte oder cyclische Alkylgruppe mit 1 bis 10 C-Atomen ist, und/oder
   - gegebenenfalls mindestens eines der C-Atome der mit m oder n indizierten Methylengruppen gemäß (I") durch S, N oder O ersetzt ist und/oder
   - unter Beibehaltung der C-terminal an die sulfonylierte Aminosäure gebundenen Iminogruppe gegebenenfalls mindestens eine der den Cyclus 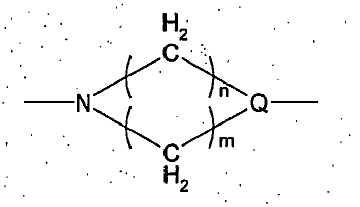 gemäß (I") bildenden Bindungen eine Doppelbindung ist, und wobei
      (i) R₁ eine gegebenenfalls teilweise hydrierte Aryl- oder Heteroarylgruppe, enthaltend mindestens eines der Atome O, N oder S, mit 5 bis 20 C-Atomen, oder eine lineare, verzweigte oder cyclische Alkylgruppe mit 1 bis 10 C-Atomen ist, wobei R₁ gegebenenfalls substituiert ist mit
         - mindestens einer Halogen- und/oder Pseudohalogengruppe und/oder
         - mindestens einer linearen, verzweigten oder cyclischen Alkyl- oder Alkyloxy- oder Alkylthiogruppe mit 1 bis 10 C-Atomen, die gegebenenfalls mindestens einfach substituiert ist mit einer Halogen-, Pseudohalogen-, Hydroxy-, Amino-, Cyano-, Amidino-, Guanidino oder Carboxylgruppe, wobei die Carboxylgruppe gegebenenfalls mit einer linearen, verzweigten oder cyclischen Alkylgruppe mit 1 bis 10 C-Atomen verestert ist, und wobei die lineare, verzweigte oder cyclische Alkylgruppe mit 1 bis 10 C-Atomen gegebenenfalls mindestens ein Heteroatom, ausgewählt aus der Gruppe, bestehend aus O, N und S, enthält, und/oder
         - mindestens einer Aryl- oder Heteroarylgruppe mit 5 bis 20 C-Atomen, wobei diese Aryl- oder Heteroarylgruppe gegebenenfalls substituiert ist mit
         - mindestens einer linearen, verzweigten oder cyclischen Alkylgruppe mit 1 bis 10 C-Atomen und/oder
            -- mindestens einer COR₂'- und/oder COOR₂'-Gruppe, wobei R₂' eine lineare, verzweigte oder cyclische Alkylgruppe mit 1 bis 10 C-Atomen ist, und/oder
            -- mindestens einer Halogengruppe und/oder
            -- mindestens einer Pseudohalogengruppe und/oder
            -- mindestens einer Alkoxygruppe oder einer Alkylthiogruppe, wobei der Alkylrest jeweils 1 bis 10 C-Atome aufweist, und/oder
            -- mindestens einer Nitrogruppe und/oder
            -- mindestens einer Halogenalkylgruppe mit 1 bis 10 C-Atomen, und wobei die Aryl- oder Heteroarylgruppe über eine Alkylengruppe mit 1 bis 3 C-Atomen oder ein Sauerstoffatom oder über ein Schwefelatom an den Rest R₁ gebunden ist;
         - mindestens einer Hydroxy-, Amino-, Cyano-, Amidino-, Guanidino-, Carboxyl- oder Carboxyalkylgruppe, wobei die Aminogruppe gegebenenfalls acyliert ist und/oder wobei die Alkylgruppe der Carboxyalkylgruppe 1 bis 10 C-Atome aufweist und/oder die Carboxylgruppe gegebenenfalls mit einer linearen, verzweigten oder cyclischen Alkylgruppe mit 1 bis 10 C-Atomen verestert oder amidiert ist;
      (ii) R₂ eine mindestens einfach substituierte Arylgruppe mit 1 bis 10 C-Atomen ist, wobei
         - gegebenenfalls mindestens eines dieser C-Atome durch S, N oder O versetzt ist,
         - mindestens ein Substituent eine Gruppe gemäß R₄ ist,
         - R₂ gegebenenfalls zusätzlich mit einer Hydroxy-, COR₂'- oder COOR₂'-Gruppe substituiert ist und R₂' eine lineare, verzweigte oder cyclische Alkylgruppe mit 1 bis 10 C-Atomen ist;
      (iii) R₃ einen Rest der folgenden Formel (II) darstellt: 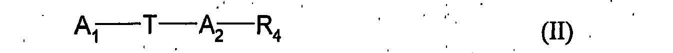 wobei
         - A₁ entweder nicht vorhanden oder eine Alkylengruppe mit 1 bis 4 C-Atomen ist, die gegebenenfalls substituiert ist mit
            -- mindestens einer Halogen- und/oder Pseudohalogengruppe und/oder
            -- mindestens einer linearen, verzweigten oder cyclischen Alkylgruppe mit 1 bis 10 C-Atomen und/oder
            -- mindestens einer Aryl- oder einer Aralkylgruppe mit 5 bis 10 C-Atomen und/oder
            -- mindestens einer Cycloalkylgruppe mit 3 bis 10-Atomenund/oder-
            -- mindestens einer Hydroxy-, Cyano-, Alkyloxy- oder Alkylthio mit 1 bis 10 C-Atomen, Carboxyl- oder Carboxyalkylgruppe, wobei die Alkylgruppe der Carboxyalkylgruppe 1 bis 10 C-Atome aufweist und/oder die Carboxylgruppe gegebenenfalls mit einem linearen, verzweigen oder cyclischen Alkylrest mit 1 bis 10 C-Atomen verestert oder amidiert ist;
         - T entweder nicht vorhanden oder eine der folgenden Gruppen ist: 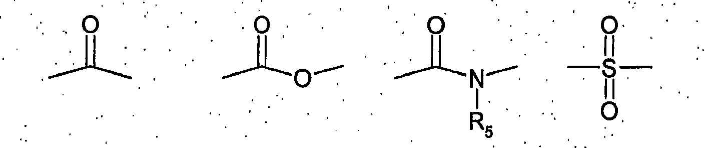 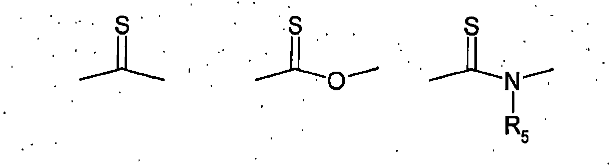 wobei R₅ Wasserstoff oder eine Alkylgruppe mit 1 bis 10 C-Atomen oder eine mit A₂ einen gegebenenfalls mindestens ein Heteroatom enthaltenden Cyclus bildende Alkylengruppe mit 1 bis 6 C-Atomen ist;
            wobei die Amid- oder Esterbindung in beiden Orientierungen eingebaut sein kann, also auch die folgenden Orientierungen mit umfasst sind: 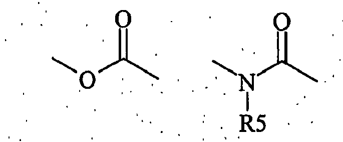 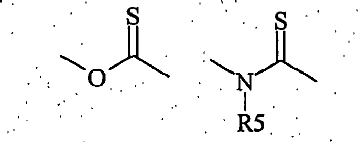
         - A₂ eine lineare, verzweigte oder cyclische Alkylengruppe mit 1 bis 10 C-Atomen oder eine Aryl-, Heteroaryl- oder Aralkylengruppe mit 1 bis 10 C-Atomen, gegebenenfalls enthaltend mindestens ein Heteroatom, ausgewählt aus der Gruppe bestehend aus N, S und O, ist, die gegebenenfalls substituiert ist mit
            -- mindestens einer Halogen-und/oder Pseudohalogengruppe und/oder
            -- mindestens einer linearen, verzweigten oder cyclischen Alkylgruppe mit 1 bis 10 C-Atomen und/oder
            -- mindestens einer Aryl- oder einer Aralkylgruppe mit 5 bis 10 C-Atomen und/oder
            -- mindestens einer Cycloalkylgruppe mit 3 bis 10 C-Atomen und/oder-
            -- mindestens einer Hydroxy-, Cyano-, Alkyloxy- oder Alkylthio- mit 1 bis 10 C-Atomen, Carboxyl- oder Carboxyalkylgruppe, wobei die Alkylgruppe der Carboxyalkylgruppe 1 bis 10 C-Atome aufweist und/oder die Carboxylgruppe gegebenenfalls mit einem linearen, verzweigen oder cyclischen Alkylrest mit 1 bis 10 C-Atomen verestert oder amidiert ist;
      (iv) R₄ eine der folgenden, gegebenenfalls modifizierten basischen Gruppen ist: 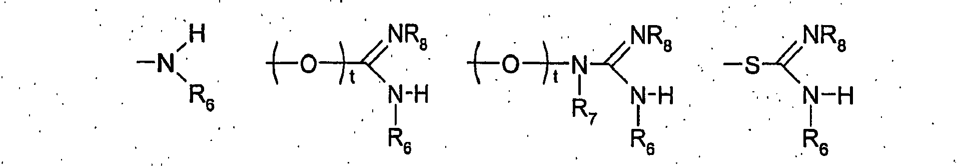 wobei t = 0, 1; R₆ und R₇ unabhängig voneinander Wasserstoff oder eine Alkylgruppe mit 1 bis 6 C-Atomen oder eine Alkylengruppe mit 1 bis 5 C-Atomen, die mit A₂ einen Cyclus bildet, oder eine Hydroxyl-, Amino-, Alkylamino, Acyl- oder Alkyloxycarbonylgruppe sind, wobei die Alkylamino-, Acyl- und Alkyloxy-carbonylgruppen unabhängig voneinander 1 bis 6 C-Atome aufweisen, und wobei R₈ Wasserstoff oder eine Alkylgruppe mit 1 bis 3 C-Atomen ist oder eine Alky-lengruppe mit 1 bis 3 C-Atomen ist, die mit R₆ einen Cyclus bildet;
      (v), Q entweder eine CH-Gruppe oder N ist;
      (vi) j = 0, 1, 2;
         k = 0, 1, 2, 3;
         m, n unabhängig voneinander = 0, 1, 2, 3,4, 5 sind, wobei m + n = 3, 4, 5;
         und wobei die Verbindung gemäß Formel,(I) weder 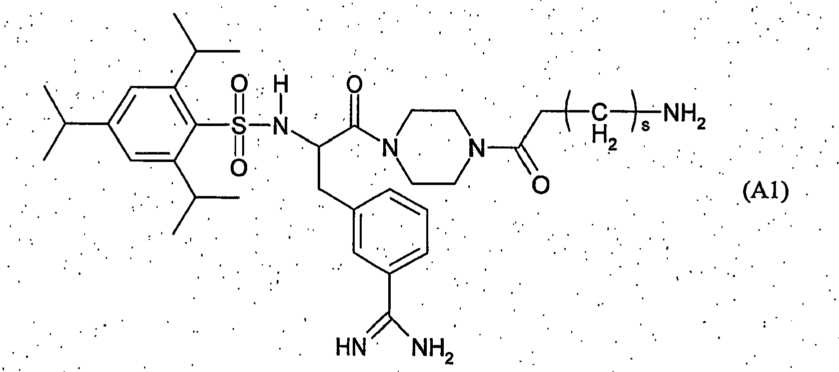 mit s = 0, 1, 2 noch 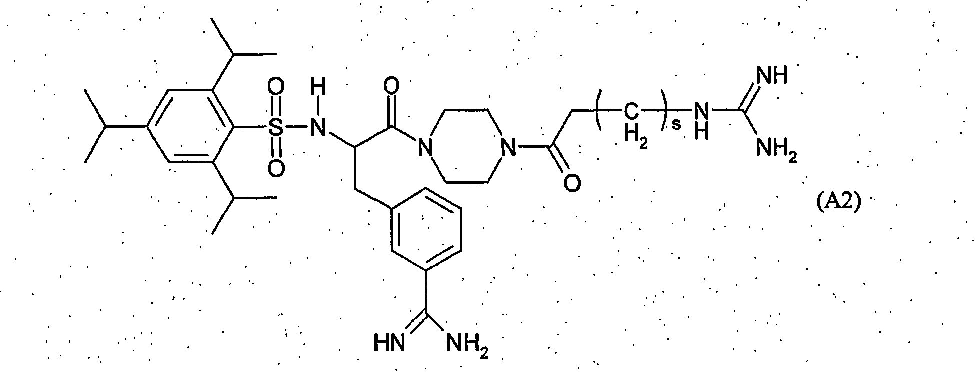 mit s = 0, 1 ist.

Liegen die oben genannten erfindungsgemäßen Verbindungen als Salz vor, so sind Salze mit Mineralsäuren und/oder Salze mit geeigneten organischen Säuren bevorzugt. Unter anderem bevorzugt liegen die erfindungsgemäßen Verbindungen als Hydrochloride oder auch als Sulfate vor. Geeignete organische Säuren sind beispielsweise Ameisensäure, Essigsäure, Methylsulfonsäure, Bernsteinsäure; Äpfelsäure und Trifluoressigsäure. Unter anderem bevorzugt sind als Salze der erfindungsgemäßen Verbindungen mit geeigneten organischen Säuren Acetate.

Gemäß einer bevorzugten Ausführungsform weist die erfindungsgemäße Verbindung eine Struktur auf, bei der mindestens einer der Reste X₁ und X₂ eine Struktur (I') aufweist. Im Rahmen dieser Ausführungsform der an die Carbonylgruppe der gemäß (I) mittelständigen Aminosäure gebundenen, nicht-cyclischen Imine sind Verbindungen bevorzugt, bei denen genau einer der Reste X₁ und X₂ eine Struktur gemäß (I') aufweist. Der nicht-cyclische Rest ist hierbei besonders bevorzugt Wasserstoff, Methyl, Ethyl oder n-Propyl, weiter bevorzugt Methyl oder Ethyl und besonders bevorzugt Methyl. Was den substituierten cyclischen Rest anbelangt, so sind Ausruhrungsformen bevorzugt, bei denen m + n gleich 3 oder 4 ist. Der in Rest R₃ zwingend enthaltene Rest R₄ kann generell im Rahmen der oben getroffenen Definitionen beliebig gewählt werden. Ganz besonders bevorzugt sind solche Reste R₄, die nicht modifiziert sind und die ausgewählt werden aus der Gruppe, bestehend aus

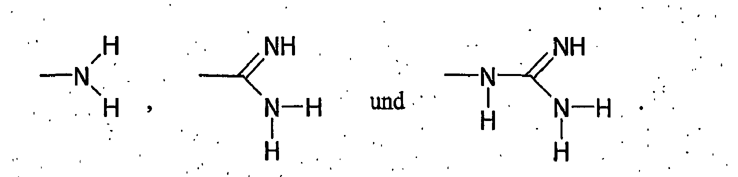

Die Indices m und n des Cyclus gemäß (I') können so gewählt werden, dass sich der Rest R₃ grundsätzlich in 2-, 3- oder, je nach Ringgröße, auch in 4-Position bezüglich des an die Carbonylgruppe der mittelständigen Aminosäure gebundenen Stickstoffs befinden kann. Bevorzugt sind beispielsweise die 3- oder die 4-Position, bei m=n=2 besonders bevorzugt die 4-Position.

Im Rahmen der Ausführungsform der an die Carbonylgruppe der gemäß (I) mittelständigen Aminosäure gebundenen, nicht-cyclischen Imine sind weiter Reste R₃ bevorzugt, bei denen A₂ nicht vorhanden ist. Weiter bevorzugt sind Reste R₃, bei denen die funktionelle Gruppe T entweder nicht vorhanden ist oder ausgewählt wird aus

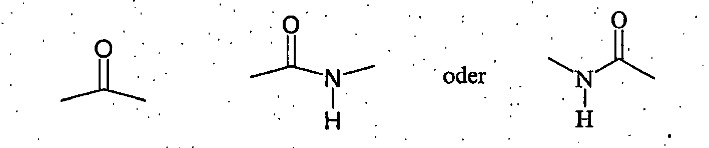

Ganz besonders bevorzugt sind Ausführungsformen, bei denen T nicht vorhanden ist. Was die Gruppe A₂ anbelangt, so sind im Rahmen der an die Carbonylgruppe der gemäß (I) mittelständigen Aminosäure gebundenen, nicht-cyclischen Imine Alkylengruppen mit 1, 2, 3, 4 oder 5 C-Atomen, insbesondere die Methylen-, Ethylen, n-Propylen, Isopropylen, Butylen und Pentylengruppe bevorzugt.

Im Falle, dass A₂ eine Aryl-, Heteroaryl- oder Arälkylengruppe ist, sind beispielsweise Gruppen der Strukturen 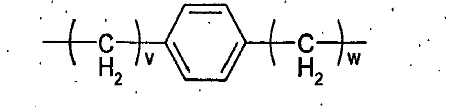 zu nennen, wobei v und w unabhängig voneinander 0, 1 oder 2 sein können und die beiden Alkylengruppen auch in 1,2- oder 1,3-Stellung zueinander positioniert sein können. Beispielweise bevorzugt ist die 1,4-Stellung. Sowohl der Arylrest als auch mindestens eine der beiden Alkylengruppen können wie oben definiert geeignet substituiert sein. Umfasst A₂ eine Heteroarylgruppe, so weist diese bevorzugt 1 bis 3 Heteroatome auf.

Grundsätzlich kann der Cyclus gemäß (I') mindestens ein Heteroatom enthalten, wobei hierbei bevorzugt Sauerstoff, Stickstoff oder Schwefel zu nennen sind. Ist das Hetero-atom beispielsweise Stickstoff, so kann dieser Stickstoff als weiteren Rest beispielsweise Wasserstoff oder eine lineare, verzweigte oder cyclische Alkylgruppe mit 1 bis 10 C-Atomen aufweisen oder mit einem benachbarten C-Atom des Cyclus eine Doppelbindung ausbilden. Gemäß einer besonders bevorzugten Ausführungsform ist keine der Methylengruppen gemäß (I') durch ein Heteroatom substituiert.

Ist der Cyclus gemäß (I') zusätzlich substituiert, so sind als zusätzliche Substituenten unter anderem Carboxyalkylgruppem der allgemeinen Struktur -COOR₂' bevorzugt, wobei weiter bevorzugt R₂' eine Alkylgruppe mit 1, 2 oder 3 C-Atomen und insbesondere bevorzugt eine Methyl- oder Ethylgruppe ist.

Gemäß einer bevorzugten Ausführungsform weisen die erfindungsgemäßen Verbindungen gemäß (b) ein an die Carbonylgruppe der gemäß (I) mittelständigen Aminosäure gebundenes cyclisches Imin und somit eine Struktur gemäß (I") auf.

Demgemäß betrifft die vorliegende Erfindung auch eine Verbindung, wie oben beschrieben, wobei diese Verbindung die Struktur (I") 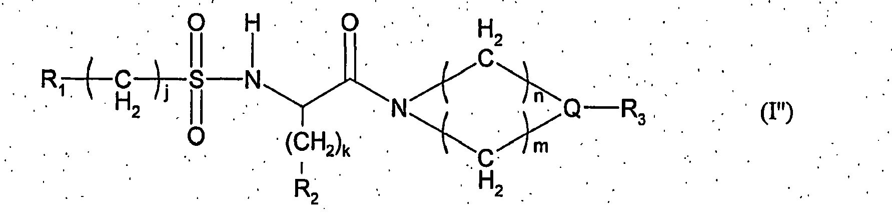 aufweist.

Der Cyclus gemäß (I") weist diesbezüglich bevorzugt 5, 6 oder 7 Ringatome auf. Demgemäß ist es denkbar, dass sich der Rest R₃ in 2-, 3- oder 4-Stellung zu dem an die Carbonylgruppe der mittelständigen Aminosäure gebundenen Imin-Stickstoff befindet. Unter anderem bevorzugt sind Ausführungsformen, gemäß denen der Cyclus des cyclischen Amins 5 oder 6 Ringatome aufweist. Besonders bevorzugt ist ein Sechsring. Gemäß dieses besonders bevorzugten Sechsrings können die Indices m und n beliebig gewählt werden. Beispiele für die möglichen Kombinationen sind etwa m=0 und n=4, m=1 und n=3, m=2 und n=2, m=3 und n=1, m=4 und n=0. Gemäß einer ganz besonderes bevorzugten Ausführungsform der erfindungsgemäßen Verbindungen ist m=n=2. Die Gruppe Q. gemäß der Struktur (I) befindet sich also ganz besonders bevorzugt in 4-Stellung zu dem an die Carbonylgruppe der mittelständigen Aminosäure gebundenen Imin-Stickstoff.

Demgemäß betrifft die vorliegende Erfindung auch eine Verbindung, wie oben beschieben, die dadurch gekennzeichnet ist, dass m = n = 2.

Im Rahmen der vorliegenden Erfindung kann der Cyclus des cyclische Imins geeignet substituiert sein. Unter den oben beschriebenen Substituenten ist unter anderem die COOR₂'-Gruppe' besonders bevorzugt, wobei R₂' wiederum bevorzugt eine Alkylgruppe mit 1, 2, 3, 4, 5 oder 6 C-Atomen und insbesondere bevorzugt eine Methylgruppe oder eine Ethylgruppe ist. Ist der Cyclus mit einem Halogen substituiert, so sind Fluor, Chlor und Brom besonders bevorzugt. Ebenso ist die Hydroxylgruppe ein geeignete Substituent. Weiter kann der Cyclus mit zwei oder mehr gleichen oder verschiedenen, insbesondere den als bevorzugt genannten Substituenten substituiert sein.

Mindestens eine der im Cyclus gemäß (I") mit m oder n indizierten Methylengruppe kann durch ein Heteroatom substituiert sein, wobei hierbei bevorzugt Sauerstoff, Stickstoff oder Schwefel zu nennen sind. Ist das Heteroatom beispielsweise Stickstoff, so kann dieser Stickstoff als weiteren Rest beispielsweise .Wasserstoff oder eine lineare, verzweigte oder cyclische Alkylgruppe mit 1 bis 10 C-Atomen aufweisen oder mit einem benachbarten C-Atom des Cyclus eine Doppelbindung ausbilden. Ganz allgemein kann der Cyclus gemäß (1") mindestens eine Doppelbindung enthalten, die entweder zwischen zwei Heteroatomen, zwei C-Atomen.oder einem C-Atom und einem Hetero-atom ausgebildet sein kann. Im Rahmen der vorliegenden Erfindung sind demgemäß Cyclen beschrieben, die mindestens eine Doppelbindung aufweisen und bei denen der an die Carbonylgruppe der mittelständigen Aminosäure gebundene Iminstickstoff beibehalten wird, also mit drei Einfachbindungen an die benachbarten Atome gebunden ist.

Gemäß einer besonders bevorzugten Ausführungsform ist keine der Methylengruppen gemäß (I") durch ein Heteroatom substituiert. Gemäß einer weiter insbesondere bevorzugten Ausführungsform sind die den Cyclus aufbauenden Methylengruppen nicht substituiert.

Demgemäß betrifft die vorliegende Erfindung auch eine Verbindung, wie oben beschrieben, wobei der durch X₁ und X₂ gebildete Cyclus folgende Struktur aufweist 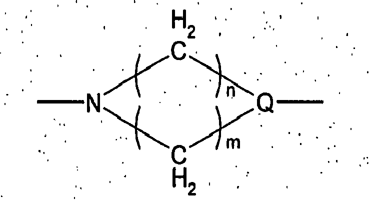 und m, n unabhängig voneinander = 0, 1, 2, 3, 4, 5 sind, wobei m + n = 3, 4, 5. Weiter bevorzugt sind dabei Verbindungen, bei denen m und n so gewählt sind, dass ein Sechsring gebildet wird. Hierbei kann sich Q demgemäß, wie bereits oben beschrieben, in 2-, 3- oder 4-Stellung zum Imin-Stickstoff befinden, wobei besonders bevorzugt die 4-Stellung ist Weiter insbesondere bevorzugt ist m =n=2.

Im Rahmen der vorliegenden Erfindung ist Q eine CH-Gruppe oder Stickstoff. Ebenso beschreibt die vorliegende Erfindung auch Verbindungen, bei denen diese CH-Gruppe statt Wasserstoff einen geeigneten Substituenten trägt. Als Substituenten sind unter anderem bevorzugt solche zu nennen, mit denen auch die Methylengruppen des Cyclus substituiert sein können.

Demgemäß beschreibt die vorliegende Erfindung auch eine Verbindung, wie oben beschrieben, die dadurch gekennzeichnet ist, dass Q ein Stickstoffatom ist.

Im Rahmen dieser Ausführungsform kann die in dem Rest R₃ gegebenenfalls enthaltene Gruppe A₁ im Rahmen der oben beschriebenen Definitionen beliebig gewählt werden. Besonders bevorzugt ist eine Gruppe A₁, die eine Methylen-öder Ethylen- oder Propylengruppe, insbesondere eine Methylen- oder Ethylengruppe ist und gegebenenfalls substituiert ist, wobei als Substituenten unter anderem besonders bevorzugt Alkylreste mit 1, 2 oder 3-Atomen, insbesondere Methyl und Ethyl, besonders bevorzugt Methyl, und/oder Halogen, insbesondere Fluor, Chlor und Brom, und/oder Cycloalkylreste mit bevorzugt 5, 6 oder 7 C-Atomen und/oder Carboxyalkylreste, wobei der Alkylrest bevorzugt Methyl oder Ethyl ist und die Carboxylgruppe bevorzugt mit einer Methyl- oder Ethylgruppe verstert oder amidiert ist, sind.

Gemäß einer ganz besonders bevorzugten Ausführungsform der erfindungsgemäßen Verbindungen, bei denen Q ein Stickstoffatom ist, ist die Gruppe A₁ nicht vorhanden.

Demgemäß beschreibt die vorliegende_ Erfindung auch eine Verbindung, wie oben beschrieben, die dadurch gekennzeichnet ist, dass Q ein Stickstoffatom ist und nicht vorhanden ist.

Hinsichtlich der im Rahmen der vorliegenden Erfindung in, Rest R₃ gegebenenfalls engthaltenen funktionellen Gruppe T können sämtliche der oben beschriebenen Gruppen gewählt werden. Insbesondere im Fall, dass Q ein Stickstoffatom ist, sind als fünktionelle Gruppen T Gruppen der Strukturen 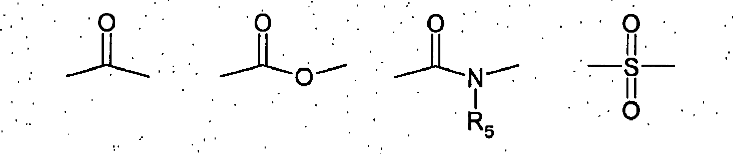 bevorzugt. Besonders bevorzugt sind für T Gruppen der Strukturen 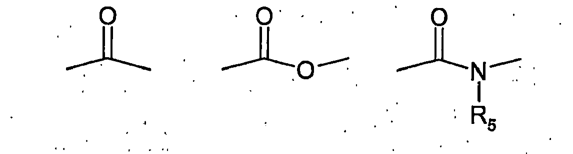 wobei weiter bevorzugt, R₅ Wasserstoff ist. Gemäß weiter besonders bevorzugter Ausführungsformen ist T ein Gruppe der Strukturen 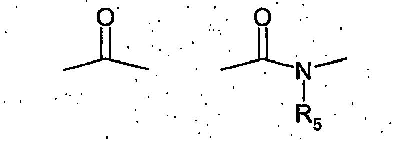 wobei ganz besonders bevorzugt T eine funktionelle Gruppe der Struktur 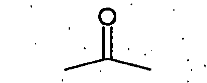 ist. Hierbei können die Amid- oder Estergruppen in beiden Orientierungen eingebaut sein.

Demgemäß beschreibt die vorliegende Erfindung auch eine Verbindung, wie oben beschrieben, die dadurch gekennzeichnet ist, dass Q ein Stickstoffatom ist und T eine fw1ktionelle Gruppe der Struktur

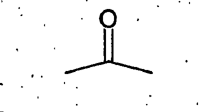

Was die im Rest R₃. zwingend vorhandene Gruppe, A₂ anbelangt, so gibt es innerhalb der oben beschriebenen Definitionen keine Einschränkungen.

Insbesondere im Fall, dass Q ein Stickstoffatom ist und weiter insbesondere, dass T eine Carbonylgruppe ist, sind für die Gruppe A₂ Alkylengruppen mit 1 bis 6 C-Atomen bevorzugt, die Methylen-, Ethylen-, Propylen-, Butylengruppe besonders bevorzugt und die Methylen-, Ethylen- und Propylengruppe insbesondere bevorzugt.

Im Falle, dass A₂ eine Aryl-, Heteroaryl- oder Aralkylengruppe ist, sind beispielsweise Gruppen der Strukturen 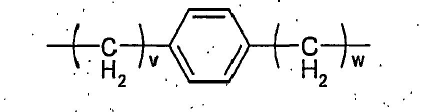 zu nennen, wobei v und w unabhängig voneinander 0, 1 oder 2 sein können und die beiden Alkylengruppen auch in 1,2- oder 1,3-Stelliing zueinander positioniert sein können. Beispielweise bevorzugt ist die 1,4-Stellung. Sowohl der Arylrest als auch mindestens eine der beiden Alkylengruppen können geeignet substituiert sein. Umfasst A₂ eine Heteroarylgruppe, so weist diese bevorzugt bis 3 Heteroatome auf.

Die Gruppe A₂ kann gemäß obiger Definition auch geeignet substituiert sein. Als Substituenten sind hierbei besonders Halogene, bevorzugt Fluor, Chlor oder Brom, und/oder Alkylreste mit bevorzugt 1, 2 oder 3 C-Atomen wie Methyl, Ethyl, n-Propyl oder Isopropyl, insbesondere bevorzugt Methyl und Ethyl, und/oder Cycloalkylreste mit bevorzugt 5, 6 oder 7 C-Atomen und/oder Carboxyalkylreste, wobei der Alkylrest bevorzugt Methyl oder Ethyl ist und die Carboxylgruppe bevorzugt mit einer Methyl- oder Ethylgruppe verestert oder amidiert ist.

Gemäß einer ganz besonders bevorzugten Elusführungsform der vorliegenden Erfindung ist die Gruppe A₂ nicht substituiert. Demgemäß beschreibt die, vorliegende Erfindung eine Verbindung, wie oben beschrieben, die dadurch gekennzeichnet ist, dass A₂ eine Methylen-, Ethylen- oder Propylengruppe ist. Insbesondere beschreibt die vorliegende Erfindung eine Verbindung, wie oben beschrieben, die dadurch gekennzeichnet ist, dass sie folgende Struktur aufweist: 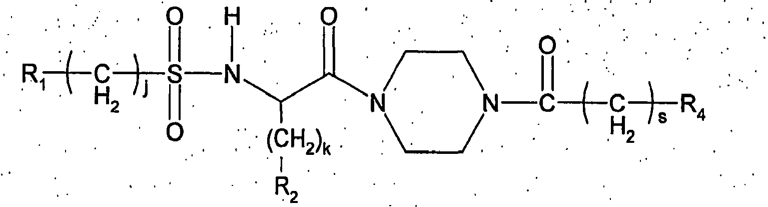 wobei s = 1,2,3.

Gemäß einer ebenfalls bevorzugten Ausführungsform beschreibt die vorliegende Erfindung eine Verbindung, wie oben beschrieben, die dadurch gekennzeichnet ist, dass sie als funktionelle Gruppe T eine Gruppe der Struktur 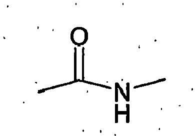 aufweist und damit folgende Struktur aufweist: 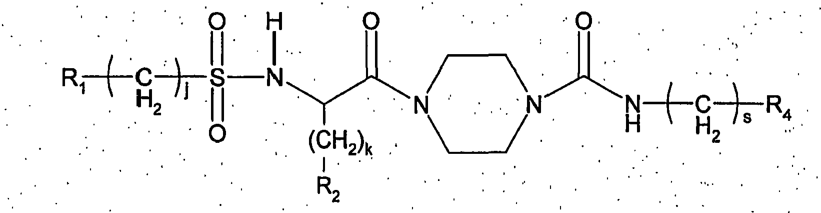 wobei s = 1, 2, 3, bevorzugt s = 2.

Als zwingend in Rest R₃ enthaltener Rest R₄ sind sämtliche der oben genannten Strukturren einsetzbar. Bevorzugt ist dabei t = 0. Insbesondere bevorzugt sind dabei Reste R₄ der folgenden Strukturen: 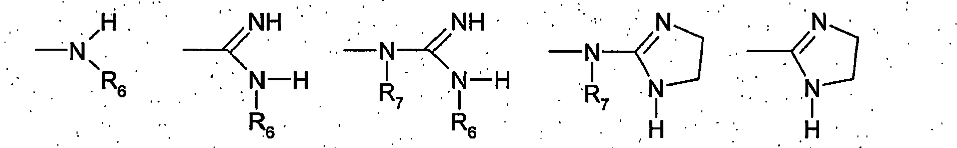 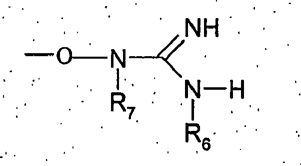 wobei weiter bevorzugt R₆ und R₇ gleich Wasserstoff sind.

Ganz besonders bevorzugt sind Reste R₄ der folgenden Strukturen: 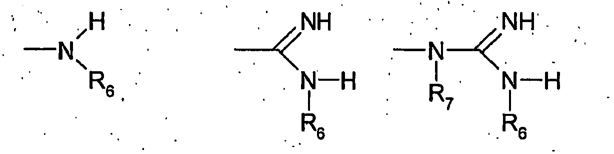 wobei weiter bevorzugt R₆ und R₇ gleich Wasserstoff sind.

Demgemäß betrifft die vorliegende Erfindung auch eine Verbindung, wie oben beschrieben, die dadurch gekennzeichnet ist, dass R₄ ausgewählt wird aus der Gruppe bestehend aus

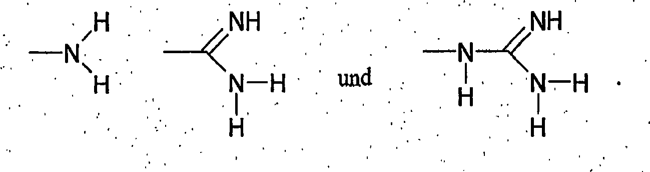

Gemäß einer ebenfalls bevorzugten Ausführungsform der vorliegenden Erfindung ist Q eine CH-Gruppe. Daher beschreibt die vorliegende Erfindung auch Verbindungen, wie oben beschrieben, die dadurch gekennzeichnet sind, dass Q eine CH-Gruppe ist .

Im Rahmen dieser Ausführungsform kann die in dem Rest R₃ gegebenenfalls enthaltene Gruppe A₁ im Rahmen der oben beschriebenen Definitionen beliebig gewählt werden. Besonders bevorzugt ist eine Gruppe A₁, die eine Methylen- oder Ethylen- oder Propylengruppe, insbesondere eine Methylen- oder Ethylengruppe ist und gegebenenfalls substituiert ist, wobei als Substituenten unter anderem besonders bevorzugt Alkylreste mit 1, 2 oder 3-Atomen, insbesondere Methyl und Ethyl, und/oder Halogen, insbesondere Fluor, Chlor und Brom, und/oder Cycloalkylreste mit bevorzugt 5,6 oder 7 C-Atomen und/oder Carboxyalkylreste, wobei der Alkylrest bevorzugt Methyl oder Ethyl ist und die Carboxylgruppe bevorzugt mit einer Methyl- oder Ethylgruppe verestert oder amidiert ist, sind.

Gemäß einer ganz besonders bevorzugten Ausführungsform der erfindungsgemäßen Verbindungen, bei denen Q eine CH-Gruppe ist, ist die Gruppe A₁ nicht vorhanden.

Demgemäß beschreibt die vorliegende Erfindung auch eine Verbindung, wie oben beschrieben, die dadurch gekennzeichnet ist, dass Q eine CH-Gruppe ist und A₁ nicht vorhanden ist:

Hinsichtlich der im Rahmen der vorliegenden Erfindung in Rest R₃ gegebenenfalls enthaltenen funktionellen Gruppe T können sämtliche der oben beschriebenen Gruppen gewählt werden. Insbesondere im Fall, dass Q eine CH-Gruppe ist, sind als funktionelle Gruppen T Gruppen der Strukturen 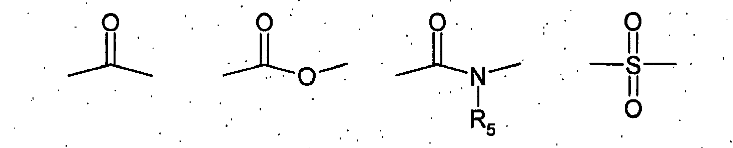 bevorzugt. Besonders bevorzugt sind für T Gruppen der Strukturen 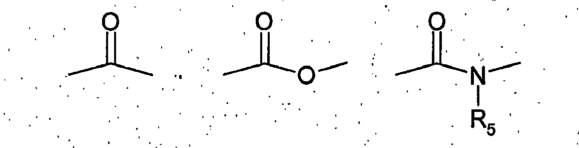 wobei weiter bevorzugt R₅ Wasserstoff ist. Gemäß weiter besonders bevorzugter Ausführungsformen ist T ein Gruppe der Strukturen 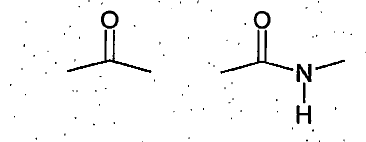 wobei ganz besonders bevorzugt T eine funktionelle Gruppe der Struktur 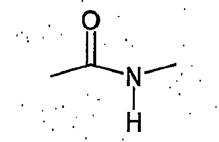 ist. Hierbei können die Amid- oder Estergruppen in beiden Orientierungen eingebaut sein.

Demgemäß beschreibt die vorliegende Erfindung auch eine Verbindung, wie oben beschieben, die dadurch gekennzeichnet ist, dass Q eine CH-Gruppe ist und T eine funktionelle Gruppe der Struktur

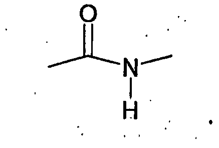

Im Falle, dass Q, eine CH-Gruppe ist und T eine der bevorzugt genannten funktionellen Gruppen ist, ist die Gruppe A₁ gemäß einer besonders bevorzugten Ausführungsform der vorliegenden Erfindung nicht vorhanden.

Daher beschreibt die vorliegende Erfindung auch eine Verbindung, wie oben beschrieben, die folgende Struktur aufweist:

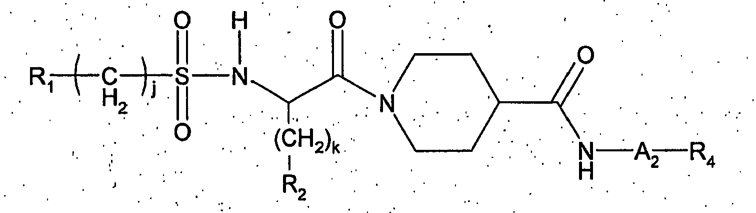

Neben der in oben stehender Strukturformel explizit beschriebenen 4-Stellung des Restes R₃ sind ebenso die 2- oder die 3-Stellung möglich, wobei die 4-Stellung bevorzugt ist.

Gemäß einer ebenfalls bevorzugten Ausführungsform der vorliegenden Erfindung ist weder die Gruppe A₁ noch die funktionelle Gruppe T vorhanden.

Daher beschreibt die vorliegende Erfindung auch ein Verbindung, wie oben beschrieben, die folgende Struktur aufweist:

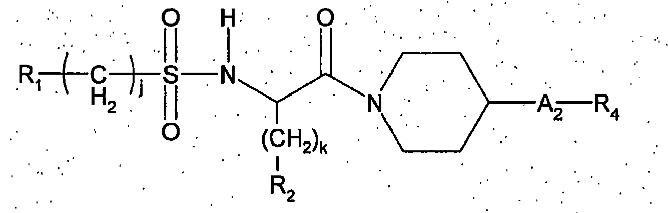

Neben der in oben stehender Strukturformel explizit beschriebenen 4-Stellung des Restes R₃ sind ebenso die 2- oder die 3-Stellung möglich, wobei die 4-Stellung bevorzugt ist.

Was die im Rest R₃ zwingend vorhandene Gruppe A₂ anbelangt, so gibt es, wie bereits oben diskutiert, innerhalb der oben beschriebenen Definitionen keine Einschränkungen.

Insbesondere im Fall, dass Q eine CH-Gruppe ist und weiter insbesondere, dass entweder T eine Gruppe der Struktur 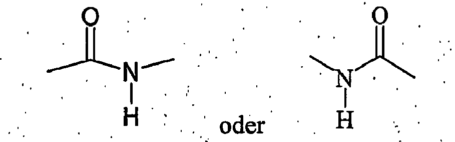 ist oder nicht vorhanden ist, sind für die Gruppe A₂ Alkylengruppen mit 1 bis 6 C-Atomen, insbesondere die Methylen-, Ethylen-, Propylen-, Butylengruppe, weiter insbesondere die Methylen-, Ethylen- und Propylengruppe und ganz besonders die Ethylengruppe bevorzugt.

Im Falle, dass A₂ eine Aryl-, Heteroaryl- oder Aralkylengruppe ist, sind beispielsweise Gruppen der Strukturen 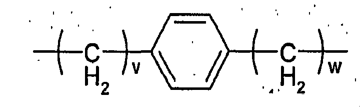 zu nennen, wobei v und w unabhängig voneinander 0, 1 oder 2 sein können und die beiden Alkylengruppen auch in 1,2- oder 1,3-Stellung zueinander positioniert sein können. Beispielweise bevorzugt ist die 1,4-Stellung. Sowohl der Arylrest als auch mindestens eine der beiden Alkylengruppen können geeignet substituiert sein. Umfasst A₂ eine Heteroarylgruppe, so weist diese bevorzugt 1 bis 3 Heteroatome auf.

Die Gruppe A₂ kann gemäß obiger Definition auch geeignet substituiert sein. Als Subsituenten sind hierbei besonders Halogene, bevorzugt Fluor, Chlor oder Brom, und/oder Alkylreste mit bevorzugt 1, 2 oder 3 C-Atomen wie Methyl, Ethyl, n-Propyl oder Isopropyl, insbesondere bevorzugt Methyl und Ethyl, und/oder Cycloalkylreste mit bevorzugt 5, 6 oder 7 C-Atomen und/oder Carboxyalkylreste, wobei der Alkylrest bevorzeugt Methyl oder Ethyl ist und die Carboxylgruppe bevorzugt mit einer Methyl- oder Ethylgruppe verestert oder amidiert ist.

Gemäß einer ganz besonders bevorzugten AusRhrungsform der vorliegenden Erfindung ist die Gruppe A₂ nicht substituiert.

Demgemäß beschreibt die vorliegende Erfindung auch Verbindungen, wie oben beschrieben, die dadurch gekennzeichnet sind, dass sie folgende Struktur aufweisen:

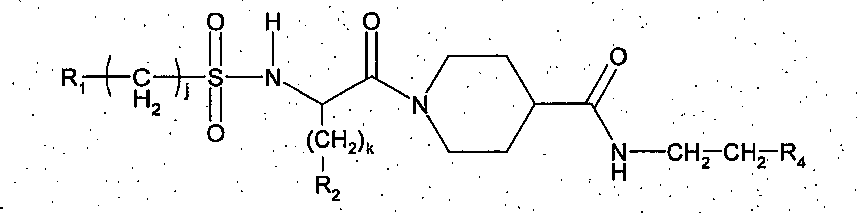

Neben der in oben stehenden Strukturformeln explizit beschriebenen 4-Stellung des Restes R₃ sind ebenso die 2- oder die 3-Stellung möglich, wobei die 4-Stellung bevorzugt ist.

Ebenso beschreibt die vorliegende Erfindung daher auch Verbindungen, wie oben beschrieben, die dadurch gekennzeichnet sind, dass sie folgende Strukturen aufweisen:

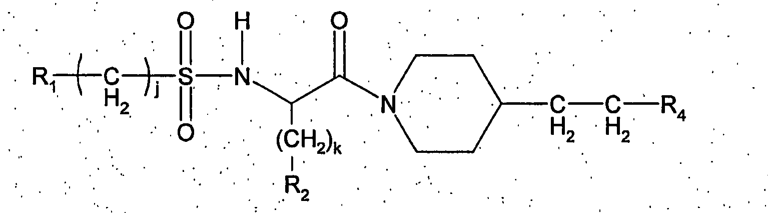

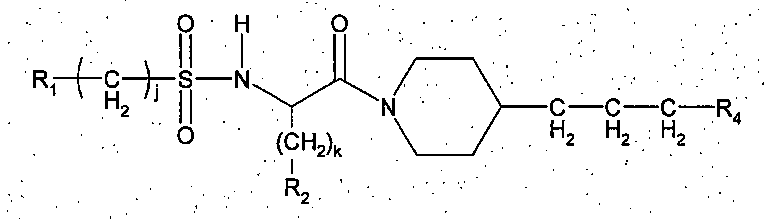

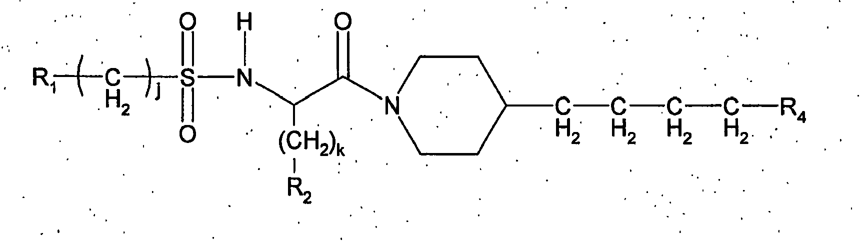

Neben der in oben stehenden Strukturformeln explizit beschriebenen 4-Stellung des Restes R₃ sind ebenso die 2- oder die 3-Stellung möglich, wobei die 4-Stellung bevorzugt ist.

Als zwingend in Rest R₃ enthaltener Rest R₄ sind sämtliche der oben genannten Strukturen einsetzbar. Bevorzugt ist dabei t = 0. Insbesondere bevorzugt sind dabei Reste R₄, der folgenden Strukturen: 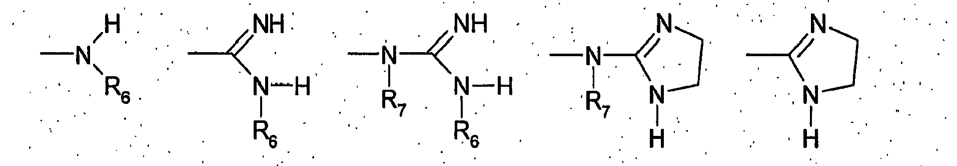 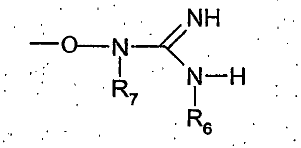 wobei weiter bevorzugte und R₇ gleich Wasserstoff sind.

Ganz besonders bevorzugt sind Reste R₄ der folgenden Strukturen: 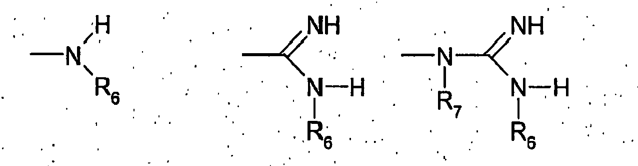 wobei weiter bevorzugt F₆ und R₇ gleich Wasserstoff sind.

Demgemäß betrifft die vorliegende Erfindung auch eine Verbindung, wie oben beschieben, die dadurch gekennzeichnet ist, dass R₄ ausgewählt wird aus der Gruppe bestehend aus

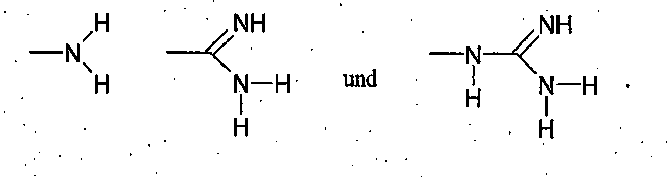

Insbesondere bevorzugt ist im Rahmen der vorliegenden Erfindung der im Rest R₃ enthaltene Rest R4 der Struktur

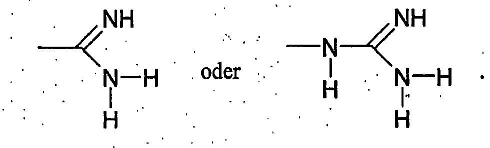

Hinsichtlich des Restes R2 sind generell sämtliche unter die Definition gemäß (ii) fallenden Reste möglich. Demgemäß sind als Arylgruppen beispielsweise die Phenylgruppe oder die Naphthylgruppe denkbar, wobei, mindestens eines der C-Atome dieser A-arylgruppe durch ein Heteroatom, ausgewählt aus der Gruppe bestehend aus S, N und O, ersetzt sein kann. Besonders bevorzugt ist als Rest R₂ ein Phenylrest. Als ein mindesten ein Heteroatom enthaltender Arylrest sind Thienyl und Pyridyl bevorzugt.

Demgemäß betrifft die vorliegende Erfindung eine Verbindung, wie oben beschrieben, die dadurch gekennzeichnet ist, dass R₂ ein mindestens einfach substituierter Phenylrest, Thienylrest oder Pyridylrest ist.

Gemäß einer besonders bevorzugten Ausführungsforin ist der R₂ ein mindestens einfach substituierter Phenylrest.

Der Arylrest, besonders bevorzugt der Phenylrest, R₂ weist mindestens einen wie oben definierten Substituenten R₄ auf, wobei eine der Strukturen 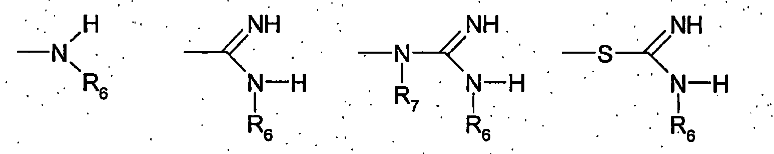 bevorzugt ist.

Besonders bevorzugt ist dabei ein Substituent R₄, der ausgewählt wird aus der Gruppe bestehend aus

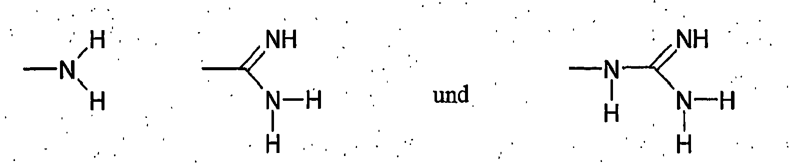

Ganz besonders bevorzugt als Rest R₄ ist ein Substituent der Struktur

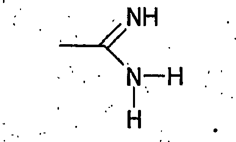

Der Substituent R₄ kann generell an sämtlichen Positionen des Arylrestes positioniert sein. Bezüglich des besonders bevorzugten Phenylrestes ist demgemäß die 2-, 3- oder 4-Stellung des Restes R₄ möglich, wobei besonders bevorzugt die 3-Stellung des Restes R₄ am Phenylrest ist.

Neben dem Rest R₄ kann der Arylrest weiter mindestens einen weiteren Substituenten aufweisen. Gemäß einer besonders bevorzugten Ausführungsform weist der Arylrest einen einzigen Substituenten auf.

Die Alkylengruppe, mit der der Rest R₂ an das alpha-C-Atom der mittelständigen Aminosäure gemäß (I) gebunden ist, weist im allgemeinen 0 bis 3 C-Atome auf. Bevorzugt weist diese Alkylengruppe 1, 2 oder 3 C-Atome, besonders bevorzugt 1 oder 2 C-Atome und ganz besonders bevorzugt 1 C-Atom auf.

Demgemäß beschreibt die vorliegende Erfindung ein Verbindung, wie oben beschrieben, die dadurch gekennzeichnet ist, dass sie die folgende Struktur aufweist:

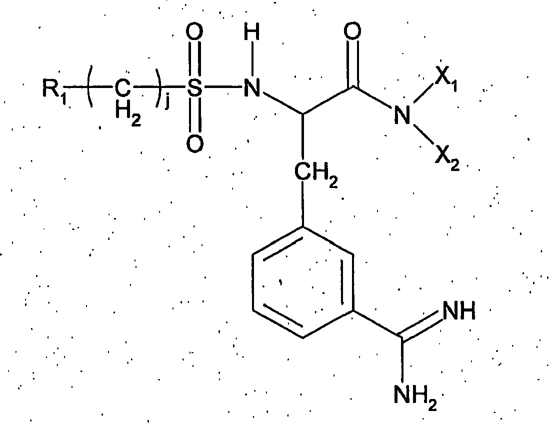

Die oben gezeigte Struktur kann am α-C-Atom des 3-Amidinophenylalanins generell sowohl in der der D- als auch in der L-Konfiguration vorliegen. Besonders bevorzugt ist im Rahmen der vorliegenden Erfindung die L-Koiülguration. Ganz allgemein können die erfinduhgsgemäßen Verbindungen gemäß (I) hinsichtlich der mittelständigen alpha-Aminosäure entweder in der L- oder in der D-Konfiguration oder als Racemat vorliegen.

Demgemäß betrifft die vorliegende Erfindung auch eine Verbindung, wie oben beschrieben, die dadurch gekennzeichnet ist, dass k = 1 und R₂ ein mit einer Amidinogruppe meta-substituierter Phenylrest ist, wobei das dadurch entstehende 3Amidinophenylalanin in der L-Konfiguration vorliegt.

Grundsätzlich können die Verbindungen gemäß (I) bei Vorliegen eines neben dem oben erwähnten asymmetrischen C-Atoms weiteren asymmetrischen C-Atoms oder eines anderen Asymmetriezentnuns sowohl in der L- als auch in der D-Konfiguration oder S- oder R-Konfiguration vorliegen. Ebenso kann die Verbindung gemäß (I) als Racemat vorliegen. Weiterhin sind Mischungen aus L- und D-Konfiguration oder S- und R-Konfiguration möglich, bei denen der Anteil der Moleküle mit D-Konfiguration oder der Anteil der Moleküle mit L-Konfiguration beziehungsweise der Anteil der Molekül mit S-Konfiguration oder der Anteil der Moleküle mit R-Konfiguration überwiegt.

Als Rest R₁ kann generell jeder der gemäß obiger Definition gemäß (i) beschriebene Rest R₁ vorliegen.

Ist der Rest R₁ beispielsweise mit mindestens einem Halogen substituiert, so sind hier-bei Fluor, Chlor und/oder Brom bevorzugt. ,

Ist der Rest R₁ beispielsweise mit mindestens einer veresterten Carboxylgruppe substituiert, so sind Methylester und/oder Ethylester bevorzugt

Ist der Rest R₁ beispielsweise mit mindestens einer Aminogruppe substituiert, so kann diese Aminogruppe acyliert sein, wobei insbesondere die Acetylgruppe bevorzugt ist.

Beispielsweise besonders bevorzugt sind mono-, bi- oder tricyclische Arylreste und Heteroarylreste, bei denen gegebenenfalls mindestens eine Doppelbindung hydriert ist und/oder die mindestens ein Heteroatom, ausgewählt aus O, S und N aufweisen, wobei ein Heteroarylrest R₁ auch zwei oder mehr gleiche, oder verschiedene Heteroatome enthaltern kann. Beispiele für bevorzugte Arylreste sind etwa Phenyl, Naphthyl, Anthracyl oder Phenanthryl. Diese Arylreste können gegebenenfalls in reduzierter oder/und oxidierter Form vorliegen. Hinsichtlich des Naphthylrestes ist beispielsweise ein 1,2-Dihydronaphthyl, ein 1,4,-Dihydronaphthyl oder auch ein 1,2,3,4-Tetrahydronaphthylrest möglich. In oxidierter Form kann der Naphthylrest beispielsweise als 1,4-Naphthochinoylrest vorlieben. Der Anthrachinoylrest kann in oxidierter . Form beispielsweise als 1,4- oder 9,10-Anthrachinoylrest oder 1,4- oder 9,10-Anthrahydrochinoylrest vorliegen, der Phenanthrylrest beispielsweise als Phenanthrenchinoylrest. Beispiele für Heteroarylreste sind etwa Pyrrolyl, Furanyl, Thiophenyl, Pyridyl, Pyrimidyl, Pyrazyl, Triazyl, Imidazolyl, Thiazolyl, Oxazolyl, Indolyl, Purinyl, Pyronyl, Pyridonyl, Quinolyl, Isochinolyl. Ebenso sind auch Reste R₁ wie Indenyl oder Tetrahydroindenyl umfasst.

Weiter kann der Rest R₁ geeignet substituiert sein, wobei als Substituenten beispielsweise lineare, verzweigte oder cyclische Alkylreste mit 1 bis 10 C-Atomen bevorzugt sind. Besonders bevorzugt sind lineare oder verzweigte Alkyreste mit 1, 2, 3, oder 4 C-Atomen. Beispielsweise besonders bevorzugt sind als Substituenten Isopropyl und tert-Butyl. Ebenso sind cyclische Alkylreste als Substituenten bevorzugt, wobei cyclische Alkylreste mit 5, 6 oder 7 C-Atomen und insbesondere 6 C-Atomen besonders bevorzugt sind. Ebenfalls zu nennen sind Aryl- oder Heteroarylgruppen als Substituenten, wobei die Heteroarylgruppen ein Heteroatom, ausgewählt aus N, S und O, enthalten und zwei oder mehr gleiche oder verschiedene Heteroatome enthalten können. Die Substituenten des Restes R₁ können wiederum selbst geeignet substituiert sein.

Sowohl Heteroaryl- und Aryl- als auch Alkylreste können hierbei durch ein Schwefelbrückenatom oder ein Sauerstoffbrückenatom oder über eine Alkylenkette mit 1-3 C-Atomen an den Rest R₁ gebunden sein. Demgemäß kann der Rest R1 beispielsweise mit einer Alkyloxy-, Alkylthio-, Aryloxy-, Arylthio, Heteroaryloxy- oder Heteroarylthiogruppe substituiert sein.

Beispiele für substituierte Reste R₁ sind etwa

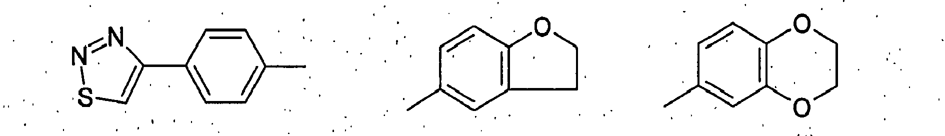

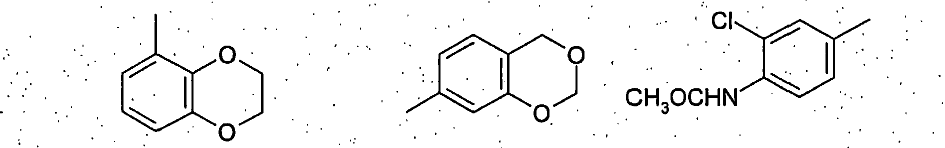

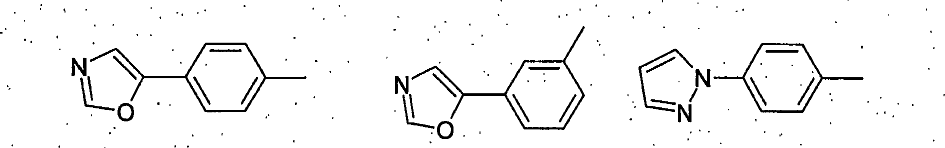

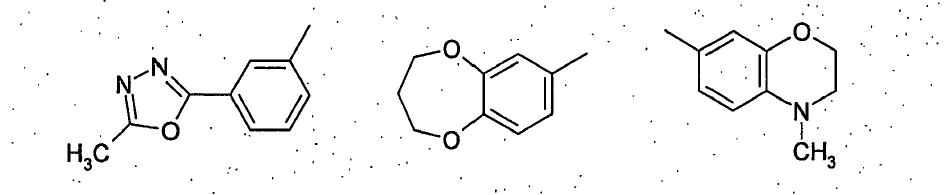

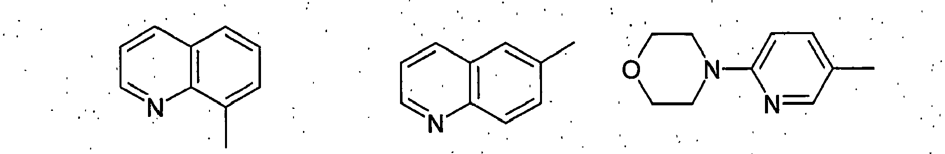

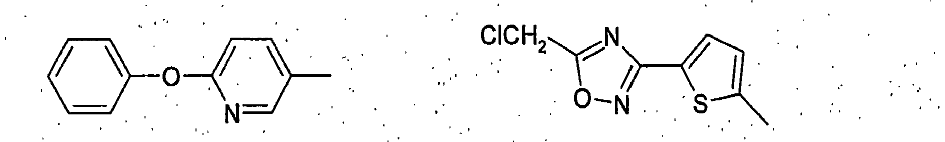

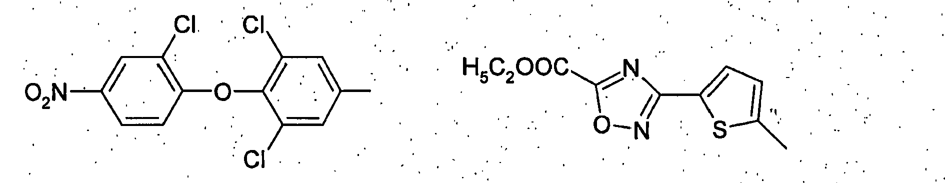

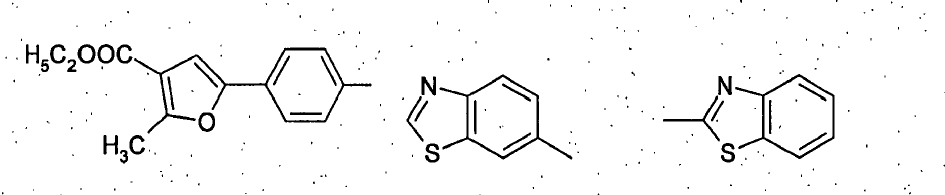

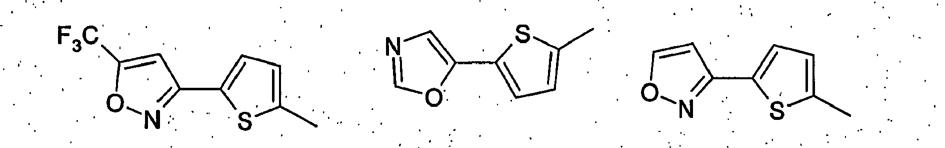

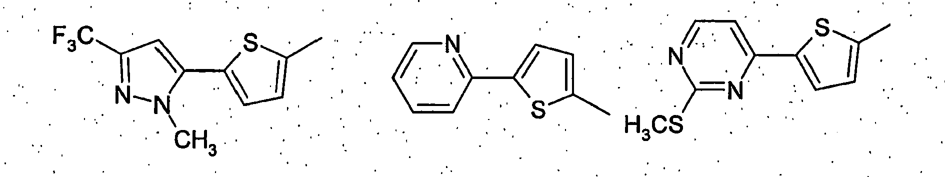

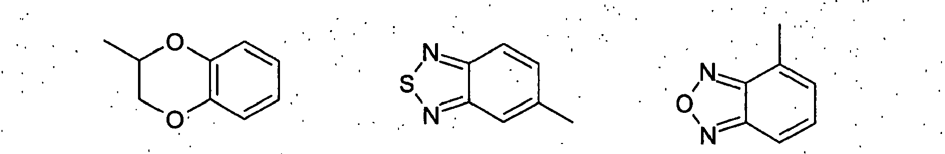

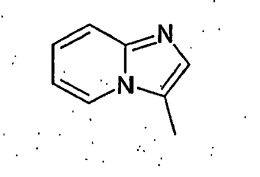

Ein bevorzugt vorliegender Arylrest kann grundsätzlich auch mehr als einen Substituenten tragen. Besonders bevorzugt sind Ausführungsformen, bei denen der Arylrest keinen, einen, zwei oder drei Substituenten aufweist. Weist der Arylrest beispielsweise drei. Substituenten auf, so sind unter anderem Alkylrest mit 1, 2 oder C-Atomen bevorzugt. Besonders bevorzugt sind Alkylreste mit 2 oder 3 C-Atomen, insbesondere bevorzugt Alkylreste mit 3 C-Atomen, und insbesondere besonders bevorzugt Isopropyl. Insbesondere bevorzugt ist hierbei beispielsweise der 2,4,6-Triisopropylphenylrest.

Weist der Arylrest beispielsweise einen Substituenten auf, so ist unter anderem der tert-Butylrest bevorzugt.

In einer weiteren geeigneten Ausführungsform ist der Rest R₁ ein Arylrest, vorzugsweise ein Phenylrest, der mit einem weiteren Arylrest oder Heteroarylrest über ein Sauerstoffbrückenatom oder über eine Alkylenkette mit 1-3 C-Atomen wiederum mit einem Aryl-, Heteroaryl- oder Alkylrest substitutiert ist. Dabei kann der Aryl- oder Heteroarlyrest, beispielsweise ein Pyridin, unsubstituiert oder ebenfalls, an geeigneter Position, in ortho, meta oder para-Positiön, substituiert sein, beispielsweise mit mindestens einer Alkylgruppe, wie zum Beispiel einer Methylgruppe und/oder mit mindestens einem Halogenatom, vorzugsweise mit einem Chloratom oder zwei Chloratomen oder einem der zwei Fluoratomen und/oder mit mindestens einer Trihalogenmethylgruppe, vorzugsweise mit einer oder zwei einer Trifluormethylgruppe(n) und/oder mit mit mindestens einer Alkoxygruppe, vorzugsweise' einer Methoxygruppe.

Beispiele derartiger Reste R₁ sind etwa 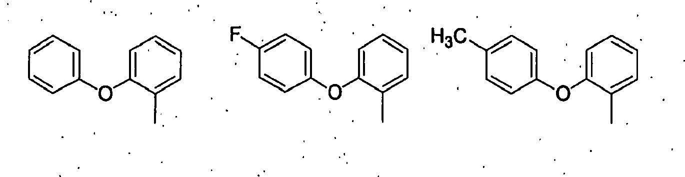 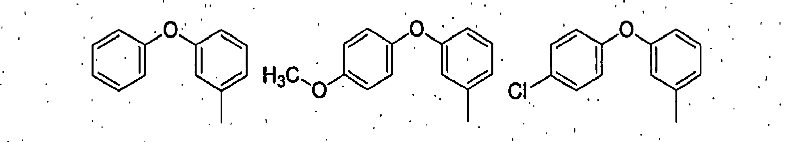 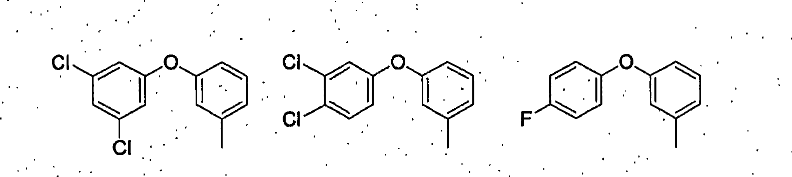 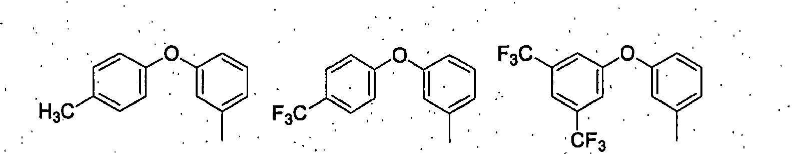 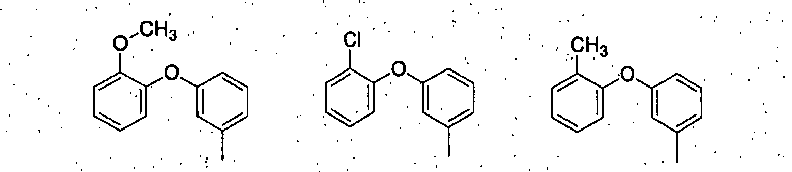 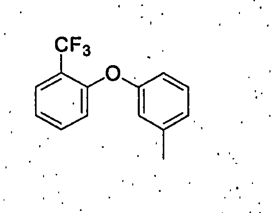 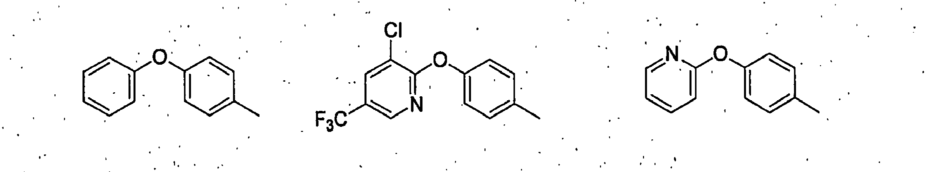 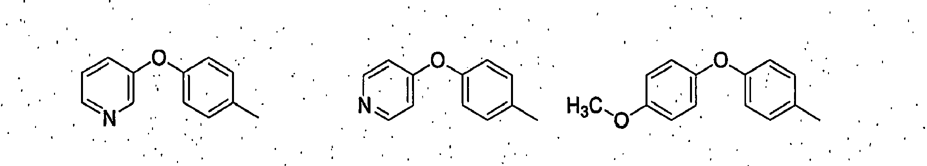 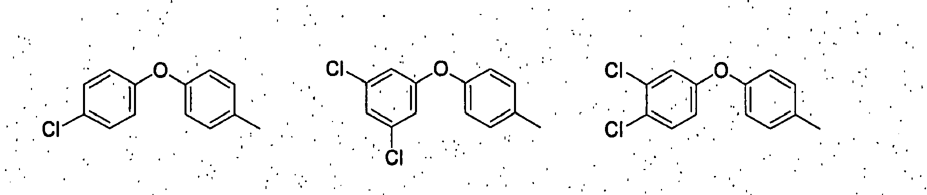 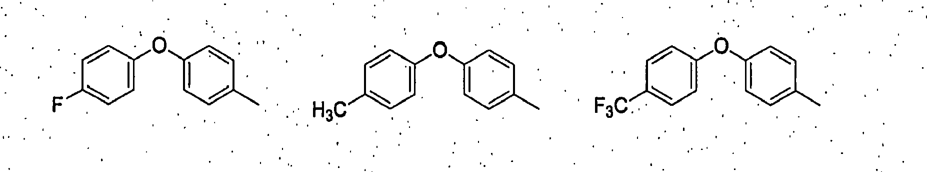 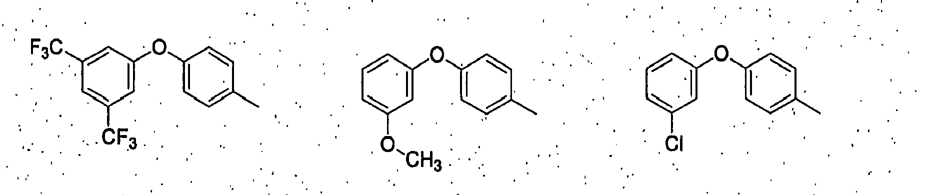 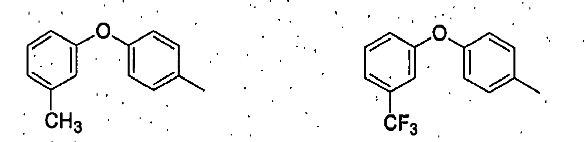 wobei der die beiden Aryle verbindende Sauerstoff durch eine Alkylenkette mit 1-3 C-Atomen ersetzt sein kann.

Gemäß einer besonders bevorzugten Ausführungsform wird der Rest R₁ ausgewählt aus tert-Butylphenyl, Cyclohexylphenyl, 5,6,7,8-Tetrahydronaphthyl, Naphthyl, Anthracyl, Anthrachinoyl und Anthrahydrochinoyl, Pyridyloxyphenyl, Phenyloxypyridyl, Pyridylalkylphenyl mit einem C₁-C₃-Alkyl.

Die Alkylengruppe, die den Rest R₁ und die Sulfonylgruppe verknüpft, weist im Allgemeinen kein, ein oder zwei C-Atome auf. Bevorzugt weist sie kein oder ein C-Atom auf, wobei sie insbesondere bevorzugt kein C-Atom aufweist und damit nicht vorhanden ist.

Eine alternative Ausführungsform der Erfindung sind Verbindungen der Formel I" 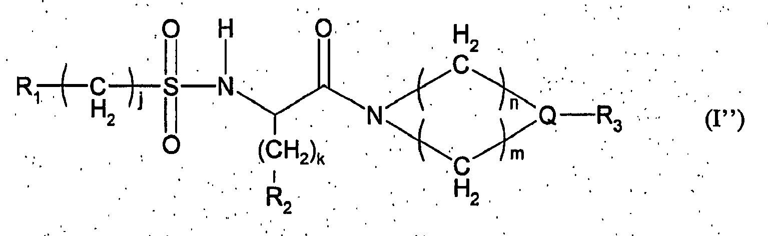 wobei R₁, R₂, Q, j und k definiert sind wie oben beschrieben, m = n = 2 ist und R₃ ein Aryl- oder Heteroarylrest ist, und wobei der Arylrest vorzugsweise ein Benzyl- oder ein Phenoxy-Rest ist und der Heteroarylrest vorzugsweise ausgewählt ist aus einem Pyridinylmethylen-, Pyridinyloxo-, Pyrimidinyloxo-, Pyrazinyloxo-, Pyridinylthiorest und wobei der Aryl- oder Heteroarylrest unsubstituiert oder substituiert ist mit mindestens einem Halogen, vorzugsweise Fluor oder Chlor, mindestens einem Alkoxyrest vorzugsweise Methoxyrest und/oder mindestens einem Trifluormethylrest.

In einer weiteren bevorzugten Ausführungsform ist der Rest R₃ ein Guanidinooxyalkylrest.

Beispiele derartiger Verbindungen sind Verbindungen in denen der Baustein 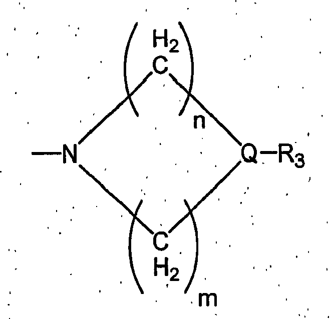 in Formel I" ersetzt ist durch, 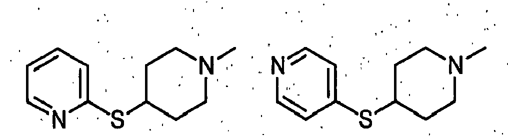 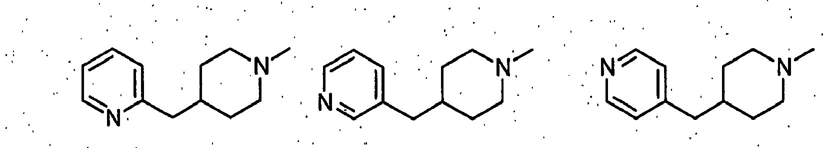 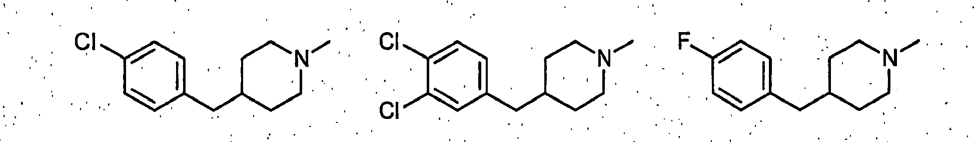 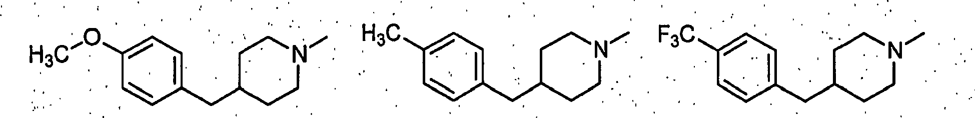 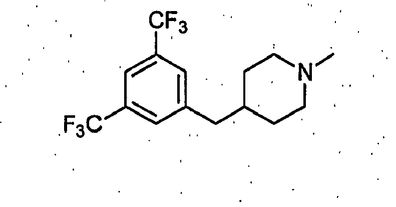 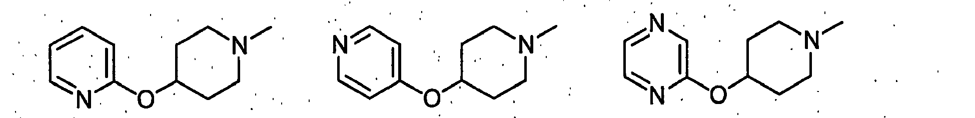 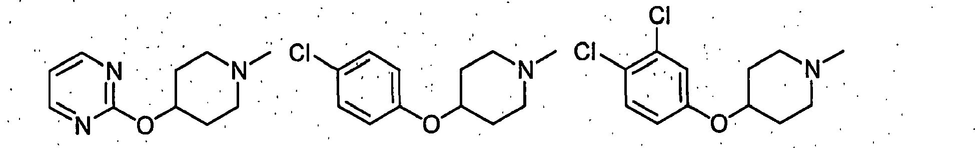 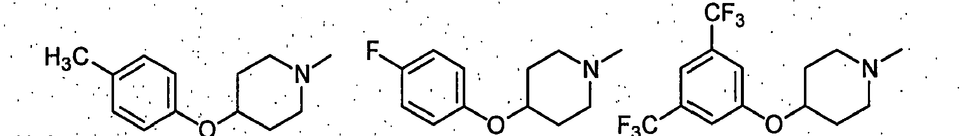 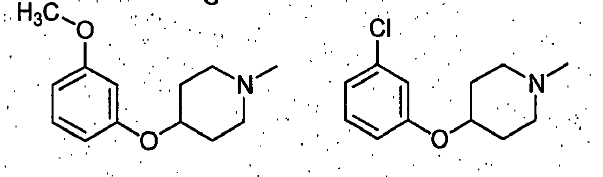 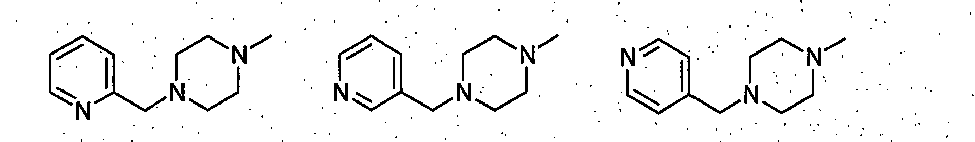 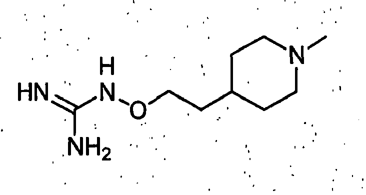 welche kommerziell erhältlich sind von Array Bipharma, Bolder Colorado, U.S.A..

Ebenso umfasst sind Verbindungen, in denen zwischen der Sulfonylgruppe und der 3-Amidinophenylalaningruppe der Formel I eine Aminosäure, vorzugsweise Glycin, eingebaut ist.

Die oben beschriebenen Verbindungen können generell gemäß,sämtlicher geeigneter Verfahren hergestellt werden. Bevorzugt werden die erfindungsgemäßen Verbindungen durch Verfahren hergestellt, bei denen in einem ersten Schritt ein Sulfonsäurechlorid mit einer Aminosäure oder einem Aminosäurederivat umgesetzt wird

Demgemäß betrifft die vorliegende Erfindung ein Verfahren zur Herstellung einer wie oben beschriebenen Verbindung, das den folgenden Schritt (S1) umfasst:
(S1) Umsetzung einer Verbindung der allgemeinen Struktur (E1') 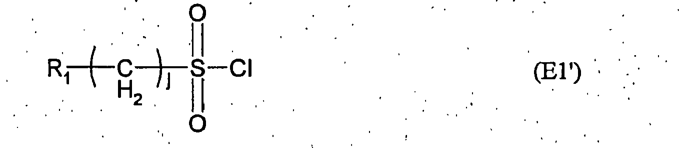 mit einer Verbindung der allgemeinen Struktur (E1") 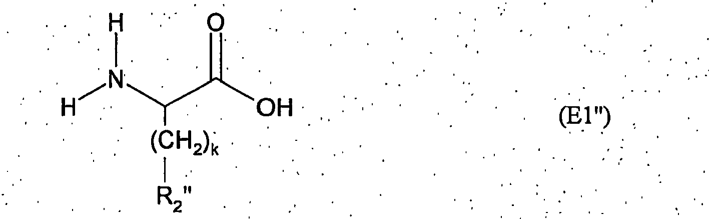 unter Erhalt einer Verbindung der allgemeinen Struktur (ZP1) 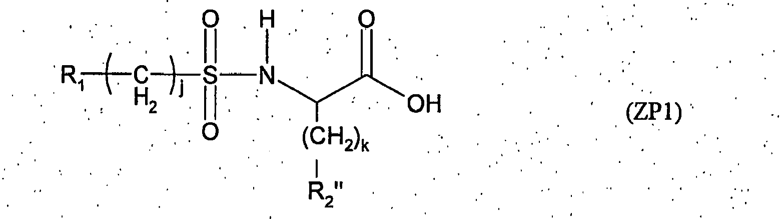 wobei R₂" der Arylrest R₂, substituiert entweder mit R₄ oder mit R₄, geschützt mit einer geeigneten Schutzgruppe; oder mit einem Substituenten, der eine Vorstufe zu R₄ darstellt.

Ist der Arylrest R₂ beispielsweise bevorzugt mit einer Amidinogruppe R₄ substituiert, so ist der Substituent, der eine Vorstufe zur Amidinogruppe darstellt, beispielsweise eine Cyanogruppe, die in einer, bevorzugt zwei oder mehr geeigneten Verfahrensstufen durch Umsetzung mit Hydroxylamin und anschließender Hydrierung zu einer Amidinogruppe umgewandelt werden kann. Diese Amidinogruppe kann im Folgenden wiederum mit einer geeigneten Schutzgruppe geschützt werden, die in einem geeigneten Verfahrensschritt wieder entfernt werden kann.

Werden erfindungsgemäße Verbindungen der allgemeinen Struktur (I") hergestellt, bei denen die Gruppe Q gleich Stickstoff ist und die Alkylengruppe A₁ nicht vorhanden ist, so sind Herstellungsverfahren bevorzugt, bei denen, ausgehend von einer Verbindung der Struktur (ZP1), diese zunächst in einem Schritt (S2') mit einer cyclischen Verbindung der allgemeinen Struktur (E2') 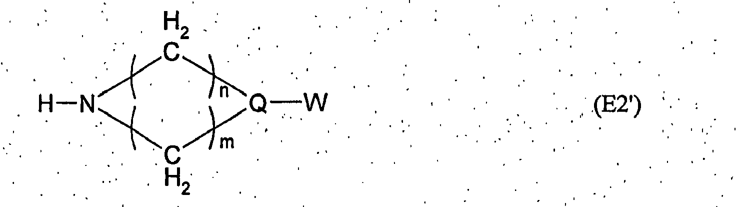 umgesetzt, wobei W eine geeignete Schutzgruppe ist. Dabei wird eine Verbindung der allgemeinen Struktur (ZP2') 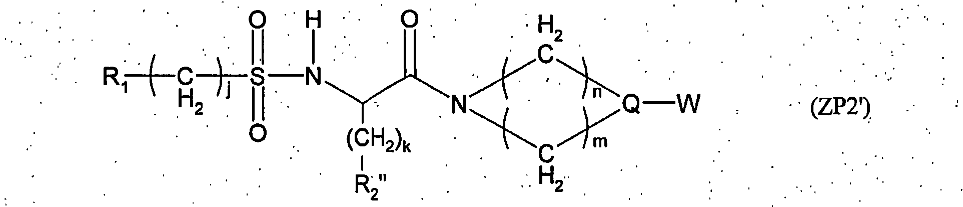 erhalten, aus der in einem nachfolgenden Schritt (S3') bevorzugt die Schutzgruppe W abgespalten wird. In einem nächsten Schritt (S4') wird die gemäß (S3') erhaltene Verbindung mit einer Verbindung der allgemeinen Struktur (E2"). 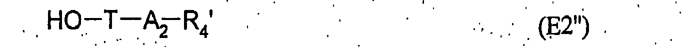 umgesetzt, wobei eine Verbindung der allgemeinen Struktur (P1) 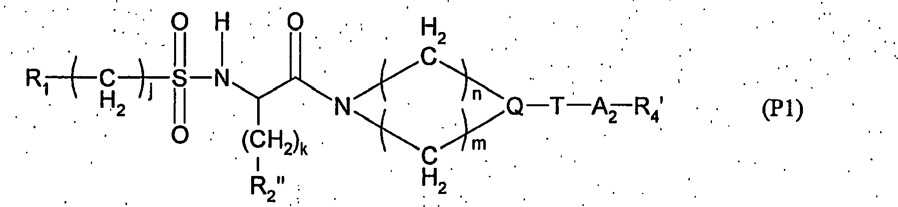 erhalten wird, wobei R₄' entweder R₄ oder R₄, geschützt mit einer geeigneten Schutzgruppe, oder eine Vorstufe zu R₄ darstellt. Ist R₄' ein mit einer Schutzgruppe geschützter Rest R₄, so wird bevorzugt nach Schritt (S4') die Schutzgruppe entfernt.

Erfindungsgemäß kann dann als Rest R₄ eine Aminogruppe vorliegen. Aus dieser kann in einem nächsten Schritt gemäß einem dem Fachmann bekannten Verfahren, beispielsweise durch Umsetzung mit Pyrazolcarboxamidin, eine Guanidinogruppe aufgebaut werden.

Ein ebenfalls bevorzugtes Verfahren zur Herstellung von Verbindung gemäß (I"), bei denen die Gruppe Q gleich Stickstoff ist und die Alkylengruppe A₁ nicht vorhanden ist, umfasst einen Schritt (S2'), bei dem eine Verbindung der allgemeinen Struktur (E2"') 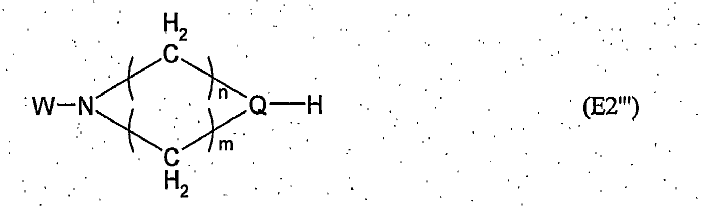 wobei W eine geeignete Schutzgruppe ist, mit einer Verbindung der allgemeinen Struktur (E2") 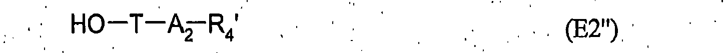 umgesetzt wird, wobei eine Verbindung der allgemeinen Struktur (ZP2") 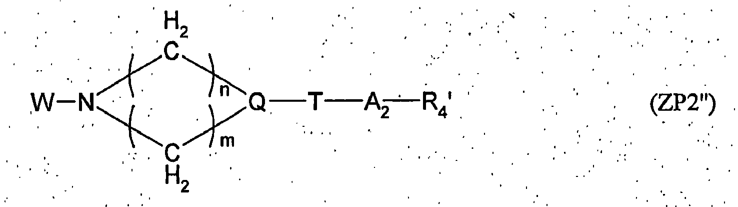 erhalten wird, bei der R₄' entweder R₄ oder R₄, geschützt mit einer geeigneten Schutzgruppe, oder eine Vorstufe zu R₄ darstellt. In diesem Verfahren wird also zuerst der C-terminale Rest der mittelständigen Aminosäure gemäß (I) aufgebaut. In einem nächsten Schritt (S3') wird bevorzugt die Schutzgruppe W abgespaltet, und in einem nächsten Schritt (S4') die gemäß (S3') erhaltene Verbindung mit einer Verbindung der allgemeinen Struktur (ZP1) unter Erhalt einer Verbindung der allgemeinen Struktur (P1) umgesetzt. Ist hierbei der Rest R₄ eine Aminogruppe, so kann auch hier, wie oben beschrieben, daraus eine Guanidinogruppe aufgebaut werden.

Werden erfindungsgemäße Verbindungen der allgemeinen Struktur (I") hergestellt, bei denen die Gruppe Q gleich CH ist, so sind Herstellungsverfahren bevorzugt, bei denen ausgehend von Verbindungen gemäß (ZP1) diese in einem Schritt (S2") mit einer Verbindung der allgemeinen Struktur (E3) 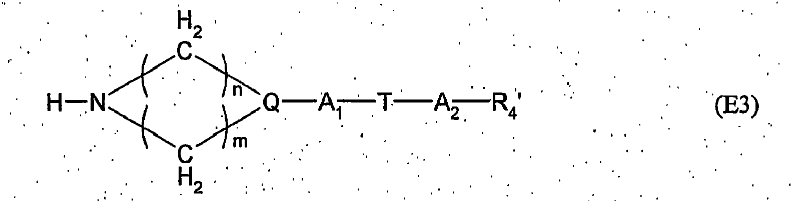 umgesetzt werden, wobei R₄' entweder R₄ oder R_{4,} geschützt mit einer geeigneten Schutzgruppe, oder eine Vorstufe zu R₄ darstellt. Durch diese bevorzugte Umsetzung wird eine Verbindung der allgemeinen Struktur (P2) 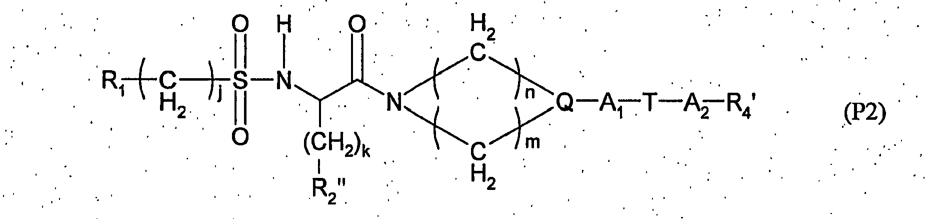 erhalten. Ist R₄' ein mit einer Schutzgruppe geschützter Rest R₄, so wird bevorzugt nach Schritt (S2") die Schutzgruppe entfernt. Erfindungsgemäß kann dann als Rest R₄ eine Aminogruppe vorliegen. Aus dieser kann in einem nächsten Schritt gemäß einem dem Fachmann bekannten Verfahren, beispielsweise durch Umsetzung mit Pyrazolcarboxamidin, eine Guanidinogruppe aufgebaut werden.

Werden erfindungsgemäße Verbindungen der allgemeinen Struktur (I") hergestellt, bei denen T gleich -(C=O)-NH-, ist, Q gleich CH ist und A₁ nicht vorhanden ist, so sind Herstellungsverfahren bevorzugt, bei denen in einem Schritt (S2"') zunächst Verbindungen der allgemeinen Struktur (E3') 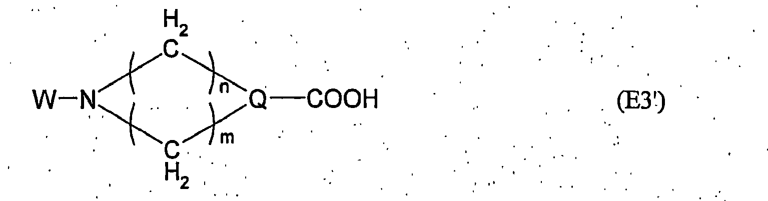 mit einer Verbindung der allgemeinen Struktur (E3") 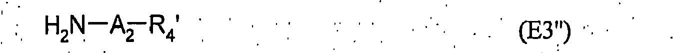 umgesetzt werden, wobei eine Verbindung der allgemeinen Struktur (ZP3) 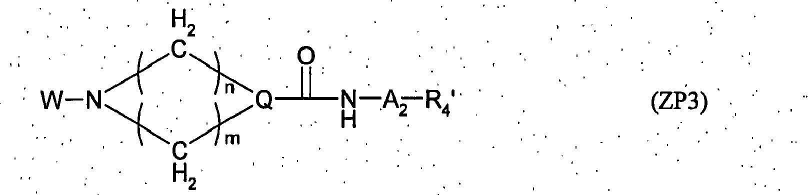 erhalten wird. Der Rest R₄' hat die bei den oben beschriebenen weiteren Herstellverfahren beschriebene Bedeutung. W ist auch hier eine geeignete Schutzgruppe. In einem nächsten Schritt wird die Schutzgruppe W gemäß einem geeigneten Verfahren bevorzugt in einem Schritt (S3"') entfernt. Bevorzugt schließt sich daran ein Schritt (S4"') an, bei dem die gemäß (S3"') erhaltenene Verbindung mit einer Verbindung gemäß (ZP1) umgesetzt wird, wobei eine Verbindung der allgemeinen Struktur (P3) 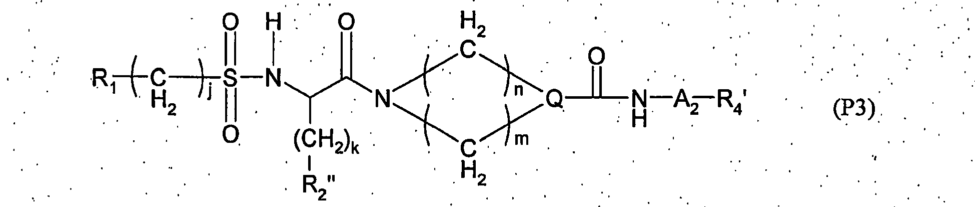 erhalten wird. Ist R₄' ein mit einer Schutzgruppe geschützter Rest R₄, so wird bevorzugt nach Schritt (S2"') die Schutzgruppe entfernt. Erfindungsgemäß kann dann als Rest R₄ eine Aminogruppe vorliegen. Aus dieser kann in einem nächsten Schritt gemäß einem dem Fachmann bekannten Verfahren, beispielsweise durch Umsetzung mit Pyrazolcarboxamidin, eine Guanidinogruppe aufgebaut werden

Gemäß einer bevorzugten Ausführungsform dieser zuletzt beschriebenen Verfahrensva- .. riante kann als Verbindung der allgemeinen Struktur (E3") beispielsweise eine Verbindung eingesetzt werden, die als Rest R₄' eine Vorstufe zu einem erfindungsgemäßen Rest R₄ aufweist, wobei diese Vorstufe eine -CN-Gruppe ist. In einem oder mehreren geeigneten Schritten wird diese -CN-Gruppe weiter bevorzugt gemäß einem dem Fachmann bekannten Verfahren, beispielsweise durch Umsetzung mit einem Hydroxylamin, anschließender Umsetzung mit Essigsäureanhydrid und nachfolgender Hydrierung, zu einer Amidinogruppe aufgebaut. Gemäß einer anderen Ausführungsform des erfindungsgemäßen Verfahrens kann das Hydroxyamidino-Derivat direkt durch Hydrierung in ein Amidin-Derivat überführt werden;, wobei die Amidinogruppe gegebenenfalls nochmals intermediär mit einer geeigneten Schutzgruppe geschützt wird. Ganz besonders bevorzugt erfolgt die Umsetzung mit Hydroxylamin nach dem Schritt (S2"') und vor dem Schritt (S3"'). Weiter besonders bevorzugt erfolgt die Umsetzung mit Essigsäureanhydrid ebenfalls vor dem Schritt (S3"'). Die Hydrierung zur Amidinogruppe erfolgt bevorzugt nach dem Schritt (S4"').

Ganz besonders bevorzugt erfolgt die Endreinigung der gemäß derart hergestellten Verbindungen über präparative, reversed-phase HPLC oder durch Kristallisation aus einem geeigneten Lösungsmittel bzw. Lösungsmittelgemischs oder durch Gegenstromverteilung.

Neben den wie oben beschriebenen Verbindungen an sich und Verfahren zu deren Herstellung betrifft die vorliegende Erfindung auch ein Arzneimittel, das eine der oben angegebenen Verbindungen enthält.

Demgemäß betrifft die vorliegende Erfindung auch ein Arzneimittel, enthaltend mindestens eine Verbindung gemäß (I) oder ein Salz dieser Verbindung sowie gegebenenfalls pharmazeutisch geeignete Hilfs- und/oder Zusatzstoffe.

Ebenso beschreibt die vorliegende Erfindung eine Verbindung gemäß (I) zur Verwendung als Arzneimittel, wobei das Arzneimittel gegebenenfalls zusätzlich pharmazeutisch geeignete Hilfs- und/oder Zusatzstoffe enthält.

Geeignete Hilfs- und/oder Zusatzstoffe, die beispielsweise der Stabilisierung und/oder Konservierung des Arzneimittels dienen, sind beispielsweise in H.' Sucker et al., Pharmazeutische Technologie, 2. Auflage, Georg Thieme Verlag, Stuttgart (1991) offenbart, wobei die diesbezügliche Offenbarung durch Bezugnahme in den Kontext der vorliegenden Erfindung aufgenommen wird. Zu den pharmazeutisch geeigneten Hilfs- und/oder Zusatzstoffen zählen beispielsweise physiologische Kochsalzlösungen, Ringer-Dextrose, Ringer-Laktat, entmineralisiertes Wasser, Stabilisatoren, Antioxidantien, Komplexbildner, antimikrobielle Verbindungen, Proteinaseinhibitoren und/oder inerte Gase.

Generell können die erfindungsgemäßen Verbindungen gemäß (I) in jedweder Form als Arzneimittel eingesetzt werden. In möglichen erfindungsgemäßen Ausführungsform wird das Arzneimittel beispielsweise in Form einer Tablette, eines Dragées, einer Kapsel, eines Pellets, Suppositoriums, einer Lösung, insbesondere einer Injektions- oder ' Infusionslösung, von Augen-, Nasen und Ohrentropfen, eines Safts, .einer Kapsel, einer Emulsion oder Suspension, eines Globuli, Styli, Aerosols, Puders, einer Paste, Creme oder Salbe eingesetzt.

Daher betrifft die vorliegende Erfindung auch ein Arzneimittel, wie oben beschrieben, das dadurch gekennzeichnet ist, dass es in Form einer Tablette, eines Dragées, einer Kapsel, eines Pellets, eines Suppositoriums, einer Lösung, insbesondere einer Injektions- oder Infusionslösung, von Augen-, Nasen- oder Ohrentropfen, eines Saftes, einer Emulsion oder Suspension, eines Globuli, eines Styli, eines Aerosols, eines Puders, einer Paste, einer Creme oder einer Salbe eingesetzt wird.

Ebenso beschreibt die vorliegende Erfindung daher auch eine Verbindung gemäß (I) zur Verwendung als Arzneimittel, wobei das Arzneimittel in Form einer Tablette, eines Drageés, einer Kapsel, eines Pellets, eines Suppositoriums, einer Lösung, insbesondere, einer Injektion- oder Infusionslösung, von Augen-, Nasen- oder Ohrentropfen, eines Saftes, einer Emulsion oder Suspension, eines Globuli, eines Styli, eines Aerosols, eines Puders, einer Paste, einer Creme oder einer Salbe eingesetzt wird.

Gemäß einer besonders bevorzugten Ausführungsform der vorliegenden Erfindung werden die oben genannten Verbindungen gemäß (I) und/oder deren Salze oder die oben genannten Arzneimittel, enthaltend diese Verbindungen gemäß (I) und/oder deren Salze und gegebenenfalls mindestens einen pharmazeutisch geeigneten Hilfs- und/oder Zusatzstoff, eingesetzt, um einen Tumor zu diagnostizieren und/oder zu therapieren. Ebenso ist auch zusätzlich oder alternativ die Prophylaxe eines Tumors möglich, wobei die Verbindungen besonders zur Verhinderung und/oder Reduktion der Metastasierung von Tumoren verwendet werden können.

Eine erfindungsgemäße Verbindung gemäß (I) oder deren Salz oder ein wie oben beschriebenes Arzneimittel kann ganz allgemein weiterhin beispielsweise in parenteraler Anwendungsform, insbesondere in intraartieller, intravenöser, intramuskulärer oder subkutaner Form, in enteraler Anwendungsform, insbesondere zur oralen oder rektalen Anwendung, oder in topischer Anwendungsform, insbesondere als Dermatikum, eingesetzt werden. Bevorzugt sind intravenöse oder subkutane Anwendungen.

Insbesondere sind diese Anwendungsformen zur Diagnose und/oder Therapie und/oder Prophylaxe eines Tumors geeignet.

Daher betrifft die vorliegende Erfindung auch die Verwendung einer Verbindung gemäß (I) oder eines Salzes dieser Verbindung oder eines wie oben beschriebenen Arzneimittels zur Diagnose, Therapie oder Prophylaxe eines Tumors und zur Verhinderung und/oder Reduktion der Metastasierung eines Tumors, insbesondere in oraler, subkutaner, intravenöser oder transdermaler Form.

Ebenso beschreibt die vorliegende Erfindung die Verwendung einer Verbindung gemäß (I) oder eines Salzes dieser Verbindung zur Herstellung eines Arzneimittels zur Diagnose, Therapie oder Prophylaxe eines Tumors und zur Verhinderung und/oder Reduktion der Metastasierung eines Tumors.

Weiter beschreibt die vorliegende Erfindung diese Verwendung zur Herstellung eines oral, subkutan, intravenös oder transdermal anzuwendenden Arzneimittels.

Gemäß einer insbesondere bevorzugten Ausführungsform der vorliegenden Erfindung wird eine erfindungsgemäße Verbindung gemäß (I) zur Reduzierung von Tumormetastasen eingesetzt.

Demgemäß betrifft die vorliegende Erfindung die oben beschriebene Verwendung der Verbindung gemäß (I) oder eines Salzes dieser Verbindung oder eines wie oben beschriebenen Arzneimittels, wobei die Bildung von Tumormetastasen reduziert wird.

Ebenso beschreibt die vorliegende Erfindung ein wie oben beschriebenes Verfahren zur Herstellung eines Arzneimittels zur Reduzierung der Bildung von Tumormetastasen.

Ebenso betrifft die vorliegende Erfindung daher insbesondere die Verwendung einer Verbindung gemäß (I) oder eines Salzes dieser Verbindung oder eines Arzneimittels, enthaltend die Verbindung gemäß (I) oder ein Salz dieser Verbindung, zur Hemmung der Matriptase.

Insbesondere betrifft die vorliegende Erfindung auch die Verwendung einer Verbindung gemäß (I) oder eines Salzes dieser Verbindung oder eines Arzneimittels, enthaltend die Verbindung gemäß (I) oder ein Salz dieser Verbindung, zur Hemmung der Matriptase, wobei die Matriptase MT-SP1 ist.

Demgemäß beschreibt die vorliegende Erfindung auch ein Verfahren zur Herstellung eines Arzneimittels, enthaltend eine Verbindung gemäß (I) oder ein Salzes dieser Verbindung, zur Hemmung der Matriptase.

Ebenso beschreibt die vorliegende Erfindung auch ein Verfahren zur Herstellung eines Arzneimittels, enthaltend eine Verbindung gemäß (I) oder ein Salzes dieser Verbindung zur Hemmung der Matriptase, wobei die Matriptase MT-SP1 ist.

Die oben beschriebenen Verbindungen können auch in Form von Prodrugs, z.B. durch Modifizierung der Amidinogruppe mit einer Hydroxyl- oder einer C₁-C₆-Alkyloxycarbonylgruppe, vorliegen, die erst nach Aufnahme in den Organismus spontan und/oder durch ein oder mehrere körpereigene Enzyme in die inhibitorisch wirksame Spezies umgewandelt werden, wodurch die Bioverfügbarkeit und die pharmakokinetischen Eigenschaften der Verbindungen verbessert werden können.

In den folgenden Beispielen und Figuren wird die Erfindung näher erläutert.

### Beschreibung der Figuren

Figur 1 zeigt die Hemmung des invasiven Wachstums durch die Matriptase Hemmstoffe 37 und 54 aus Beispiel 9.
Figur 2 zeigt die Hemmung des proHGF induzierten "scatterings" von PC-3 Zellen durch die Matriptase Inhibitoren 37 und 54 aus Beispiel 10.

### Beispiele

### Methoden

**Analytische HPLC:** Shimadzu LC-10A System, Säule: Phenomenex Luna C₁₈, 5 µm, 100 Å(250 x 4,6 mm) Lösungsmittel A: 0,1 % TFA in Wasser, B: 0,1 % TFA in ACN, Gradient: 10 % B bis 70 % B in 60 min, 1 ml/min Fluss, Detektion bei 220 oder 215 nm.

**Präparative HPLC:** Shimadzu LC-8A System, Säule: Phenomenex Luna C₁₈, 5 µm, 100 Å (250 x 30 mm) Lösungsmittel A: 0,1 % TFA in Wasser, B: 0,1 % TFA in ACN, Gradient: 10 % B bis 55 % B in 120 min, 10 ml/min Fluss, Detektion bei 220 nm.

**Massenspektroskopie:** Die Massenspektren wurden auf einem Kompact Probe der Firma Kratos (Manchester, England) mit einem Flugzeitmessungsdetektor und α-Cyano-Hydroxyzimtsäure als Matrix, bzw. auf einem ESI-MS LCQ der Firma Finnigan (Bremen, Deutschland), gemessen.

### Verwendete Abkürzungen

- Ac: Acetyl
- AcOH: Essigsäure
- ACN: Acetonitril
- β-Ala: β-Alanin
- Boc: tert.-Butyloxycarbonyl
- CKIBE: Chlorkohlensäureisobutylester
- DAE: 1,2-Diaminoethan
- DCM: Dichlormethan
- DIEA: Diisopropylethylamin
- DMF: N,N-Dimethylformamid
- iNip: Isonipecotinsäure
- Ki: Hemmkonstante
- NMM: N-Methylmorpholin
- Phe(3-AcOxam): 3-(Acetyloxamidino)Phenylalanin
- Phe(3-Am): 3-Amidinophenylalanin
- Phe(3-CN): 3-(Cyano)Phenylalanin
- Phe(3-Oxam): 3-(Oxamidino)Phenylalanin
- PyBop: Benzotriazol-1-yl-N-oxytris(pyrrolidino)phosphoniumhexafluorophosphat
- Pzd: Piperazid
- RT: Raumtemperatur
- TFA: Trifluoressigsäure
- THF: Tetrahydrofuran
- Tips: 2,4,6-(Triisopropyl)Phenylsulfonyl
- Z: Benzyloxycarbonyl

### Beispiel 1: Synthese von Anthracensulfonyl-Phe(3-Am)-Pzd-β-Ala x 2 TFA (Verbindung 10 aus Tabelle 1)

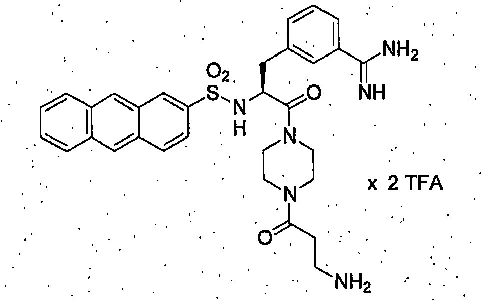

### 1a) Boc-Pzd-βAla-Z

2 g (8,96 mmol) Z-βAla-OH wurden in 20 ml THF gelöst und bei -15 °C mit 0,99 ml (8,96 mmol) NMM und 1,17 ml (8,96 mmol) CKIBE versetzt. Der Ansatz wurde 10 min bei -15 °C gerührt, dann wurden 1,67 g (8,96 mmol) Boc-Piperazid (Fluka) und zusätzlich 400 µl (3,6 mmol) NMM zugegeben. Der Ansatz wurde eine weitere Stunde bei -15 °C und weiter über Nacht bei Raumtemperatur gerührt. Anschließend wurde das Lösungsmittel im Vakuum entfernt, der Rückstand in Essigester aufgenommen, jeweils 3 x mit 5 % KHSO₄, gesättigter NaHCO₃-Lösung und NaCl-gesättigtem Wasser gewaschen und anschließend über Na₂SO₄ getrocknet. Das Lösungsmittel wurde im Vakuum entfernt. Als Rückstand verblieb ein helles Öl, welches über Nacht im Kühlschrank kristallisierte.
Ausbeute: 3,2 g (8,17 mmol), HPLC: 51,69 % B

### 1b)H-Pzd-βAla-Z x HCl

3,2 g (8,17 mmol) Boc-Pzd-βAla-Z wurden in Eisessig angelöst, mit 50 ml 1N HCl in Eisessig versetzt und 1h unter gelegentlichem Schütteln bei Raumtemperatur stehen gelassen. Das Lösungsmittel wurde teilweise im Vakuum entfernt und das Produkt durch Zugabe von Diethylether gefällt abgesaugt, nochmals mit Diethylether gewaschen und im Vakuum getrocknet.
Ausbeute: 2,13 g (6,5 mmol) weißer Feststoff, HPLC: 28,19 % B

### 1c) Boc-Phe(3-CN)-OH

2,5 g (13,1 mmol) H-Phe(3-CN)-OH wurden in 100 ml Dioxan gelöst und bei 0 °C mit 13 ml (13 mmol) IN NaOH und 3,16 g (14,5 mmol) Boc-Pyrocarbonat versetzt. Der Ansatz wurde, 20 min bei 0 °C und anschließend 4h bei Raumtemperatur gerührt, dabei wurden insgesamt 7ml (7 mmol) IN NaOH portionsweise zugegeben, dadurch wurde der pH-Wert auf 8-8,5 konstant gehalten. Das Lösungsmittel wurde im Vakuum entfernt, der Rückstand mit Essigester aufgenommen, jeweils 3 x mit 5 % KHSO₄ und 3 x mit NaCl-gesättigtem Wasser gewaschen und anschließend über Na₂SO₄ getrocknet. Das Lösungsmittel wurde im Vakuum entfernt. Als Rückstand wurde ein weißer Feststoff erhalten.
Ausbeute: 2,11 g (7,3 mmol) weißer Feststoff, HPLC: 45,93 % B

### 1d) Boc-Phe(3-AcOxam)-OH

2,11 g (7,3 mmol) Boc-Phe(3-CN)-OH wurden in 100 ml Methanol gelöst und mit 760 mg (10,95 mmol) Hydroxylamin x HCl und 1,9 ml (10,95 mmol) DIEA versetzt. Der Ansatz wurde 6 h unter Rückfluss gerührt. Es wurden nochmals 266 mg (3,84 mmol) Hydroxylamin x HCl und 665 µl (3,84 mmol) DIEA zugegeben und der Ansatz wurde weitere 3 h unter Rückfluß und anschließend über Nacht bei Raumtemperatur gerührt. Anschließend wurde das Lösungsmittel im Vakuum entfernt. Als Rückstand verblieb ein helles Öl, das in 50 ml Eisessig gelöst und mit 2 ml (22 mmol) Acetanhydrid versetzt wurde. Der Ansatz wird 30 min bei Raumtemperatur gerührt. Das Lösungsmittel wurde im Vakuum entfernt, der Rückstand mit Essigester aufgenommen, jeweils 3 x mit 5 % KHSO₄ und NaCl-gesättigtem Wasser gewaschen und anschließend über Na₂SO₄ getrocknet. Das Lösungsmittel wurde im Vakuum entfernt.
Ausbeute: 3,31 g (farbloses Öl), HPLC: 26,59 % B

### 1e) Boc-Phe(3-AcOxam)-Pzd-βAla-Z

0,92 g (2,8 mmol) H-Pzd-βAla-Z x HCl und 1,02 g (2,8 mmol) Boc-Phe(3-AcOxam)-OH wurden in 40 ml DMF gelöst und bei 0 °C mit 1,46 g (2,8 mmol) PyBop und 1,46 ml (8,4 mmol) DIEA versetzt. Der Ansatz wurde 20 min bei 0 °C und weitere 2h bei Raumtemperatur gerührt. Anschließend wurde das Lösungsmittel im Vakuum entfernt, der Rückstand in Essigester aufgenommen, jeweils 3 x mit 5 % KHSO₄, gesättigter NaHCO₃-Lösung und NaCl-gesättigtem Wasser gewaschen und anschließend über Na₂SO₄ getrocknet. Das Lösungsmittel wurde im Vakuum entfernt.
Ausbeute: 2,49 g (helles Öl), HPLC: 48,13 % B

### 1f) H-Phe(3-AcOxam)-Pzd-βAla-Z x HCl

2,49 g Boc-Phe(3-AcOxam)-Pzd-βAla-Z (Rohprodukt) wurden in Eisessig angelöst, mit 30 ml 1N HCl in Eisessig versetzt und 1h unter gelegentlichem Schütteln bei Raumtemperatur stehen gelassen. Das Lösungsmittel wurde teilweise im Vakuum entfernt und das Produkt durch Zugabe von Diethylether gefällt, abgesaugt, nochmals mit Diethylether gewaschen und im Vakuum getrocknet.
Ausbeute: 1,32 g (2,3 mmol) weißer Feststoff, HPLC: 32,89 % B

### 1g) Anthracensuflonyl-Phe(3-AcOxam)-Pzd-βAla-Z

Bei 0 °C wurden und 207,7 mg (0,361 mmol) H-Phe(3-AcOxam)-Pzd-βAla-Z, 127 µl (0,73 mmol) DIEA und 100 mg (0,361 mmol) Anthracensulfonylchlorid (Fluka) in 10 ml DMF gelöst. Der Ansatz wurde 20 min bei 0 °C und anschließend über Nacht bei Raumtemperatur gerührt. Das Lösungsmittel wurde im Vakuum entfernt, der Rückstand in Essigester aufgenommen, jeweils 3 x mit 5 % KHSO₄, gesättigter NaHCO₃-Lösung und NaCl-gesättigtem Wasser gewaschen und anschließend über Na₂SO₄ getrocknet. Das Lösungsmittel wurde im Vakuum entfernt. Als Rückstand blieb ein helles Öl, das ohne weitere Reinigung direkt für den nächsten Syntheseschritt eingesetzt wurde HPLC: 57,65 % B

### 1h) Anthracensuflonyl-Phe(3-Am)-Pzd-βAla

Das Rohprodukt 1g wurde in 50 ml 90 % Essigsäure und 5 ml 1 N HCl gelöst und mit 30 mg Katalysator (10% Pd/C) versetzt. Der Ansatz wurde bei 40 °C und Normaldruck über Nacht mit Wasserstoff hydriert. Anschließend wurde der Katalysator abfiltriert und das Lösungsmittel im Vakuum eingeengt. Ein Teil des Rohproduktes wurde mit präparativer reversed-phase HPLC gereinigt.
HPLC: 34,11 % B
MS: berechnet 586,24 (monoisotopic), gefunden 587,79 [M+H]⁺

### Beispiel 2: Synthese von Anthracensulflonyl-Phe(3-Am)-Pzd-CO-CH₂-CH₂-Guanidino x 2 TFA (Verbindung 11 aus Tabelle 1)

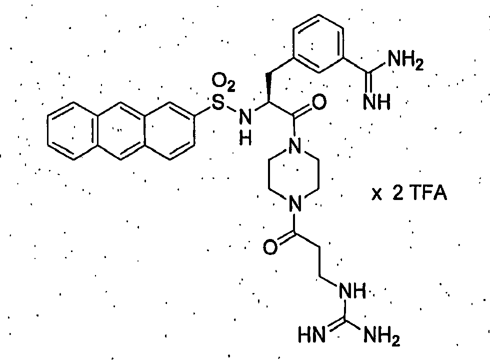

### 2a) Anthracensulfonyl-Phe(3-Am)-Pzd-CO-CH₂-CH₂-Guanidino

Ca. 115 mg Rohprodukt an Anthracensuflonyl-Phe(3-Am)-Pzd-βAla (1h) wurde in 10 ml DMF gelöst und mit 90,3 mg (0,616 mmol) Pyrazolcarboxamidin x HCl und 107 µl (0,616 mmol) DIEA versetzt. Der Ansatz wurde über Nacht gerührt und anschließend das Lösungsmittel im Vakuum entfernt. Der verbleibende Rückstand wurde im Vakuum getrocknet und ohne weitere Reinigung mit präparativer reversed-phase HPLC gereinigt.
HPLC: 35,09 % B
MS: berechnet 628,26 (monoisotopic), gefunden 629,4 [M+H]⁺

### Beispiel 3: Synthese von 2,4,6-Triisopropyl-phenylsulfonyl-Phe(3-Am)-iNip-DAE-H x 2 TFA (Verbindung 3 aus Tabelle 1)

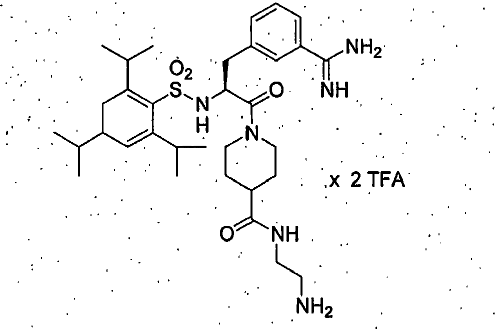

### 3a) Tips-Phe(3-CN)-OH

Zu einer Lösung von 1,05 eq. H-L-Phe(3-CN)-OH (3 g, 15,8 mmol) in Dioxan und 2,1 eq. 1M NaOH (31,5 ml) wurden unter Rühren bei RT eine Lösung von 1 eq. Tips-Cl (97 %ig, 4,69 g, 15 mmol) in Dioxan zugetropft. Dabei wurde der pH-Wert der Lösung kontrolliert und mit 1M NaOH auf pH 8-9 gehalten. Nach 4 h wurde das LM im Vakuum entfernt, der Rückstand in Essigester aufgenommen, 3 x sauer (5 % KHSO₄) und 3 x neutral (gesättigte NaCl-Lösung) gewaschen. Die organische Phase wurde über Na₂SO₄ getrocknet, das LM im Vakuum entfernt und das erhaltene Produkt aus Essigester/Hexan umkristallisiert (gelblich, kristalline Verbindung).
Ausbeute: 6,6 g (96,3 %)
HPLC: 71,1 % B

### 3b) Tips-Phe(3-OAm)-OH

Eine Lösung von 1 eq. Tips-Phe(3-CN)-OH (3 g, 6,6 mmol), 1,5 eq. Hydroxylamin x HCl (685 mg, 9,9 mmol) und 3 eq. DIEA (3,4 ml, 19,8 mmol) wurden in absolutem Ethanol gelöst, 4h unter Rückfluß gekocht und anschließend unter nachdosieren von Hydroxylamin x HCl und Base (DIEA, pH 8-9) bei RT gerührt, bis in der HPLC kein Ausgangsmaterial mehr gefunden wurde. Nach Entfernen des LM wurde der Rückstand in Essigester aufgenommen, 3 x sauer (5 % KHSO₄) und 3 x neutral (gesättigte NaCl-Lösung) gewaschen. Die organische Phase wurde über Na₂SO₄ getrocknet, das LM im Vakuum entfernt und das erhaltene Produkt aus Essigester/Hexan umkristallisiert.
Ausbeute: 1,63 g (Weiße Kristalle; 50,4 %),
HPLC: 51,0 % B

### 3c) Tips-Phe(3-AcOAm)-OH

1 eq. Tips-Phe(3-OAm)-OH (2,5 g, 5,1 mmol) wurde in 100 ml Eisessig gelöst, anschließend wurde 1,5 eq. Essigsäureanhydrid (724 µl, 7,6 mmol) zugegeben und 15 min gerührt. Nach Entfernen des LM im Vakuum fällt das Produkt als weißes Pulver an.
Ausbeute: 2,7 g (99,4 %)
HPLC: 64,5 % B

### 3d) H-iNip-DAE-Z x HCl

497 mg (2,17 mmol) Boc-Isonipecotinsäure wurden mit 250 µl (2,27 mmol) NMM in 10 ml getrocknetem THF gelöst. Bei -15°C wurden 296 µl (2,27 mmol) Isobutylchloroformiat zugegeben und der Ansatz weitere 10 min gerührt. Anschließend wurden 500 mg (2,17 mmol) N-Z-1,2-Diaminoethan x HCl und 250 µl (2,27 mmol) NMM zugegeben, der Ansatz wurde weiter 1 h bei -15 °C und dann 4h bei RT gerührt.

Das LM wurde im Vakuum entfernt, der Rückstand in Essigester aufgenommen und 3 x sauer (5 % KHSO₄), 1 x neutral (gesättigte NaCl-Lösung), 3 x basisch (NaHCO₃ gesättigt) und 3 x neutral (gesättigte NaCl-Lösung) gewaschen. Die Essigester-Phase wurde anschließend über Na₂SO₄ getrocknet und das LM im Vakuum entfernt, wobei das Produkt als amorphe Substanz anfiel (HPLC: 50,3 % B). Das Rohprodukt wurde in 20 ml 1 N Chlorwasserstoff in Eisessig gelöst und 1h bei RT stehen gelassen. Anschließend wurde das LM im Vakuum entfernt und das Produkt lyophilisiert.
Ausbeute: 722 mg
HPLC: 26,4 % B

### 3e) Tips-Phe(3-Am)-iNip-DAE-H x 2 TFA

150 mg (0,28 mmol) Tips-Phe(3-AcOAm)-OH wurden mit 97 mg (0,28 mmol) H-iNip-DAE-Z x HCl in 5 ml DMF gelöst und unter Rühren im Eisbad auf 0 °C gekühlt. Zu der gekühlten Lösung wurden 122 µl (0,70 mmol) DIEA und 154 mg (0,29 mmol) PyBOP zugegeben. Nach 15 min wurde das Eisbad entfernt und weitere 2 h bei RT gerührt. Anschließend wurde das LM im Vakuum entfernt und der Rückstand in Essigester aufgenommen und 3 x sauer (5 % KHSO₄), 1 x neutral (gesättigte NaCl-Lösung), 3 x basisch (NaHCO₃ gesättigt) und 3 x neutral (gesättigte NaCl-Lösung) gewaschen. Die Essigester-Phase wurde anschließend über Na₂SO₄ getrocknet und das LM im Vakuum entfernt. Der Rückstand wurde in 90 %igem Eisessig gelöst, mit 10 Gewichtsprozent Katalysator (10 % Pd/C) versetzt und mit Wasserstoff bei RT über Nacht hydriert. Der Katalysator wurde abfiltriert und das LM im Vakuum bis zur Trockene entfernt und der Rückstand mittels präparativer reversed-phase HPLC gereinigt.
Ausbeute: 92 mg
HPLC: 43,3 % B
MS: berechnet 626,36 (monoisotopic), gefunden 628,1 [M+H]⁺

### Beispiel 4: Synthese von 2,4,6-Triisopropyl-phenylsulfonyl-Phe(3Am)-iNip-NH-CH₂-CH₂-Guanidino x 2 TFA (Verbindung 34 aus Tabelle 1)

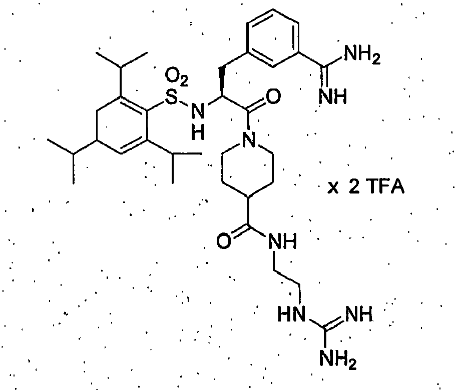

### 4a) Tips-L-Phe(3-Am)-iNip-NH-CH₂₋CH₂-Guanidino x 2 TFA

85 mg (0,1 mmol) Tips-Phe(3-Am)-iNip-DAE-H x 2 TFA wurden in 5 ml DMF gelöst und mit 30 mg (0,2 mmol) Pyrazolcarboxalmidin und 55 µl (0,3 mmol) DIEA versetzt. Der Ansatz wurde über Nacht bei RT gerührt. Anschließend wurde das Lösungsmittel im Vakuum entfernt und der Rückstand mit präparativer reversed-phase HPLC gereinigt.
HPLC: 43,7% B
MS: berechnet 668,38 (monoisotopic), gefunden 669,8 [M+H]⁺

### Beispiel 5: Synthese von 2-Nas-Phe(3-Am)-4(Aminoethyl)piperidid x 2 TFA (Verbindung 36 aus Tabelle 1)

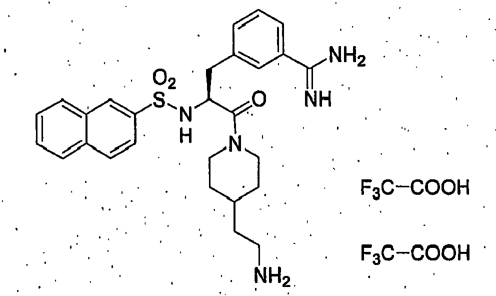

### 5a) 2-Nas-Phe(3-CN)-OH

Zu 1,9 g (10 mmol) H-Phe(3CN)-OH, gelöst in 100 ml Dioxan/Wasser-Gemisch und 22 ml 1 N NaOH-Lösung, wurden bei 0 °C 2,49 g (11 mmol) 2-Nas-chlorid (gelöst in Dioxan) über einen Zeitraum von 30 min zugetropft. Der Ansatz wurde 1 h bei 0 °C und weiter über Nacht bei Raumtemperatur gerührt. Anschließend wurde das Lösungsmittel im Vakuum entfernt und der Rückstand in Wasser gelöst (mit NaOH auf pH 8-9 eingestellt). Die Wasserphase wurde 2 x mit Diethylether extrahiert und anschließend der pH mit 1 N HCl auf pH 3-4 eingestellt. Das Produkt wurde 3 x mit Essigester extrahiert und die Essigesterphase jeweils 3 x mit 5 % KHSO₄ und NaCl-gesättigtem Wasser gewaschen und anschließend über Na₂SO₄ getrocknet. Das Lösungsmittel wurde im Vakuum entfernt. Es verblieb ein helles Öl, das im Kühlschrank auszukristallisierte.
Ausbeute: 3,51 g (9 mmol), HPLC: 51,02 % B

### 5b) 2-Nas-Phe(3-AcOxam)-OH

3,5 g (9 mmol) 2-Nas-Phe(3CN)-OH wurden in 100 ml Methanol gelöst und mit 1,04 g (15 mmol) Hydroxylamin x HCl und 2,61 ml (15 mmol) DIEA versetzt. Der Ansatz wurde 6 h unter Rückfluss gerührt. Danach wurden nochmals 700 mg (10 mmol) Hydroxylamin x HCl und 1,74 ml (10 mmol) DIEA zugegeben. Der Ansatz wurde weitere 4 h unter Rückfluß und anschließend über Nacht bei Raumtemperatur gerührt. Anschließend wurde das Lösungsmittel im Vakuum entfernt. Als Rückstand verblieb ein helles Öl, das in 50 ml Eisessig gelöst und mit 2,83 ml (30 mmol) Acetanhydrid versetzt wurde. Der Ansatz wurde 1 h bei Raumtemperatur gerührt. Das Lösungsmittel wurde im Vakuum entfernt, der Rückstand mit Essigester aufgenommen, 1 x mit 5 % KHSO₄-Lösung und 3 x mit NaCl-gesättigtem Wasser gewaschen und anschließend über Na₂SO₄ getrocknet. Das Lösungsmittel wurde im Vakuum nahezu entfernt. Das Produkt begann langsam auszukristallisieren und wurde abgesaugt.
Ausbeute: 3,02 g (6,64 mmol) heller Feststoff, HPLC: 44,04 % B

### 5c) 2-Nas-Phe(3-AcOxam)-4(Aminoethyl)piperidid x HCl

100 mg (0,22 mmol) 2-Nas-Phe(3-AcOxam)-OH und 50 mg (0,22 mmol) 4-(2-Boc-Aminoethyl)piperidin (Tyger Scientific Inc., Princeton, NY) wurden in 10 ml DMF gelöst und bei 0 °C mit 115 mg (0,22 mmol) PyBop und 115 µl (0,22 mmol) DIEA versetzt. Der Ansatz wurde 20 min bei 0 °C und weitere 3 h bei Raumtemperatur gerührt. Anschließend wurde das Lösungsmittel im Vakuum entfernt. Der Rückstand wurde mit Essigester aufgenommen, 2 x mit 5 % KHSO₄-Lösung, 1 x mit NaCl-gesättigtem Wasser, 2 x mit ges. NaHCO₃-Lösung und 3 x mit NaCl-gesättigtem Wasser gewaschen und anschließend über Na₂SO₄ getrocknet. Das Lösungsmittel wurde im Vakuum entfernt.

Das Rohprodukt wurde in Eisessig angelöst, mit 5 ml 1N HCl in Eisessig versetzt und 1h unter gelegentlichem Schütteln bei Raumtemperatur stehen gelassen. Das Lösungsmittel wurde im Vakuum entfernt, es verblieb ein helles Öl.
Ausbeute: 105 mg Öl, HPLC: 35,66 % B

### 5d) 2-Nas-Phe(3-Am)-4(Aminoethyl)pieridid x 2 TFA

Das Rohprodukt 5c wurde in 50 inl 90 % Essigsäure gelöst und mit 15 mg Katalysator (10% Pd/C) versetzt. Der Ansatz wurde bei 40 °C und Normaldruck über Nacht mit Wasserstoff hydriert. Anschließend wurde der Katalysator abfiltriert und das Lösungsmittel im Vakuum eingeengt. Ein Drittel des Rohproduktes wurde mit präparativer reversed-phase HPLC gereinigt.
Ausbeute: 17,6 mg HPLC: 29,03 % B
MS: berechnet 507,23 (monoisotopic), gefunden 508,4 [M+H]⁺

### Beispiel 6: Synthese von 2-Nas-Phe(3-Am)-4(Guanidinoethyl)piperidid x 2 TFA (Verbindung 37 aus Tabelle 1)

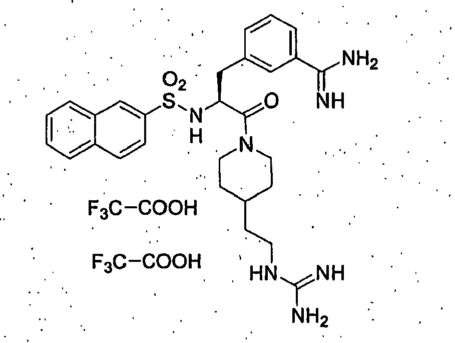

### 6a) 2-Nas-Phe(3-Am)-4(Guanidmoethyl)piperidid x 2 TFA

Ca. 80 mg Rohprodukt an 2-Nas-Phe(3-AcOxam)-4(Aminoethyl)piperidid x HCl (5d) wurde in 5 ml DMF gelöst und mit 65 mg (0,45 mmol) Pyrazolcarboxamidin x HCl und 105 µl (0,6 mmol) DIEA versetzt. Nach 3 h wurden nochmals 21,5 mg (0,15 mmol) Pyrazolcarboxamidin x HCl und 35 µl (0,15 mmol) DIEA zugegeben und der Ansatz weiter über Nacht gerührt. Das Lösungsmittel wurde im Vakuum entfernt und der verbleibende Rückstand mit präparativer reversed-phase HPLC gereinigt.
Ausbeute: 42 mg HPLC: 31,19 % B
MS: berechnet 549,25 (monoisotopic), gefunden 550,4 [M+H]⁺

### Beispiel 7: Herstellung der katalytischen Domäne von Matriptase

**Klonierung:** Die katalytische Domäne von Matriptase wurde mit folgendem Primerpaar durch PCR amplifiziert:
Sense Primer:
   5'- GGCAATTC**CATATG**AAACATCACCATCATCACC**AT***GTTGTTGGGGGCACGGATGCG*-3'
Antisense Primer:
   5'- GCAT**GAATTC***TTATACCCCAGTGTTCTCTTTGATCCA*-3

Sense Primer und, Antisense Primer wurden so, gewählt, dass am 5'-Ende vor der Proteasedomäne (kursiv) eine Ndel Schnittstelle (fett) gefolgt von der Peptidsequenz Met Lys (His)₆ bzw. an das 3'-Ende von Matriptase (kursiv) eine EcoR1 Schnittstelle (fett) eingefügt wurden. Das PCR Produkt wurde über Nde1 und EcoR1 in pET24 (Novagen), einen Vektor für Expression in *Escherichia coli,* kloniert.

Die katalytische Domäne von Matriptase wurde in inaktiver und unlöslicher Form in *Escherichia coli* exprimiert, gereinigt, rückgefaltet und anschließend aktiviert Die Schritte waren im Einzelnen:
**Expression und Reinigung:** BL21 (DE3) Zellen (Novagen), die den Vektor aus der oben beschriebenen.Klonierung enthielten, wurden in LB, 30 µg/ml Kanamycin bei 37 °C und 250 rpm inkubiert. Die Expression wurde bei einer OD₆₀₀ von 0,6 durch Zugabe von 1 mM IPTG induziert und die Inkubation für eine Stunde fortgesetzt. Dann wurden die Zellen pelletiert, mit 5 ml Bug Buster^{™} Protein Extraction Reagent (Novagen) aufgeschossen und die DNA mit 25 U/ml pro 1 g Zellpellet Benzonase^{®} Nuclease (Novagen) verdaut. Die Proteinaggregate wurden gewaschen und mit 5 ml Denaturierungspuffer (6 M.Guanidinium HCL, 10 mM Tris HCl, 100 mM Na Phosphat, pH 8,0) pro 1 g Pellet denaturiert. Unlösliche Bestandteile wurden abzentrifugiert (16 000g, 30 min, 20°C), der Überstand filtriert (0,2 µm), mit 10 mM β-Mercaptoethanol versetzt und anschließend zur Reinigung der katalytischen Domäne von Matriptase auf eine Metal-Chelat-Chromatographie Säule gegeben (1 ml NiNTA (Qiagen) pro 10 ml Überstand).
   Die Säule wurde gewaschen (8 M Urea, 10 mM Tris HCl, 100 mM Na Phosphat, pH 6,3) und das angereinigte Protein mit 8 M Urea, 10 mM Tris HCl, 100 mM Na Phosphat, pH 4,5 eluiert.
**Rückfaltung:** Die Matriptase enthaltenden Fraktionen wurden vereinigt, mit Glutathion derivatisiert und anschließend zur Rückfaltung in einer Endkonzentration von 50 µg/ml in Rückfaltungspuffer (50 mM Tris HCl, 0,5 M L-Arginin, 20 mM, CaCl₂, 1 mM EDTA, 0,1 M NaCl, pH 7,5) verdünnt. Nach 3-tägiger Inkubation bei Raumtemperatur wurde der Rückfaltungsansatz filtriert, auf eine Konzentration von > 300 µg/ml aufkonzentriert (Centricon Plus-80, Amicon) und mittels Gelfiltration (PD 10 Säulen, Pharmacia) in Aktivierungspuffer (20 mM Na Phosphat, 150 mM NaCl, pH 7,0) umgepuffert.
**Aktivierung:** Da ein korrekt prozessierter N-Terminus Voraussetzung für die Aktivität von Serinproteasen ist, musste zur Aktivierung der rückgefalteten Matriptase das Peptid MK(His)₆ am N-Terminus entfernt werden. Hierzu wurde der Rückfaltungsansatz für 2 h bei 30°C mit 2,5 mU pro 50 µg Protein aktivierter DAPase^{™} (Qiagen) inkubiert und die aktivierte Matriptase von der nicht aktivierten Matriptase und der DAPase über Metalchelat Chromatographie getrennt.

Die Ausbeute an aktiver Matriptase betrug ca. 0,9 mg/l Bakterienkultur. Die proteolytische. Aktivität wurde durch Spaltung des chromogenen Substrates Pefachrome tPA (Pentapharm) nachgewiesen.

### Beispiel 8: Bestimmung der Hemmwirkung von Matriptase mit den in Tabelle 1 aufgelisteten Inhibitoren

Zur Bestimmung der Hemmwirkung wurden 200 µl Tris-Puffer (0,05 M, 0,154 M NaCl, 5 % Ethanol, pH 8,0; enthält den Inhibitor), 25 µl Substrat (CH₃SO₂-D-HHT-Gly-Arg-pNA; 2 und 1 mM) und 50 µl Matriptase (0,5 µg/ml) bei 25 °C inkubiert. Nach 3 min wurde die Reaktion durch Zugabe von 25 µl Essigsäure (50%) unterbrochen und die Absorption bei 405 nm mittels Microplate Reader (Dynatech MR 5000) bestimmt. Die Kᵢ-Werte wurden nach Dixon (Biochem. J. 55, 170-171, 1953) durch lineare Regression mittels eines Computerprogramms ermittelt. Die Kᵢ-Werte (Tabelle 1) sind das Mittel aus mindestens drei Bestimmungen.

**Tabelle 1: Bestimmung der Kᵢ-Werte für die Hemmung von Matriptase**

| **Nr.** | **Inhibitorstruktur** | **K₁/**µ**M** |
|---|---|---|
| 1 | 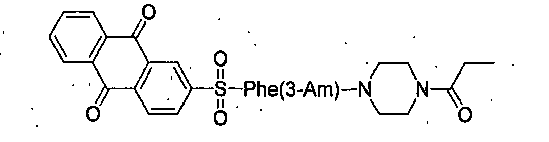 | 1,5 |
| 2 | 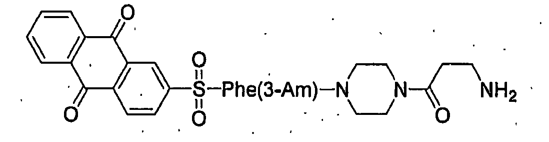 | 0,16 |
| 3 | 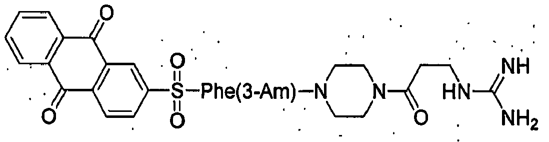 | 0,053 |
| 4 | 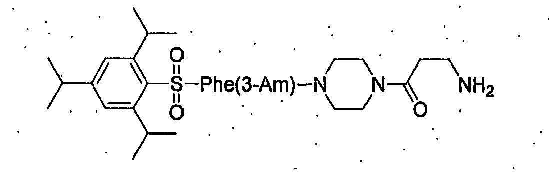 | 0,14 |
| 5 | 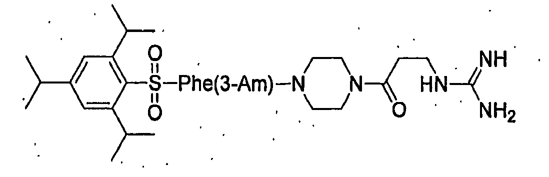 | 0,057 |
| 6 | 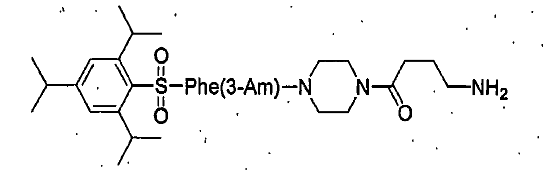 | 0,14 |
| 7 | 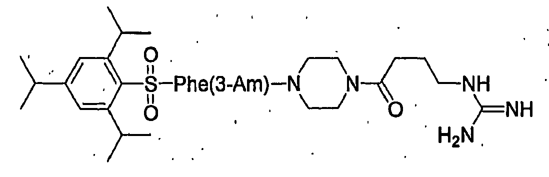 | 0,056 |
| 8 | 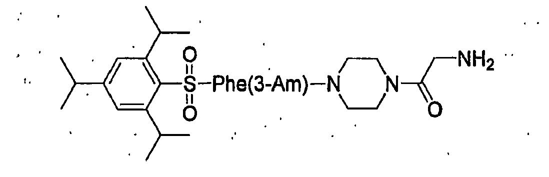 | 0,69 |
| 9 | 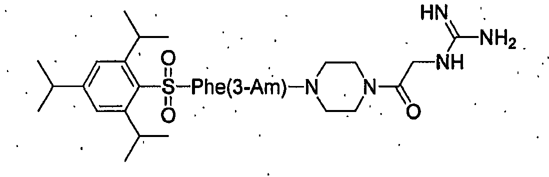 | 0,33 |
| 10 | 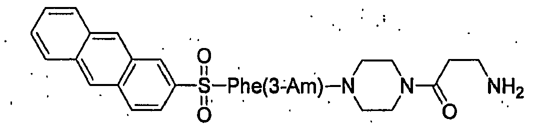 | 0,27 |
| 11 | 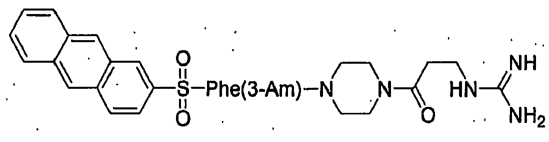 | 0,044 |
| 12 | 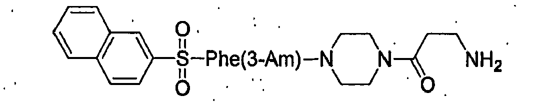 | 0,83 |
| 13 | 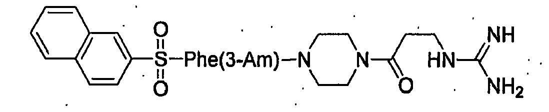 | 0,21 |
| 14 | 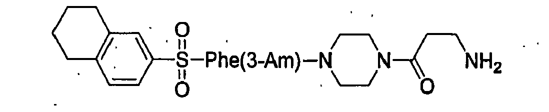 | 0,40 |
| 15 | 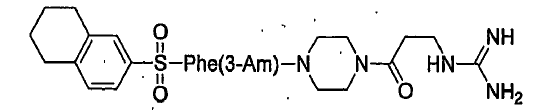 | 0,3 |
| 16 | 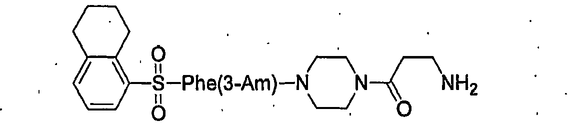 | 0,99 |
| 17 | 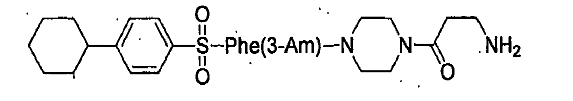 | 0,14 |
| 18 | 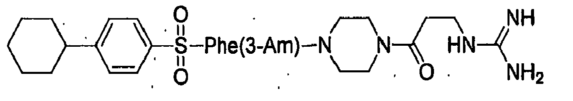 | 0,089 |
| 19 | 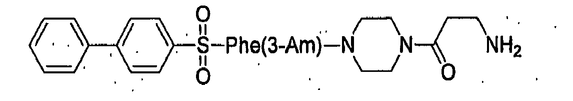 | 0,60 |
| 20 | 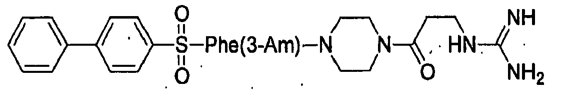 | 0,25 |
| 21 | 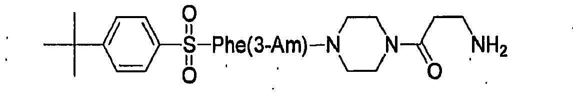 | 0,47 |
| 22 | 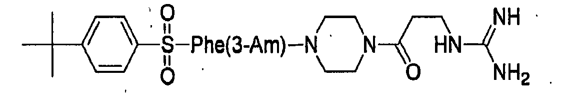 | 0,2 |
| 23 | 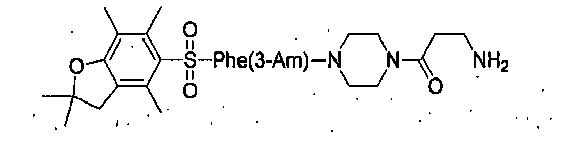 | 3,3 |
| 24 | 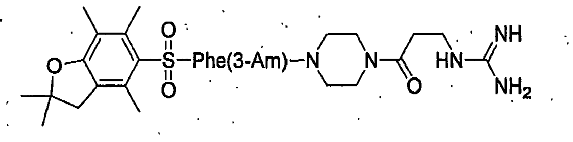 | 0,73 |
| 25 | 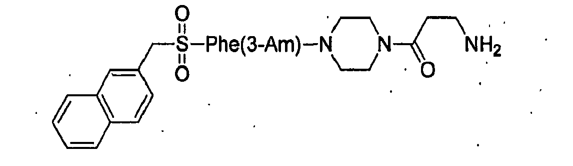 | 2,4 |
| 26 | 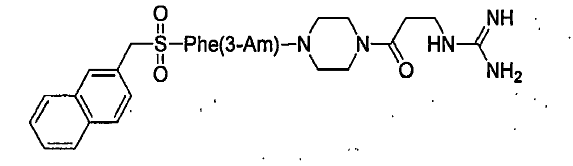 | 0,4 |
| 27 | 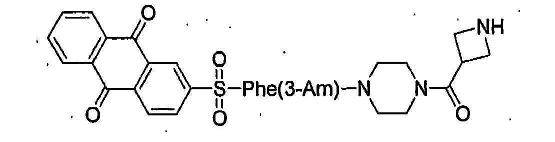 | 0,46 |
| 28 | 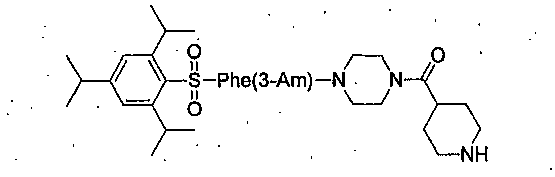 | 0,11 |
| 29 | 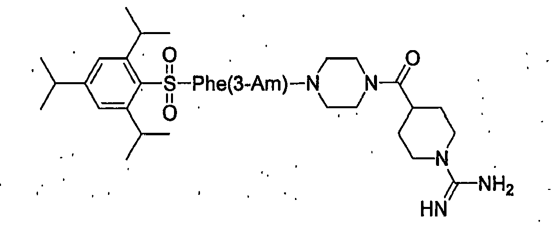 | 0,014 |
| | | |
| 30 | 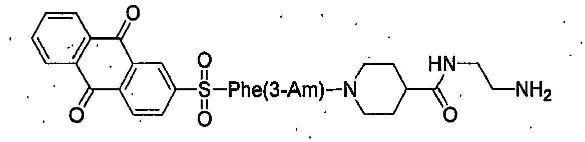 | 0,021 |
| 31 | 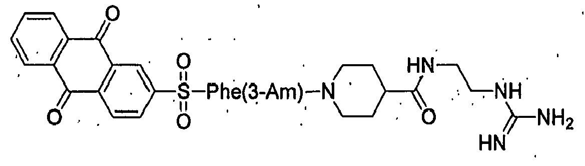 | 0,013 |
| 32 | 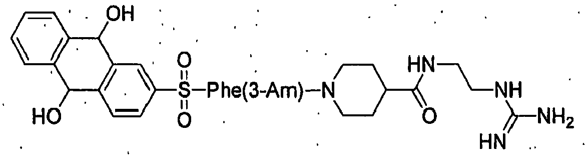 | 0,0098 |
| 33 | 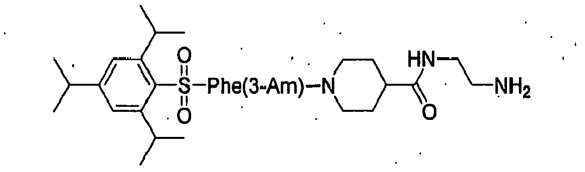 | 0,38 |
| 34 | 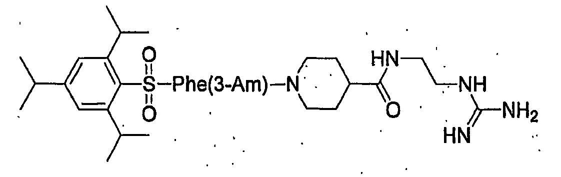 | 0,14 |
| 35 | 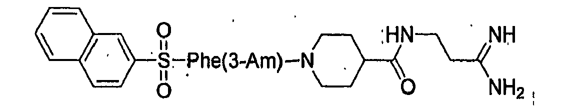 | 0,16 |
| 36 | 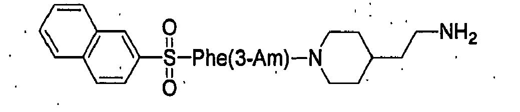 | 0,11 |
| 37 | 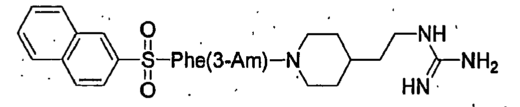 | 0,046 |
| | | |
| 38 | 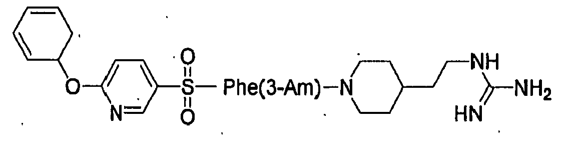 | 0,013 |
| 39 | 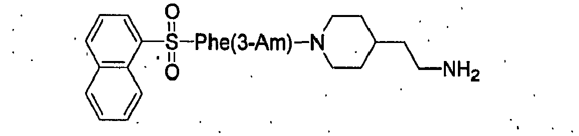 | 0,17 |
| 40 | 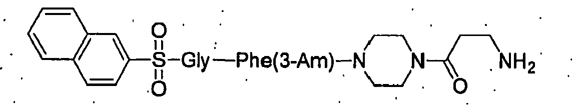 | 7,2* |
| 41 | 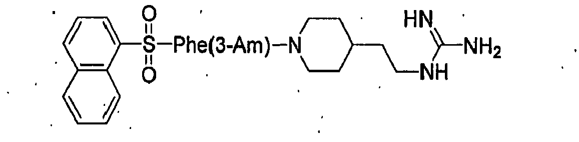 | 0,074 |
| 42 | 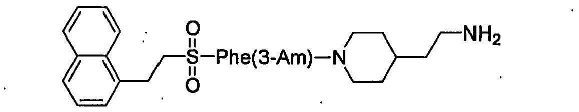 | 0,36 |
| 43 | 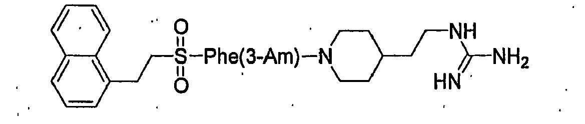 | 0,18* |
| 44 | 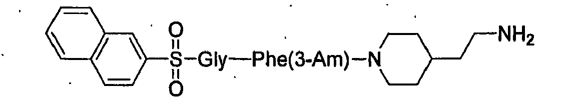 | 21 |
| 45 | 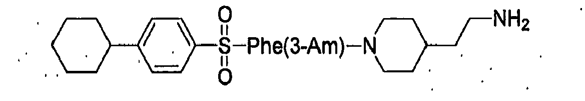 | 0,14 |
| 46 | 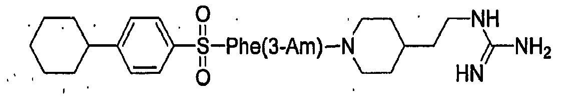 | 0,088 |
| 47 | 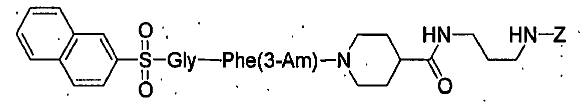 | 3,9 |
| 48 | 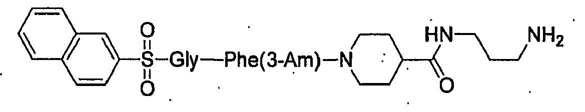 | 0,36 |
| 49 | 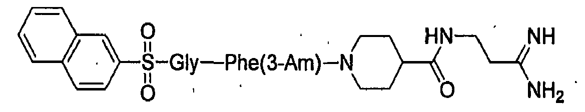 | 0,16 |
| | | |
| 50 | 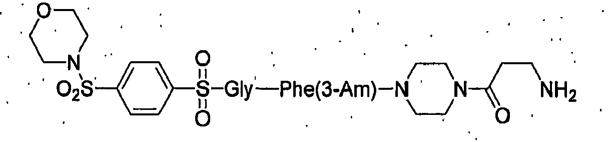 | 3,5 |
| 51 | 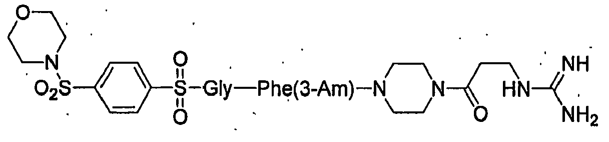 | 0,89 |
| 52 | 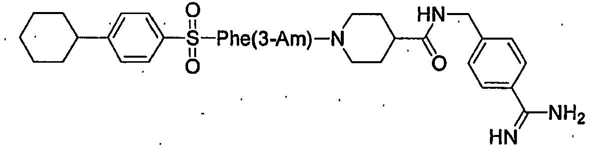 | |
| 53 | 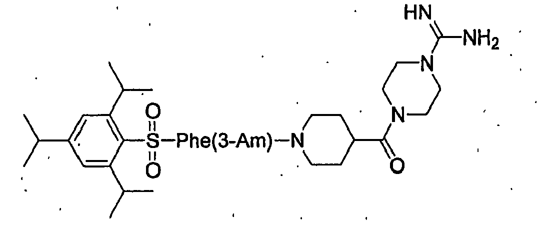 | 0,073 |
| 54 | 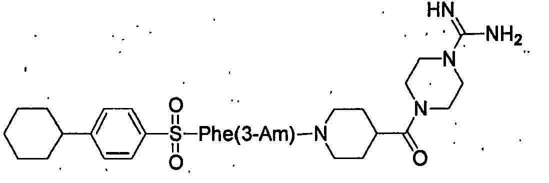 | 0,032 |
| 55 | 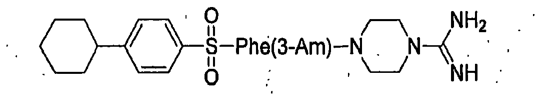 | 0,98 |
| 56 | 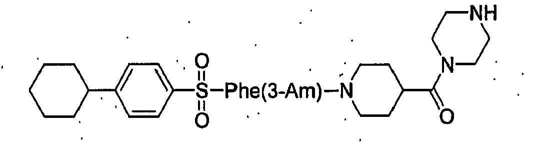 | 0,24 |
| 57 | 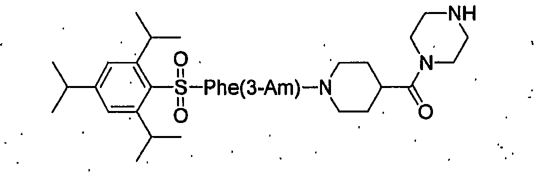 | 0,086 |
| 58 | 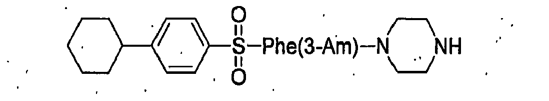 | 0,55 |
| 59 | 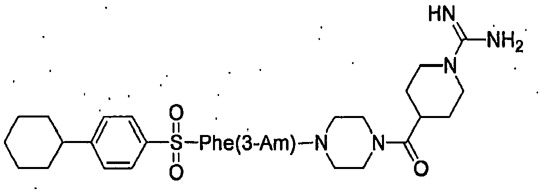 | 0,061 |
| 60 | 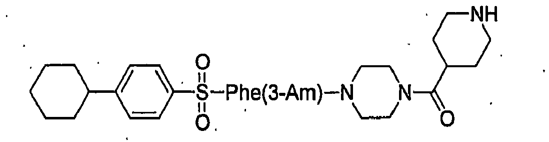 | 0,31 |
| 61 | 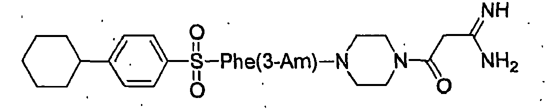 | 0,38 |
| 62 | 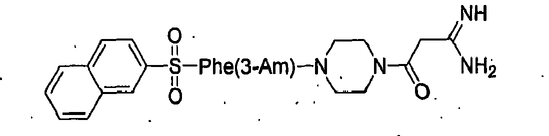 | 1,1 |
| 63 | 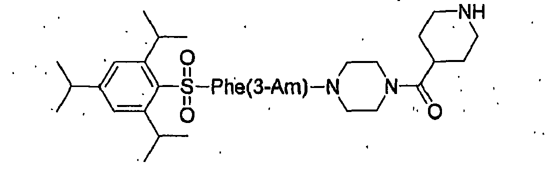 | 0,1 |
| 64 | 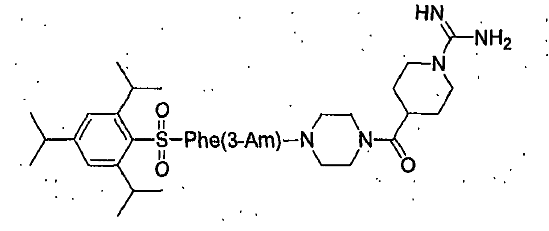 | 0,014 |
| 65 | 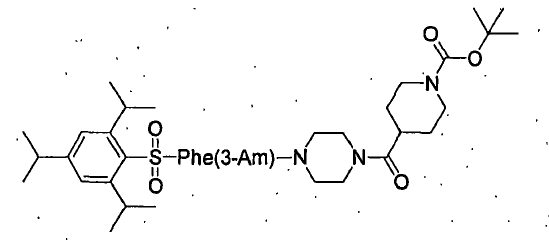 | 0,83 |
| 66 | 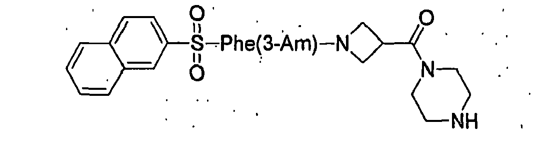 | 13 |
| 67 | 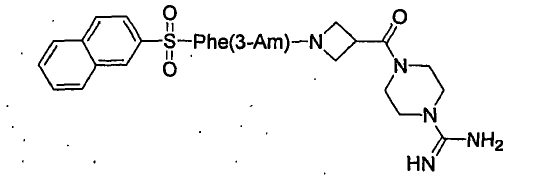 | 3,4 |
| 68 | 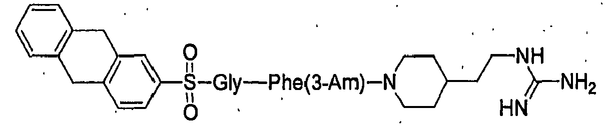 | 0,038 |
| 69 | 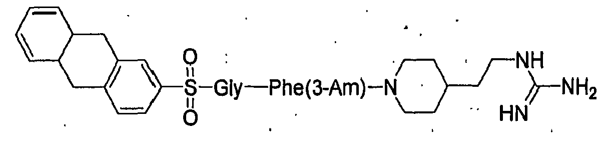 | 0,037 |
| 70 | 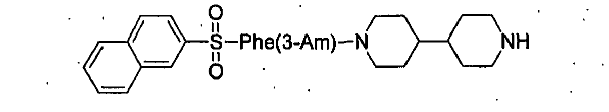 | 0,12 |
| 71 | 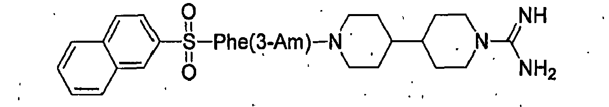 | 0,45 |

### Beispiel 9: Hemmung des invasiven Wachstums durch Matriptase Inhibitoren (Matrigel Assay)

Ein gängiges Testsystem für invasives Wachstum auf zellulärere Ebene ist der Matrigel-Invasionsassay. Dabei werden Zellen auf eine künstliche extrazelluläre Matrix aufgebracht und untersucht, wie viele Zellen diese innerhalb eines bestimmten Zeitraumes durchwandern.

Beispielhaft ist hier für die Matriptase Inhibitoren 37 und 54 gezeigt, dass das invasive Wachstum beeinflusst und die Wanderung von Matriptase-exprimierenden Kolonkarzinomzelllinie DLD-1 durchs Matrigel gehemmt wird:
Die Vertiefungen einer 'Transwell' Platte wurden mit je 10 µg Matrigel beschichtet, je 160 000 DLD-1 Zellen (Dexter et al, Cancer Research 39: 1020-1025 (1979)) in 100 µl Medium (RPMI 1640, mit 2 % Serumersatz Ultroser HY) wurden aufgebracht und das invasive Wachstum durch die Zugabe von ProHGF in 400 µl Medium mit und ohne Inhibitor (30 µM) stimuliert. Nach 48-stündiger Inkubation bei 37°C und 5 % CO₂ wurden die Zellen, die durch die Matrix gewandert waren, fixiert, angefärbt und bei 100-fächer Vergrößerung fotografiert.

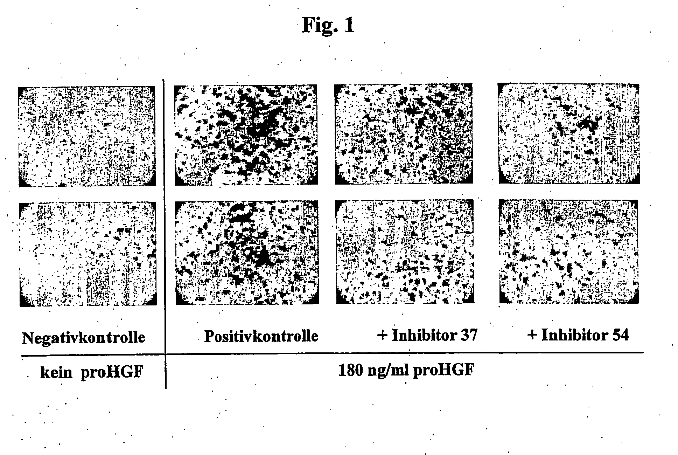

Wie Figur 1 zeigt, wird die Invasion der DLD-1 Zellen durch die extrazelluläre Matrix durch die Zugabe von proHGF stimuliert. Dies deutet darauf hin, dass das Zymogen an der Zelloberfläche aktiviert wird. Dieser Effekt wird durch die Matriptase Inhibitoren deutlich gehemmt.

### Beispiel 10: Hemmung, des-Zell-Scatterings durch Matriptase Inhibitoren

Aufgrund seiner Fähigkeit, das Ablösen von Zellen vom inselförmigen Verband sowie die Ausbreitung der Zellen zu induzieren, wird HGF auch als 'scatter factor bezeichnet. Diese Funktion kann auf zellulärer Ebene mit Hilfe des sogenannte 'Scatter Assays' nachgewiesen werden. Hierbei werden Zellen ausgesät und ihre .durch die Zugabe von, HGF stimulierte Ausbreitung nach einem bestimmten Zeitpunkt dokumentiert. HGF kann durch die Aktivierung der inaktive Proform von HGF (proHGF) gebildet werden.

Im Folgenden wird beispielhaft für die Matriptase Inhibitoren 37 und 54 gezeigt, dass die Aktivierung von proHGF durch zelluläre Matriptase verhindert und das 'Scattering' von proHGF stimulierten Zellen reduziert wird.

Hierfür wurden je 500 Matriptase-exprimierende Prostatakärzinomzellen (PC-3) pro Vertiefung einer 96-well Platte ausgesät, über Nacht in 100 µl Medium ohne fötales Kälberserum (Nut Mix. F-12, 2% Ultroser HY) inkubiert (37°C, 5% CO₂ und dann das 'Scattering' mit und ohne Matriptase Inhibitoren (3 µM) durch die Zugabe von proHGF stimuliert. Nach 6 Tagen wurden die Zellen fixiert, gefärbt und repräsentative .Ausschnitte bei 100-facher Vergrößerung fotografiert.

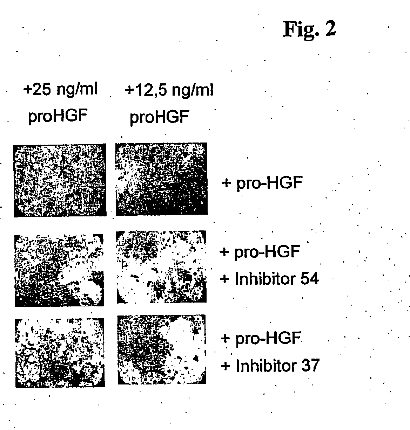

Wie Figur 2 zeigt, hemmen die Matriptase Inhibitoren 37 und 54 das proHGF induzierte 'Scattering' von PC-3 Zellen.

In den verwendeten Konzentrationen haben weder proHGF noch die Inhibitoren einen Einfluss auf die Proliferation von PC-3 Zellen. Dies spricht dafür, dass der gezeigte

Effekt auf einem veränderten 'Scatter'-Verhalten und nicht einer veränderten Verdopplungszeit der Zellen beruht.

Die folgenden Seiten 74 bis 88 beinhalten spezielle Ausführungsbeispiele.
1. Verbindung gemäß Formel (I) 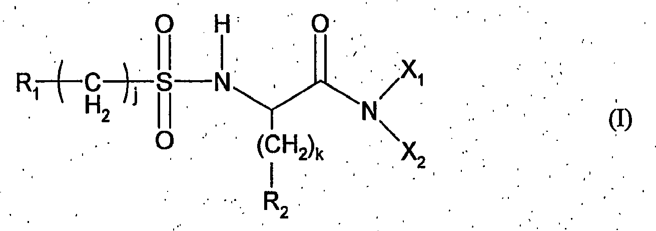 oder ein Salz oder ein Prodrug dieser Verbindung, wobei
   (a) X₁ und X₂ unabhängig voneinander Wasserstoff oder ein Alkylrest mit 1,2 oder 3 C-Atomen sind und mindestens einer der Reste X₁ und X₂ ein Rest der Struktur (I') 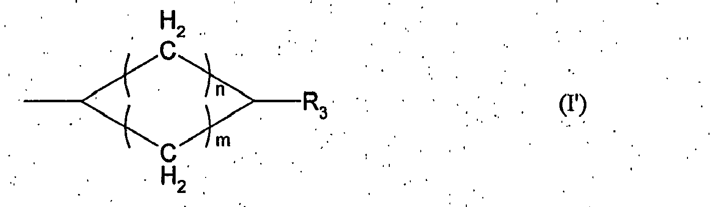 ist, wobei
      - gegebenenfalls mindestens eine der mit m oder n indizierten Methylengruppen gemäß (I') mindestens einfach mit einer Hydroxyl-, einer Halogen-, einer Pseudohalogen- oder einer COOR₂'-Gruppe substituiert ist und R₂' eine lineare, verzweigte oder cyclische Alkylgruppe mit 1 bis 10 C-Atomen ist, und/oder
      - gegebenenfalls mindestens eines der C-Atome der mit m oder n indizierten Methylengruppen gemäß (I') durch S, N oder O ersetzt ist und/oder
      - gegebenenfalls mindestens eine der den Cyclus 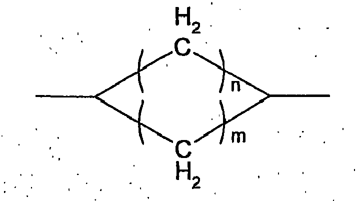 gemäß (I') bildenden Bindungen eine Doppelbindung ist, oder wobei
   (b) X₁ und X₂ derart zu einem Cyclus verbrückt sind, dass die Verbindung gemäß (I) die Struktur (I") 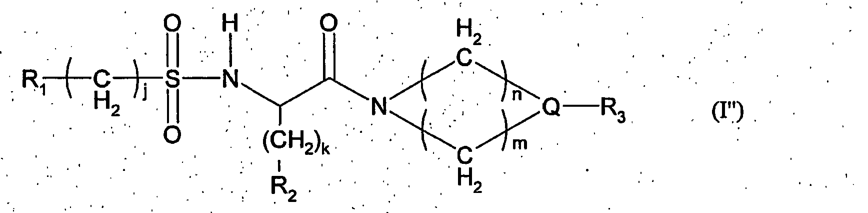 aufweist, wobei
      - gegebenenfalls mindestens eine der mit m oder n indizierten Methylengruppen gemäß (I") mindestens einfach mit einer Hydroxyl-, einer Halogen-, einer Pseudohalogen- oder einer COOR₂'-Gruppe substituiert ist und R₂' eine lineare, verzweigte oder cyclische Alkylgruppe mit 1 bis 10 C-Atomen ist, und/oder
      - gegebenenfalls mindestens eines der C-Atome der mit m oder n indizierten Methylengruppen gemäß (I") durch S, N oder O ersetzt ist und/oder
      - unter Beibehaltung der C-terminal an die sulfonylierte Aminosäure gebundenen Iminogruppe gegebenenfalls mindestens eine der den Cyclus 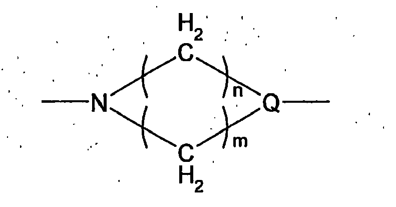 gemäß (I") bildenden Bindungen eine Doppelbindung ist, und wobei
         (i) R₁ eine gegebenenfalls teilweise hydrierte Aryl- oder Heteroarylgruppe, Atomen, oder eine lineare, verzweigte oder cyclische Alkylgruppe mit 1 bis 10 C-Atomen ist, wobei R₁ gegebenenfalls substituiert ist mit
      - mindestens einer Halogen- und/oder Pseudohalogengruppe und/oder
      - mindestens einer linearen, verzweigten oder cyclischen Alkyl- oder Alkyloxy- oder Alkylthiogruppe mit 1 bis 10 C-Atomen, die gegebenenfalls mindestens einfach substituiert ist mit einer Halogen-, Pseudohalogen-, Hydroxy-, Amino-, Cyano-, Amidino-, Guanidino oder Carboxylgruppe, wobei die Carboxylgruppe gegebenenfalls mit einer linearen, verzweigten oder cyclischen Alkylgruppe mit,1 bis 10 C-Atomen verestert ist, und wobei die lineare, verzweigte oder cyclische Alkylgruppe mit 1 bis 10 C-Atomen gegebenenfalls mindestens ein Heteroatom, ausgewählt aus der Gruppe bestehend aus O, N und S, enthält, und/oder
      - mindestens eine Aryl- oder Heteroarylgruppe mit 5 bis 20 -Atomen, wobei diese Aryl- oder Heteroarylgruppe gegebenenfalls substituiert ist mit
         -- mindestens einer linearen, verzweigten oder cyclischen Alkylgruppe mit 1 bis 10 C-Atomen und/oder
         -- mindestens einer COR₂'- und/oder COOR₂'-Gruppe, wobei R₂' eine lineare, verzweigte oder cyclische Alkylgruppe mit 1 bis 10 C-Atomen ist, und/oder
         -- mindestens einer Halogengruppe und/oder
         -- mindestens einer Pseudohalogengruppe und/oder
         -- mindestens einer Alkoxygruppe oder einer Alkylthiogruppe, wobei der Alkylrest jeweils 1 bis 10 C-Atome aufweist, und/oder
         -- - mindestens einer Nitrogruppe und/oder
         -- mindestens einer Halogenalkylgruppe mit 1 bis 10 C-Atomen, und wobei die Aryl- oder Heteroarylgruppe über eine Alkylengruppe mit 1 bis. 3 C-Atomen oder ein Sauerstoffatom oder über ein Schwefelatom an den Rest R₁ gebunden ist;
            mindestens einer Hydroxy-, Amino-, Cyano-, Amidino-, Guanidino-, Carboxyl- oder Carboxyalkylgruppe, wobei die Aminogruppe gegebenenfalls acyliert ist und/oder wobei die Alkylgruppe der Carboxylkylgruppe 1 bis 10 C-Atome aufweist und/oder die Carboxylgruppe gegebenenfalls mit einer linearen, verzweigten oder cyclischen Alkylgruppe mit 1 bis 10 C-Atomen verestert oder amidiert ist;
            (ii) R₂ eine mindestens einfach substituierte Arylgruppe mit 1 bis 10 C-Atomen ist, wobei
      - gegebenenfalls mindestens eines dieser C-Atome durch S, N oder O ersetzt ist,
      - mindestens ein Substituent eine Gruppe gemäß R₄ ist,
         R₂ gegebenenfalls zusätzlich mit einer Hydroxy-, COR₂'- oder COOR₂'-Gruppe substituiert ist und R₂' eine lineare, verzweigte oder cyclische Alkylgruppe mit 1 bis 10 C-Atomen ist;
         (iii) R₃ einen Rest der folgenden Formel (II) darstellt: 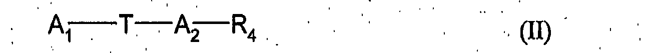 wobei
      - A₁ entweder nicht vorhanden oder eine Alkylengruppe mit 1 bis 4 C-Atomen ist, die gegebenenfalls substituiert ist mit
         -- mindestens einer Halogen- und/oder Pseudohalogengruppe und/oder
         -- mindestens einer linearen, verzweigten oder cyclischen Alkylgruppe mit 1 bis 10 C-Atomen und/oder
         -- mindestens einer Aryl- oder einer Aralkylgruppe mit 5 bis 10 C-Atomen und/oder
         -- mindestens einer Cycloalkylgruppe mit 3 bis 10-Atomen und/oder-
         -- mindestens einer Hydroxy-, Cyano-, Alkyloxy- oder Alkylthio mit 1 bis 10 C-Atomen, Carboxyl- oder Carboxyalkylgruppe, wobei die Alkylgruppe der Carboxyalkylgruppe 1 bis 10 C-Atome aufweist und/oder die Carboxylgruppe gegebenenfalls
            mit einem linearen, verzweigten oder cyclischen Alkylrest mit 1 bis 10 C-Atomen verestert oder amidiert ist; T entweder nicht vorhanden oder eine der folgenden Gruppen ist: 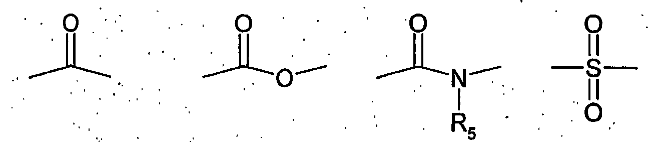 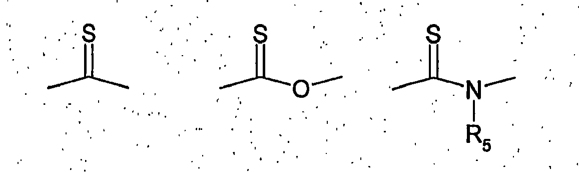 wobei R₅ Wasser-stoff oder eine Alkylgruppe mit 1 bis 10 C-Atomen oder eine mit A₂ einen gegebenenfalls mindestens ein Heteroatoms enthaltenden Cyclus bildende Alkylengruppe mit 1 bis 6 C-Atomen ist;
      - A₂ eine lineare, verzweigte oder cyclische Alkylengruppe mit 1 bis 10 C-Atomen oder eine Aryl-, Heteroaryl- oder Aralkylengruppe mit 1 bis 10 C-Atomen, gegebenenfalls enthaltend mindestens ein Hetero-atom, ausgewählt aus der Gruppe bestehend aus N, S und O, ist, die gegebenenfalls substituiert ist mit
         -- mindestens einer Halogen- und/oder Pseudohalogengruppe und/oder
         -- mindestens einer linearen, verzweigten oder cyclischen Alkylgruppe mit 1 bis 10 C-Atomen und/oder
         -- mindestens einer Aryl- oder einer Aralkylgruppe mit 5 bis 10 C-Atomen und/oder
         -- mindestens einer Cycloalkylgruppe mit 3 bis 10 C-Atomen und/oder-
         -- mindestens einer Hydroxy-, Cyano-, Alkyloxy- oder Alkylthiomit 1 bis 10 C-Atomen, Carboxyl- oder Carboxyalkylgruppe, wobei die Alkylgruppe der Carboxyalkylgruppe 1, bis 10 C-Atome aufweist und/oder die Carboxylgruppe gegebenenfalls mit einem linearen, verzweigten oder cyclischen Alkylrest mit 1 bis 10 C-Atomen verestert oder amidiert ist;
            (iv) R₄ eine der folgenden, gegebenenfalls modifizierten basischen Gruppen ist: 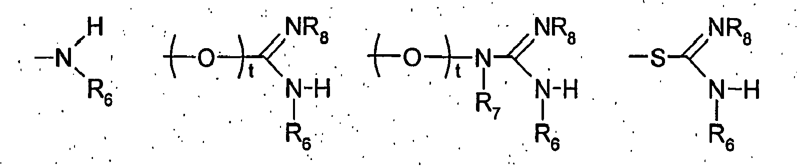 wobei t = 0, 1; R₆ und R₇ unabhängig voneinander, Wasserstoff oder eine Alkylgruppe mit 1 bis 6 C-Atomen oder eine Alkylengruppe mit 1 bis 5 C-Atomen, die mit A₂ einen Cyclus bildet, oder eine Hydroxyl-, Amino-, Alkylamino, Acyl- oder Alkyloxycarbonylgruppe sind, wobei die Alkylamino-Acyl- und Alkyloxycarbonylgruppen unabhängig voneinander 1 bis 6 C-Atome aufweisen; und wobei R₈ Wasserstoff oder eine Alkylgruppe mit 1 bis 3 C-Atömen ist oder eine Alkylengruppe mit 1 bis 3 C-Atomen ist, die mit R₆ einen Cyclus bildet;
            (v) Q entweder eine CH-Gruppe oder N ist;
            (vi) j = 0, 1, 2;
            k = 0,1, 2, 3;
            m, n unabhängig voneinander =0,1,2, 3,4, 5 sind, wobei m + n = 3, 4, 5;
            und wobei die Verbindung gemäß Formel (I) weder 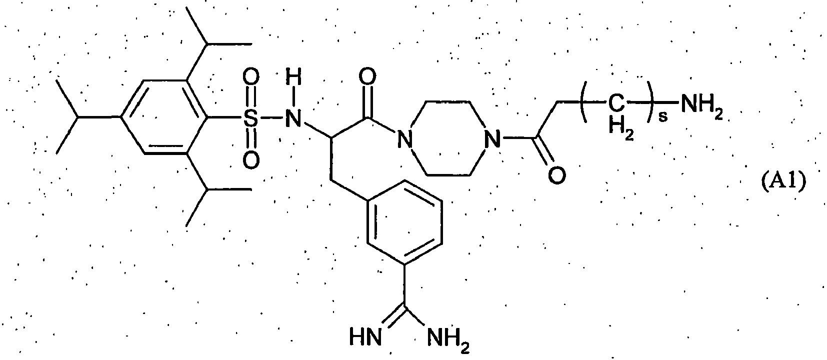 it s = 0, 1, 2 noch 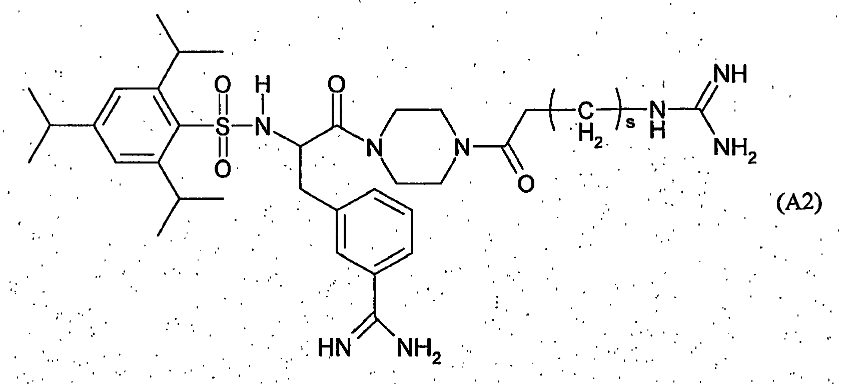 mit s = 0, 1 ist.
2. Verbindung nach 1, **dadurch gekennzeichnet, dass** T vorhanden und T eine der Gruppen wie m 1 definiert, ist, wobei die Amid- und Estergruppen in beiden Orientierungen eingebaut sein können.
3. Verbindung nach 1 oder 2, **dadurch gekennzeichnet, dass** sie die Struktur (I") aufweist.
4. Verbindung nach 1 bis 3, wobei j = 0 und R₁ ein höchstens zweifach substituierter Arylrest ist.
5. Verbindung nach 4, wobei der Arylrest über ein SauerstoHbruckenatom oder ein Schwefelbrückenatom oder über eine C₁-C₃ Alkylenkette mit einem weitieren Arylrest oder einem Heteroarylrest substituiert ist.
6. Verbindung nach 5, wobei der Arylrest, der über ein Sauerstoffbrückenatom mit einem weiteren Arylrest oder einem Heteroarylrest substituiert ist, ein Phenylrest oder ein Pyridylrest ist.
7. Verbindung nach; 3 oder 4, wobei der Arylrest oder der Heteroarylrest mindestens, einen Substituenten trägt ausgewählt aus der Gruppe Chlor, Fluor, Trifluormethyl, Methyl, Methoxy.
8. Verbindung nach 4, wobei der Arylrest substituiert ist mit mindestens einer Alkoxy-Gruppe.
9. Verbindung nach einem der 1 bis 4, wobei R₁ ausgewählt wird aus der Gruppe bestehend aus tert-Butylphenyl, Cyclohexylphenyl, 5,6,7,8-Tetrahydronaphthyl, Naphthyl, Anthracyl; Anthrachinoyl und Anthrahydrochinoyl, Pyrldylrixyphenyl, Phenyloxypyridyl, Pyridylalkylphenyl mit einem C₁-C₃-Alkyl.
10. Verbindung nach einem der. 1 bis 9, wobei R₂ ein mindestens einfach substituierter Phenylrest, Thienylrest oder Pyridylrest ist.
11. Verbindung nach einem der 1 bis 10, wobei k = 1 und R₂ ein mit einer Amidinogruppe meta-substituierter Phenylrest ist, wobei das dadurch entstehende 3-Amidinophenylalanin in der L-Konfiguration vorlegt.
12. Verbindung nach einem der 1 bis 11, wobei m = n = 2.
13. Verbindung nach einem der 1 bis 12, wobei der durch X₁ und X₂ gebildete Cyclus folgende Struktur aufweist: 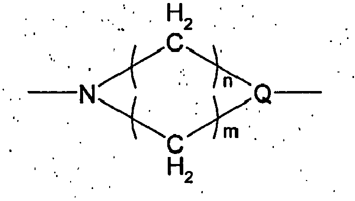
14. Verbindung nach einem der 1 bis 13, wobei A₁ nicht vorhanden und T 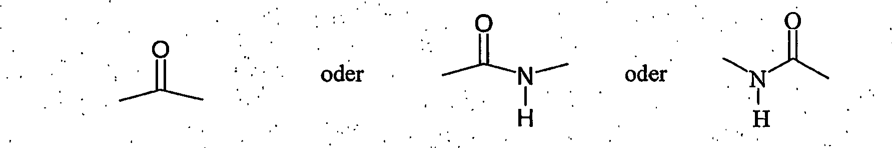 ist.
15. Verbindung nach einem der, 1 bis 14, wobei A₂ eine Methylen-, Ethyllen- oder Propylengruppe ist und R₄ ausgewählt wird aus 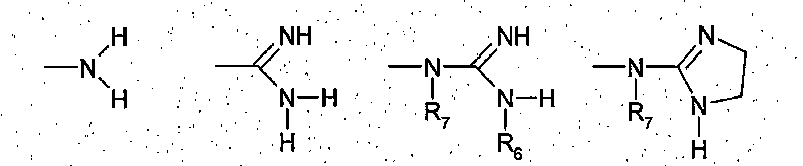 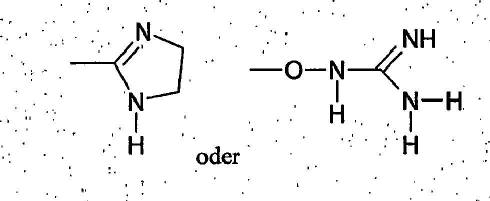
16. Verbindung nach einem der 2 bis 15, wobei m = n = 2 ist und R₃ ein uanidinooxyalkylrest ist oder ein Aryl- oder Heteroarylrest ist, und wobei der Arylrest vorzugsweise ein Benzyl- oder Phenoxy-Rest ist und der Heteroarylrest vorzugsweise ausgewählt ist aus einem Pyridinylmethylen-, Pyridinyloxo-, Pyrimidinyloxo-, Pyrazinyloxo-, Pyridinylthiorest und wobei der Aryl- oder Heteroaarylrest unsubstituiert oder substituiert ist mit mindestens einem Halogens mindestens einem Methoxyrest und/oder mindestens einem Trifluormethylrest.
17. Verbindung nach einem der vorherigen **dadurch gekennzeichnet, dass** zwischen dem Sulfenylrest und dem 3-Amidinophenylalaninrest der Formel I eine Aminosäure, vorzugsweise ein Glycin, eingebaut ist.
18. Verfahren zur Herstellung einer Verbindung gemäß einem der 1 bis 17 oder einer Verbindung (A1) oder (A2), umfassend den Schritt (S1): (S1) Umsetzung einer Verbindung der allgemeinen Struktur (E1') 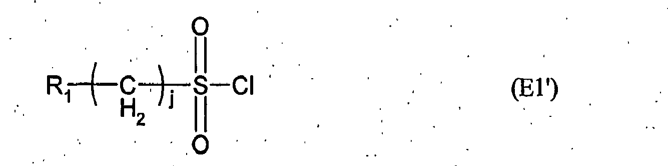 mit einer Verbindung der allgemeinen Struktur (E1") 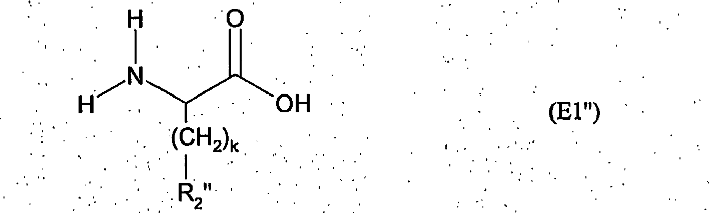 unter Erhalt einer Verbindung der allgemeinen Struktur (ZP1) 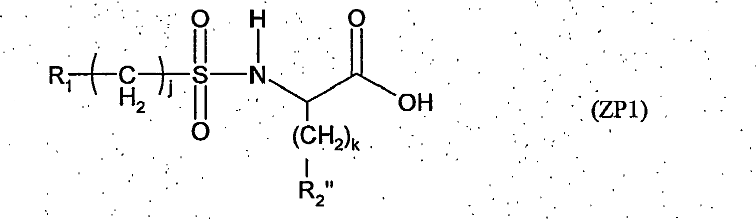 wobei R₂" der Arylrest R₂, substituiert entweder mit R₄ oder mit R₄, geschützt mit einer geeigneten Schutzgruppe, oder mit einem Substituenten, der eine Vorstufe zu R₄ darstellt.
19. Verfahren nach 18, wobei Q = N und A₁ nicht vorhanden ist, zusätzlich umfassend die Schritte (S2'), (S3') und (S4'):
   (S2') Umsetzung der Verbindung (ZP1) mit einer Verbindung der allgemeinen Struktur (E2') 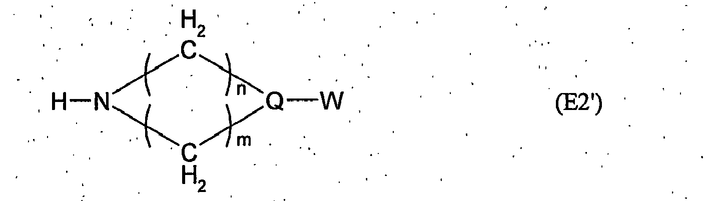 wobei W eine geeignete Schutzgruppe ist, unter Erhalt einer Verbindung der allgemeinen Struktur (ZP2') 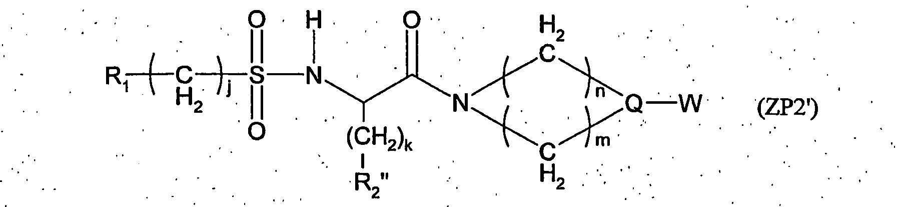 (S3') Abspaltung der Schutzgruppe W;
      (S4') Umsetzung der gemäß (S3') erhaltenen Verbindung mit einer Verbindung der allgemeinen Struktur (E2") 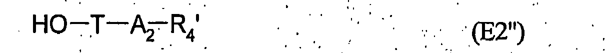 unter Erhalt einer Verbindung der allgemeinen Struktur (P1) 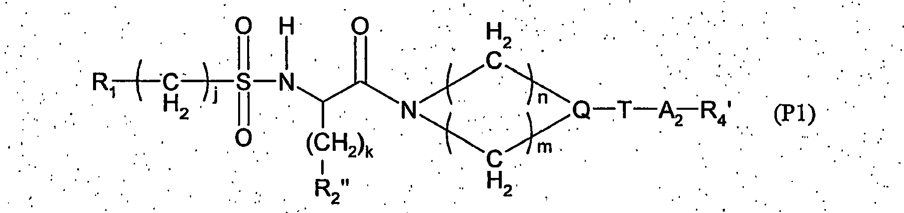 wobei R₄' entweder R₄ oder R₄, geschützt mit einer geeigneten Schutzgruppe, oder eine Vorstufe zu R₄ darstellt; oder
      (S2') Umsetzung einer Verbindung der allgemeinen Struktur (E2"'). 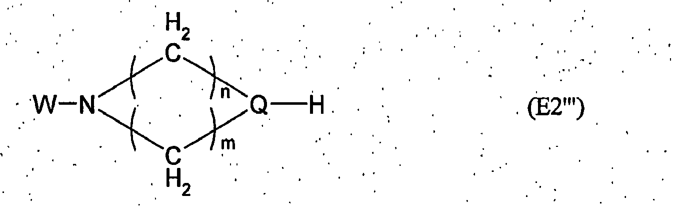 wobei W eine geeignete Schutzgruppe ist, mit einer Verbindung der allgemeinen Struktur (E2") 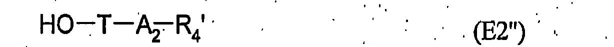 unter Erhalt einer Verbindung der allgemeinen Struktur (ZP2") 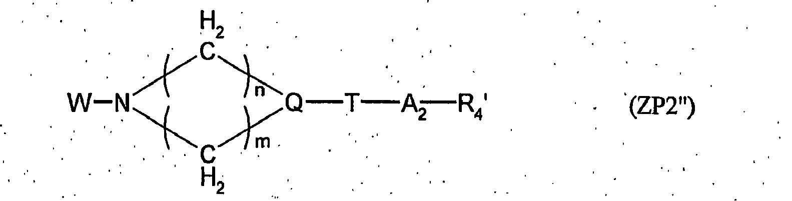 wobei R₄' entweder R₄ oder R₄, geschützt mit einer geeigneten Schutzgruppe, oder eine Vorstufe zu R₄ darstellt;
      (S3') Abspaltung der Schutzgruppe W;
      (S4') Umsetzung der gemäß (S3') erhaltenen Verbindung mit einer Verbindung
      der allgemeinen Struktur (ZP1) unter Erhalt einer Verbindung der allgemeinen Sümktur (P1).
20. Verfahren nach 18 wobei Q = CH, zusätzlich umfassend den Schritt (S2"):
   (S2") Umsetzung der Verbindung gemäß (ZP1) mit einer Verbindung der allgemeinen Struktur (E3) 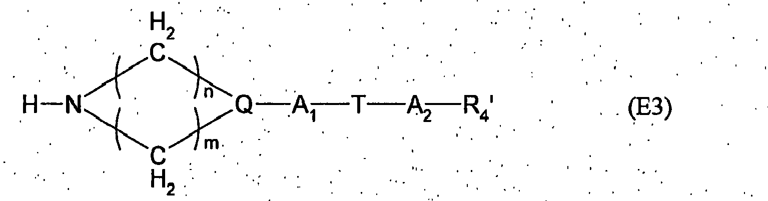 wobei R₄' entweder R₄ oder R₄, geschützt mit einer geeigneten Schutzgruppe, oder eine Vorstufe zu R₄ darstellt, unter Erhalt einer Verbindung der allgemeinen Struktur (P2) 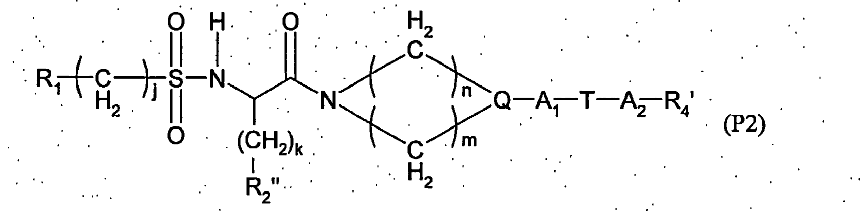
21. Verfahren nach 18, wobei T = -(C=O)-NH-, Q = CH und A₁ nicht vorbanden, zusätzlich umfassend die Schritte (S2"'), (S3"') und (S4"'):
   (S2"') Umsetzung einer Verbindung der allgemeinen Struktur (E3') 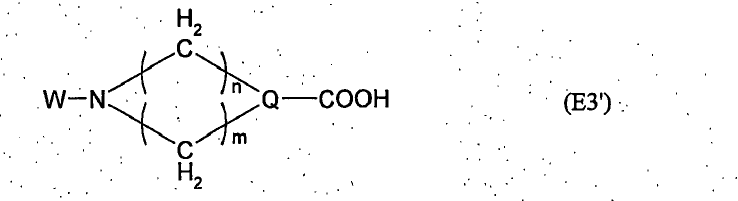 mit einer Verbindung der allgemeinen Struktur (E3") 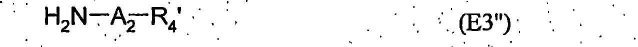 unter Erhalt einer Verbindung der allgemeinen Struktur (ZP3) 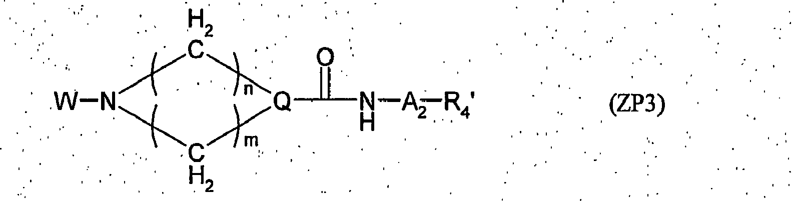 (S3"') Entfernung der Schutzgruppe W;
      (S4") Umsetzung der gemäß (S3"') erhaltenen Verbindung mit einer Verbindung gemäß (ZP1) unter Erhalt einer Verbindung der allgemeinen Struktur (P3) 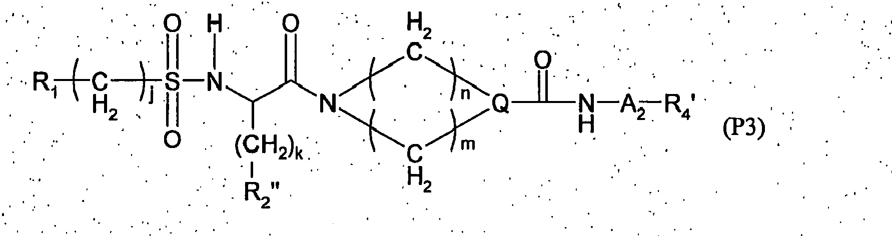
22. Verfahren nach 18, zusätzlich umfassend den Schritt Umsetzen der Verbindung (ZP1) mit einer Verbindung der allgemeinen Struktur (E2') 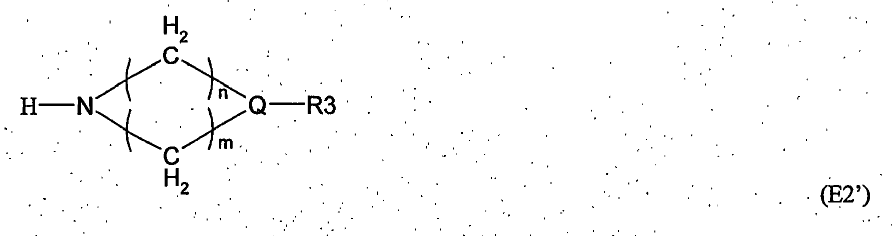 wobei Q = N ist, m=n=2 und R3, wie in 15 definiert ist.
23." Arzneimittel, enthaltend mindestens eine Verbindung gemäß einem der bis 17 oder eine Verbindung (A1) oder (A2) oder ein Salz dieser Verbindungen.
24. Arzneimittel nach 23, **dadurch gekennzeichnet, dass** es in Form einer Tablette, eines Dragees, einer Kapsel, eines Pellets, eines Suppositoriums, einer Lösung, insbesondere einer Injektions- oder Infusionslösung, von Augen-, Nasen- oder Ohrentropfen, eines Saftes, einer Emulsion oder Suspension, eines Globuli, eines Styli, eines Äerosols, eines Puders; einer Paste, einer Creme oder einer Salbe eingesetzt wird.
25. Verwendung einer Verbindung gemäß einem dei 1 bis 17 oder einer Verbindung (A1) oder (A2) oder eines Salzes dieser Verbindungen oder eines Arzneimittels gemäß 23 oder 24 zur Diagnose, Therapie oder Prophylaxe eines Tumors, insbesondere in oraler, subkutaner, intravenöser oder transfermaler Form.
26. Verwendung nach 25, wobei die. Bildung von Tumormetastasen reduziert wird.
27. Verwendung einer Verbindung gemäß einem der 1 bis 17 oder einer Verbindung (A1) oder (A2) oder eines Salzes dieser Verbindungen oder eines Arzneimittels gemäß 23 oder 24 zur Hemmung der Matriptase.
28. Verwendung nach 27, **dadurch gekennzeichnet, dass** die Matriptase MT-SP1 ist.

## Patentansprüche

1. Verbindung gemäß Formel 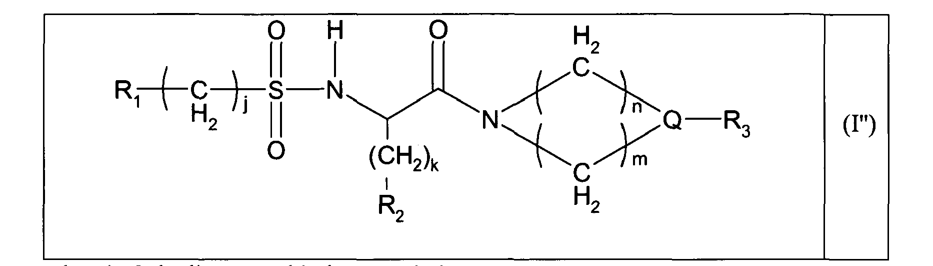 oder ein Salz dieser Verbindung, wobei
(i) R₁ eine gegebenenfalls teilweise hydrierte Aryl- oder Heteroarylgruppe, enthaltend mindestens eines der Atome O, N oder S, mit 5 bis 20 C-Atomen, oder eine lineare, verzweigte oder cyclische Alkylgruppe mit 1 bis 10 C-Atomen ist, wobei R₁ gegebenenfalls substituiert ist mit
- mindestens einer Halogen- und/oder Pseudohalogengruppe und/oder
- mindestens einer linearen, verzweigten oder cyclischen Alkyl- oder Alkyloxy- oder Alkylthiogruppe mit 1 bis 10 C-Atomen, die gegebenenfalls mindestens einfach substituiert ist mit einer Halogen-, Pseudohalogen-, Hydroxy-, Amino-, Cyano-, Amidino-, Guanidino oder Carboxylgruppe, wobei die Carboxylgruppe gegebenenfalls mit einer linearen, verzweigten oder cyclischen Alkylgruppe mit 1 bis 10 C-Atomen verestert ist, und wobei die lineare, verzweigte oder cyclische Alkylgruppe mit 1 bis 10 C-Atomen gegebenenfalls mindestens ein Heteroatom, ausgewählt aus der Gruppe bestehend aus O, N und S,
enthält, und/oder
- mindestens eine Aryl- oder Heteroarylgruppe mit 5 bis 20 C-Atomen, wobei diese Aryl- oder Heteroarylgruppe gegebenenfalls substituiert ist mit
-- mindestens einer linearen, verzweigten oder cyclischen Alkylgruppe mit 1 bis 10 C-Atomen und/oder
-- mindestens einer COR₂'- und/oder COOR₂'-Gruppe, wobei R₂' eine lineare, verzweigte oder cyclische Alkylgruppe mit 1 bis 10 C-Atomen ist, und/oder
-- mindestens einer Halogengruppe und/oder
-- mindestens einer Pseudohalogengruppe und/oder
-- mindestens einer Alkoxygruppe oder einer Alkylthiogruppe, wobei der Alkylrest jeweils 1 bis 10 C-Atome aufweist, und/oder
-- mindestens einer Nitrogruppe und/oder
-- mindestens einer Halogenalkylgruppe mit 1 bis 10 C-Atomen,
und wobei die Aryl- oder Heteroarylgruppe über eine Alkylengruppe mit 1 bis 3 C-Atomen oder ein Sauerstoffatom oder über ein Schwefelatom an den Rest R₁ gebunden ist;
- mindestens einer Hydroxy-, Amino-, Cyano-, Amidino-, Guanidino-, Carboxyl- oder Carboxyalkylgruppe, wobei die Aminogruppe gegebenenfalls acyliert ist und/oder wobei die Alkylgruppe der Carboxyalkylgruppe 1 bis 10 C-Atome aufweist und/oder die Carboxylgruppe gegebenenfalls mit einer linearen, verzweigten oder cyclischen Alkylgruppe mit 1 bis 10 C-Atomen verestert oder amidiert ist;
(ii) R₂ eine mindestens einfach substituierte Arylgruppe mit 1 bis 10 C-Atomen ist, wobei
- gegebenenfalls mindestens eines dieser C-Atome durch S, N oder O ersetzt ist,
- mindestens ein Substituent eine Gruppe gemäß R₄ ist,
- R₂ gegebenenfalls zusätzlich mit einer Hydroxy-, COR₂'- oder COOR₂'-Gruppe substituiert ist und R₂' eine lineare, verzweigte oder cyclische Alkylgruppe mit 1 bis 10 C-Atomen ist;
(iii) R₃ einen Rest der folgenden Formel (II) darstellt: 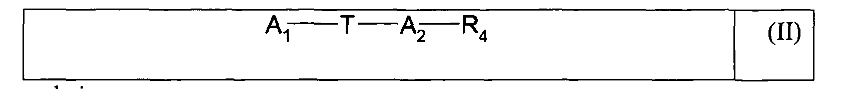 wobei
- A₁ entweder nicht vorhanden oder eine Alkylengruppe mit 1 bis 4 C-Atomen ist, die gegebenenfalls substituiert ist mit
-- mindestens einer Halogen- und/oder Pseudohalogengruppe und/oder
-- mindestens einer linearen, verzweigten oder cyclischen Alkylgruppe mit 1 bis 10 C-Atomen und/oder
-- mindestens einer Aryl- oder einer Aralkylgruppe mit 5 bis 10 C-Atomen und/oder
-- mindestens einer Cycloalkylgruppe mit 3 bis 10-Atomen und/oder-
-- mindestens einer Hydroxy-, Cyano-, Alkyloxy- oder Alkylthio mit 1 bis 10 C-Atomen, Carboxyl- oder Carboxyalkylgruppe, wobei die Alkylgruppe der Carboxyalkylgruppe 1 bis 10 C-Atome aufweist und/oder die Carboxylgruppe gegebenenfalls mit einem linearen, verzweigten oder cyclischen Alkylrest mit 1 bis 10 C-Atomen verestert oder amidiert ist;
- T entweder nicht vorhanden oder eine der folgenden Gruppen ist: 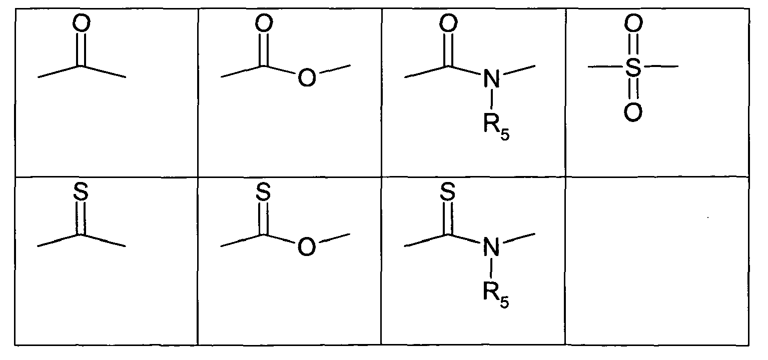 wobei R₅ Wasserstoff oder eine Alkylgruppe mit 1 bis 10 C-Atomen oder eine mit A₂ einen gegebenenfalls mindestens ein Heteroatom enthaltenden Cyclus bildende Alkylengruppe mit 1 bis 6 C-Atomen ist;
- A₂ eine lineare, verzweigte oder cyclische Alkylengruppe mit 1 bis 10 C-Atomen oder eine Aryl-, Heteroaryl- oder Aralkylengruppe mit 1 bis 10 C-Atomen, gegebenenfalls enthaltend mindestens ein Heteroatom, ausgewählt aus der Gruppe bestehend aus N, S und O, ist, die gegebenenfalls substituiert ist mit
-- mindestens einer Halogen- und/oder Pseudohalogengruppe und/oder
-- mindestens einer linearen, verzweigten oder cyclischen Alkylgruppe mit 1 bis 10 C-Atomen und/oder
-- mindestens einer Aryl- oder einer Aralkylgruppe mit 5 bis 10 C-Atomen und/oder
-- mindestens einer Cycloalkylgruppe mit 3 bis 10 C-Atomen und/oder-
-- mindestens einer Hydroxy-, Cyano-, Alkyloxy- oder Alkylthio- mit 1 bis 10 C-Atomen, Carboxyl- oder Carboxyalkylgruppe, wobei die Alkylgruppe der Carboxyalkylgruppe 1 bis 10 C-Atome aufweist und/oder die Carboxylgruppe gegebenenfalls mit einem linearen, verzweigten oder cyclischen Alkylrest mit 1 bis 10 C-Atomen verestert oder amidiert ist;
(iv) R₄ eine der folgenden, gegebenenfalls modifizierten basischen Gruppen ist: 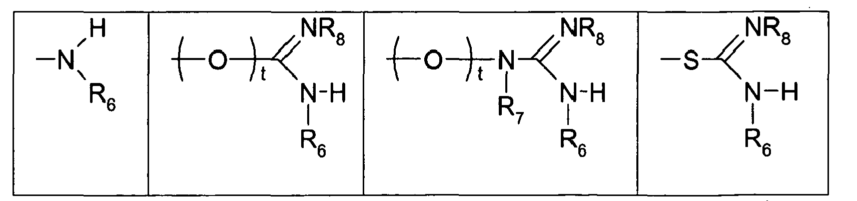 wobei t = 0, 1; R₆ und R₇ unabhängig voneinander, Wasserstoff oder eine Alkylgruppe mit 1 bis 6 C-Atomen oder eine Alkylengruppe mit 1 bis 5 C-Atomen, die mit A₂ einen Cyclus bildet, oder eine Hydroxyl-, Amino-, Alkylamino, Acyl- oder Alkyloxycarbonylgruppe sind, wobei die Alkylamino-, Acyl- und Alkyloxycarbonylgruppen unabhängig voneinander 1 bis 6 C-Atome aufweisen, und wobei R₈ Wasserstoff oder eine Alkylgruppe mit 1 bis 3 C-Atomen ist oder eine Alkylengruppe mit 1 bis 3 C-Atomen ist, die mit R₆ einen Cyclus bildet;
(v) Q N ist;
(vi) j = 0, 1, 2;
k = 0, 1, 2, 3;
m, n unabhängig voneinander = 0, 1, 2, 3, 4, 5 sind, wobei m + n = 3, 4, 5;
und wobei die Verbindung gemäß Formel (I") weder 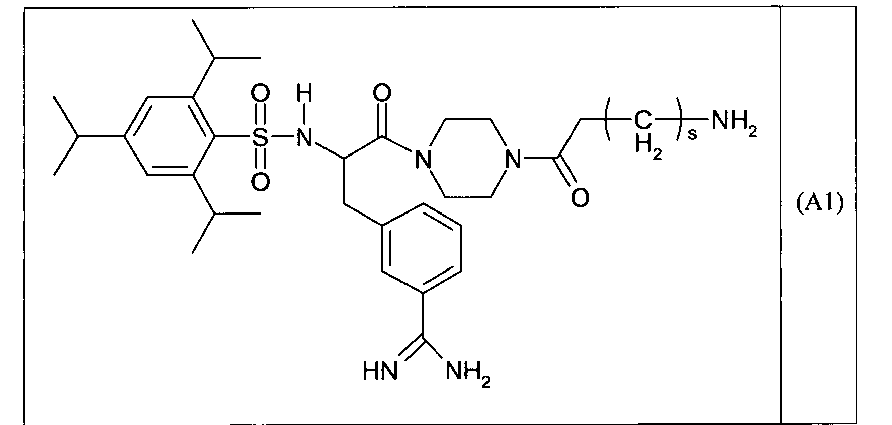 mit s = 0, 1, 2 noch 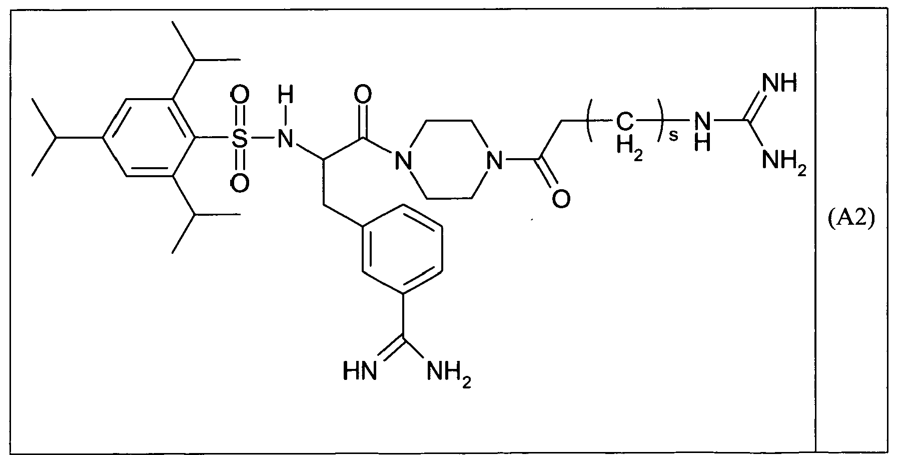 mit s = 0, 1 ist.

2. Verbindung nach Anspruch 1, **dadurch gekennzeichnet, dass** T vorhanden und T eine der Gruppen wie in Anspruch 1 definiert, ist, wobei die Amid- und Estergruppen in beiden Orientierungen eingebaut sein können.

3. Verbindung nach Ansprüchen 1 bis 2, wobei j = 0 und R₁ ein höchstens zweifach substituierter Arylrest ist.

4. Verbindung nach Anspruch 3, wobei der Arylrest über ein Sauerstoffbrückenatom oder ein Schwefelbrückenatom oder über eine C₁-C₃ Alkylenkette mit einem weiteren Arylrest oder einem Heteroarylrest substituiert ist, insbesondere wobei der Arylrest, der über ein Sauerstoffbrückenatom mit einem weiteren Arylrest oder einem Heteroarylrest substituiert ist, ein Phenylrest oder ein Pyridylrest ist.

5. Verbindung nach Anspruch 3, wobei der Arylrest oder der Heteroarylrest mindestens einen Substituenten trägt ausgewählt aus der Gruppe Chlor, Fluor, Trifluormethyl, Methyl, Methoxy, insbesondere wobei der Arylrest substituiert ist mit mindestens einer Alkoxy-Gruppe.

6. Verbindung nach einem der Ansprüche 1 bis 3, wobei R1 ausgewählt wird aus der Gruppe bestehend aus tert-Butylphenyl, Cyclohexylphenyl, 5,6,7,8-Tetrahydronaphthyl, Naphthyl, Anthracyl, Anthrachinoyl und Anthrahydrochinoyl, Pyridyloxyphenyl, Phenyloxypyridyl, Pyridylalkylphenyl mit einem C₁-C₃-Alkyl.

7. Verbindung nach einem der Ansprüche 1 bis 6, wobei R₂ ein mindestens einfach substituierter Phenylrest, Thienylrest oder Pyridylrest ist.

8. Verbindung nach einem der Ansprüche 1 bis 7, wobei k = 1 und R₂ ein mit einer Amidinogruppe meta-substituierter Phenylrest ist, wobei das dadurch entstehende 3-Amidinophenylalanin in der L-Konfiguration vorliegt.

9. Verbindung nach einem der Ansprüche 1 bis 8, wobei m = n = 2.

10. Verbindung nach einem der Ansprüche 1 bis 9, wobei A₁ nicht vorhanden und T 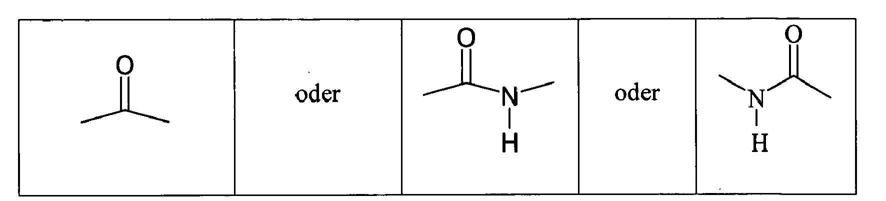 ist.

11. Verbindung nach einem der Ansprüche 1 bis 10, wobei A₂ eine Methylen-, Ethylen- oder Propylengruppe ist und R₄ ausgewählt wird aus

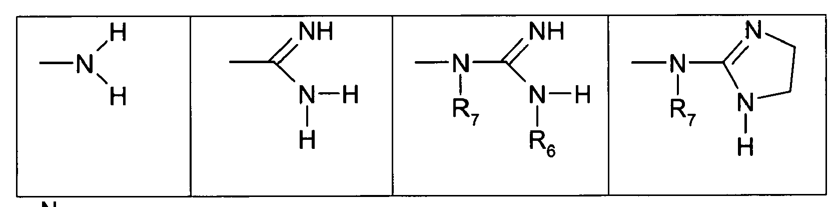

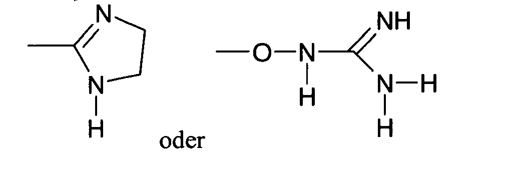

12. Verbindung nach einem der Ansprüche 2 bis 11, wobei m = n = 2 ist und R₃ ein Guanidinooxyalkylrest ist oder ein Aryl- oder Heteroarylrest ist, und wobei der Arylrest vorzugsweise ein Benzyl- oder Phenoxy-Rest ist und der Heteroarylrest vorzugsweise ausgewählt ist aus einem Pyridinylmethylen-, Pyridinyloxo-, Pyrimidinyloxo-, Pyrazinyloxo-, Pyridinylthiorest und wobei der Aryl- oder Heteroarylrest unsubstituiert oder substituiert ist mit mindestens einem Halogen, mindestens einem Methoxyrest und/oder mindestens einem Trifluormethylrest.

13. Arzneimittel, enthaltend mindestens eine Verbindung gemäß einem der Ansprüche 1 bis 12, insbesondere **dadurch gekennzeichnet, dass** es in Form einer Tablette, eines Dragees, einer Kapsel, eines Pellets, eines Suppositoriums, einer Lösung, insbesondere einer Injektions- oder Infusionslösung, von Augen-, Nasen- oder Ohrentropfen, eines Saftes, einer Emulsion oder Suspension, eines Globuli, eines Styli, eines Aerosols, eines Puders, einer Paste, einer Creme oder einer Salbe eingesetzt wird.

14. Verwendung einer Verbindung gemäß einem der Ansprüche 1 bis 12 oder eines Arzneimittels gemäß Anspruch 13 zur Diagnose, Therapie oder Prophylaxe eines Tumors, insbesondere in oraler, subkutaner, intravenöser oder transdermaler Form, insbesonere wobei die Bildung von Tumormetastasen reduziert wird.

15. Verwendung einer Verbindung gemäß einem der Ansprüche 1 bis 12 oder eines Arzneimittels gemäß Anspruch 13 zur Hemmung der Matriptase, insbesondere **dadurch gekennzeichnet, dass** die Matriptase MT-SP1 ist.
